# EUROPÄISCHE PATENTSCHRIFT

(11) **EP 1 326 632 B1**
(45) Veröffentlichungstag und Bekanntmachung des Hinweises auf die Patenterteilung: **06.09.2006**
(21) Anmeldenummer: 01986607.8
(22) Anmeldetag: 11.10.2001
(51) Int. Cl.: A61K 38/58, A61K 31/00, A61K 38/06, A61K 38/07, A61K 38/05, A61K 38/04, A61P 31/12

(54) **PROTEASOME INHIBITOREN ZUR BEHANDLUNG VON HEPATITIS-VIRUS INFEKTIONEN**
PROTEASOME INHIBITORS FOR THE TREATMENT OF HEPATITIS VIRUS INFECTIONS
INHIBITEURS DU PROTEASOME POUR LE TRAITEMENT D'INFECTIONS D'HEPATITE VIRALE

(30) Priorität: 12.10.2000 DE 10051716; 03.10.2001 DE 10149398
(43) Veröffentlichungstag der Anmeldung: 16.07.2003
(60) Teilanmeldung: 04090076.3
(73) Patentinhaber: VIROMICS GMBH, 07743 Jena (DE)
(72) Erfinder: SCHUBERT, Ulrich, 07407 Uhlstädt (DE); WILL, Hans, 22549 Hamburg (DE); TESSMER, Uwe, 20253 Hamburg (DE); SIRMA, Hüsseyin, 69168 Wiesloch (DE); PRASSOLOW, Alexij, 22529 Hamburg (DE); SCHUBERT, Evelyn, 07407 Uhlstädt (DE); HOHENBERG, Heinz, 22767 Hamburg (DE); WELKER, Reinhold, 72770 Reutlingen (DE)
(74) Vertreter: Wehlan, Helmut
(86) Internationale Anmeldenummer: PCT/DE2001/003908
(87) Internationale Veröffentlichungsnummer: WO 2002/030455

(56) Entgegenhaltungen:
- EP-A- 0 652 290
- WO-A-00/33654
- WO-A-01/46448
- WO-A-96/32105
- WO-A-99/54317
- US-A- 6 083 903
- SCHUBERT U ET AL: "CD4 glycoprotein degradation induced by human immunodeficiency virus type 1 vpu protein requires the function of proteasomes and the ubiquitin-conjugating pathway" JOURNAL OF VIROLOGY, THE AMERICAN SOCIETY FOR MICROBIOLOGY, US, Bd. 72, Nr. 3, März 1998 (1998-03), Seiten 2280-2288, XP002121469 ISSN: 0022-538X
- EVERETT ROGER D: "A surprising role for the proteasome in the regulation of herpesvirus infection." TRENDS IN BIOCHEMICAL SCIENCES, Bd. 24, Nr. 8, August 1999 (1999-08), Seiten 293-295, XP004174246 ISSN: 0968-0004
- EVERETT R.D., ET AL.: "A viral activator of gene expression functions via the ubiquitun-proteasome pathway" THE EMBO JOURNAL, Bd. 17, Nr. 24, 1998, Seiten 7161-7169, XP002196934
- FINE S M ET AL: "Proteasome blockers inhibit TNF-alpha release by lipopolysaccharide stimulated macrophages and microglia: implications for HIV-1 dementia." JOURNAL OF NEUROIMMUNOLOGY, (1999 MAR 1) 95 (1-2) 55-64. , XP001069595
- FUJITA K ET AL: "Rapid degradation of CD4 in cells expressing human immunodeficiency virus type 1 Env and Vpu is blocked by proteasome inhibitors." JOURNAL OF GENERAL VIROLOGY, (1997 MAR) 78 ( PT 3) 619-25. , XP001069588
- ELLIOTT P.J. ET AL: "Recent advances in understanding proteasome function." CURRENT OPINION IN DRUG DISCOVERY AND DEVELOPMENT, (1999) 2/5 (484-490). , XP001070303

## Beschreibung

Die Erfindung betrifft Mittel zur Behandlung von Infektionen mit Hepatitis- Viren. Gegenstand der Erfindung sind Mittel - die als Wirkstoff Proteasom-Inhibitoren enthalten - zur Hemmung der Freisetzung, der Reifung, der Infektiosität und somit der Replikation von Hepatitisviren.

Weiterhin können diese Mittel für die Behandlung, Therapie und Hemmung einer Virushepatitis verwendet werden. Es wird gezeigt, dass die Anwendungen dieser Mittel zur Freisetzung von nicht infektiösen Hepatitisviren aus infizierten Zellen führen. Diese Mittel können daher die Ausbreitung einer akuten Infektion mit Hepatitisviren begrenzen. Des weiteren sind die Mittel für nicht proliferierende Hepatozyten weniger toxisch als für Non-Parenchynizellen der Leber und Leberkarzinomzellen- Damit sind diese Mittel für eine bevorzugte Zerstörung von Leberkarzinomzellen in mit HBV (Hepatitis-B-Viren, Abkürzungsverrxichnis hinter den Beispielen) und HCV (Hepatitis-C-Viren) infizierten Patienten und Tieren geeignet. Bei den Mitteln handelt es sich um verschiedene Substanzklassen, welchen gemeinsam ist, dass sie das Ubiquitin-Proteasom-System inhibieren. Speziell sind diese Mittel dadurch charakterisiert, dass diese Substanzen, die Proteasom-Inhibitoren, die Aktivität der hauptsächlichen zellulären Protease, das ist das Proteasom, in den behandelten Zellen inhibieren. Am.Beispiel des Enten-Hepatitisvirus wie auch des humanen Hepatitis-B-Virus wird gezeigt, dass die Anwendung von Proteasom-Inhibitoren die Freisetzung von infektiösen Enten-Hepatitisviren und humanen Hepatitis-B-Viren aus bereits infizierten Hepatozyten drastisch reduzieren (gezeigt am Beispiel des Enten-Hepatitisvirus). Die Proteasam-Inhibitoren können daher die Virämie sowohl bei einer Neuinfektion als auch bei chronischen Infektionen mit Hepatitisviren unterdrücken, und der Erfolg einer Viruseliminierung durch das eigene Immunsystem und/oder durch bekannte Mittel mit ähnlicher oder anderer Wirkung kann erhöht werden. Die Folgen einer HBV- und HCV-Infektion - wie z.B. Leberschädigungen unterschiedlichen Schweregrades bis hin zur häufig tödlich verlaufenden fulminanten Hepatitis, Entwicklung einer Leberzirrhose/Fibrose und eines Leberkarzinoms - können durch den Einsatz dieser Mittel, den Proteasom-Inhibitoren, verhindert, gemindert oder revertiert werden. Anwendungsgebiete für diese Erfindungen ist somit die anti-virale Therapie von Hepatitisvirus-Infektionen, insbesondere zur Verhinderung der Etablierung sowie der Aufrechterhaltung einer akuten und chronischen HBV- und HCV-Infektion.

### Charakteristik des bekannten Standes

### 0. Einleitung

Seit Anfang der 80er Jahre hat die Pandemie des erworbenen Immunmangelsyndroms (Aquired Immunodeficiency Syndrome, AIDS) Millionen von HIV-infizierten Menschen mit einer heimtückischen, multisystemischen und ultimativ bislang unheilbaren Krankheit konfrontiert. Zum gegenwärtigen Zeitpunkt ist weder eine wirksame Strategie zur Vakzinierung gegen HIV bekannt, noch sind andere Mechanismen der Stimulation einer nach wie vor wenig verstandenen natürlichen Immunität gegenüber einer HIV-Infektion für eine breite Anwendung applizierbar. Für die Therapie einer bereits etablierten HIV-Infektion oder zum Schutz vor einer systemischen Manifestierung einer HIV-Infektion unmittelbar nach Aufnahme des Virus sind verschiedene anti-retrovirale Medikamente im Einsatz. Diese stellen im wesentlichen Substanzen dar, welche die viralen Enzyme Reverse Transkriptase (RT) sowie Protease (PR) hemmen. Neben dem Problem der Unverträglichkeit besteht die hauptsächliche Limitierung dieser Medikamente in der enormen, bis 10⁶-Mal höheren Mutationsrate (verglichen mit der Replikation humaner DNA) von HIV. Der dadurch bedingte Polymorphismus führt unweigerlich und in relativ kurzer Zeit zum Auftreten von HIV-Mutanten, die gegenüber einzelnen oder sogar kombinierten anti-HIV-Therapeutika, insbesondere der HAART-Therapie (highly active antiretroviral therapy - Patentschrift WO 00/33654), resistent sind. Das Ziel der weiteren HIV-Forschung besteht damit in der Identifizierung von zellulären Angriffspunkten ("Targets") für eine anti-retrovirale Therapie. Dies betrifft zelluläre Faktoren, Enzyme oder komplexe Mechanismen, die für die Replikation von HIV in der Wirtszelle essentiell sind und selektiv manipuliert werden können, ohne die Vitalität des Gesamtorganismus wesentlich zu beeinträchtigen. Diesen Anspruch erfullt die überraschende Feststellung, welche in dieser Erfindung beschrieben wird, und zwar, dass die Mittel, die Proteasom-Inhibitoren, späte Prozesse in der Replikation von HIV-1 und HIV-2 inhibieren und dadurch die Freisetzung und die Formierung von infektiösen Nachkommenviren verhindern.

WO 01/46448 offenbart eine Methode zur Behandlung von Virus-Infektionen verursacht unter anderem durch den human Cytomegalovirus, Herpes Simplex Virus oder auch Varicella Zoster Virus durch die Verwendung von Z-Leu-Leu-H, E64d und anderen Protease-Inhibitoren. EP-A-0 652 290 beschreibt die Verwendung von N-Acetyl-Leu-Leu-NorL und anderen peptidischen Aldehyd-Protease-Inhibitoren in der Behandlung von Virusinfektionen (z.B. AIDS) durch die Inhibierung von Chymotrypsin-ähnlichen Serinproteasen. US 6,083,903 offenbart die Verwendung von Borestern und -säuren MG-261 bis MG-369 als Proteasom-Inhibitor in der Behandlung von AIDS. WO 00/33654 beschreibt den Einsatz des Proteasom-Inhibitors Ritonavir zur Behandlung von AIDS und AIDS-begleitenden Krankheiten unter anderem in der HAART-Therapie. WO 96/32105 offenbart neue Proteasom-Inhibitoren, die strukturell von Lactacystein und Lactacystein β-Lacton abgeleitet sind, und in der Behandlung von HIV-Infektionen als auch in der Diagnostik eingesetzt werden. Die wissenschaftlichen Veröffentlichen Schubert et al., J. of Virology, 1998, S. 2280-2288; Everett et al., EMBO Journal, 1998, S. 7161-7169; sowie Roger D. Everett., Trends in Biochemical Sciences, 1999, S. 293-295 beschreiben die Rolle des Proteasomes bei viralen Infektionen durch Herpes-Viren und bei AIDS. Darüber hinaus weisen Fujita et al., J. of General Virology, 1997, S. 619-625 und Fine et al., J. of Neuroimmunology, 1999, S. 55-64 auf eine mögliche potentielle Rolle von Proteasom-Inhibitoren bei der Behandlung von HIV-Demenz sowie deren Wirkung gegen die Zersetzung von HIV-infizierter CD4-positiver Zellen hin.

Die Infektion mit Hepatitis-B-Viren (ca. 5% der Weltbevölkerung sind betroffen) und Hepatitis-C-Viren (ca. 3% der Weltbevölkerung sind betroffen) gehört neben HIV/AIDS-Problematik zu den großen Weltgesundheitsproblemen. Infektionen mit HBV oder HCV haben häufig einen chronischen Virus-Trägerstatus zur Folge. Zu den Krankheitssymptomen der Infektionen gehören Leberentzündungen verschiedener Schweregrade bis hin zum Leberversagen (fulminante Hepatitis), ein erhöhtes Risiko der Entwicklung einer Leberzirrhose und Fibrose sowie die Entstehung von Leberkarzinomen. Neuinfektionen mit HBV können relativ effizient, aber wegen der Existenz von Impfversagem und Immunescape-Varianten nicht komplett, durch eine prophylaktische Immunisierung verhindert werden. Für eine HCV-Neuinfektion gibt es bisher keinen Impfschutz. Trotz der Vielzahl von Medikamenten für die Therapie einer chronischen HCV- und HBV-Infektion, die alle mit Nebenwirkungen belastet sind und im wesentlichen aus Zytokinen (Interferon alpha und Varianten hiervon) sowie Nukleosidanaloga bestehen, ist es bisher nicht möglich, die Mehrzahl der chronischen Träger von HBV und HCV zufriedenstellend zu therapieren, da entweder die Patienten nicht ansprechen auf die Medikamente oder nur kurzzeitige Besserung eintritt und in der Regel das Virus durch die Behandlung nicht vollständig eliminiert werden kann. Auch die passive Gabe von HBV-spezifischen neutralisierenden Antikörpern und/oder Nukleosidanaloga oder anderen Medikamenten bei Lebertransplantierten verhindert in der Regel nicht die Neuinfektion der transplantierten Leber. Immunsuppression von Patienten mit abklingenden Hepatitiden kann zur Reaktivierung von latent vorhandenen Viren fuhren. Das Hauptproblem bei Nukleosidanaloga ist die hohe Mutationsrate sowohl von HBV als auch von HCV, wodurch sich Medikamentenresistente Virusstämme während der Behandlung entwickeln. Um die Probleme der bisher verfügbaren antiviralen und therapeutischen Mittel für die Hepatitis B und C zu umgehen, sind neue Therapieansätze notwendig, welche - ähnlich der anti-retroviralen Therapie - konservierte zelluläre Faktoren beeinflussen, die für die Vermehrung dieser Viren in der Wirtszelle essentiell sind. Solche Mittel werden in dieser Erfindung beschrieben. Und zwar betrifft dies die überraschende Erkenntnis, dass Proteasom-Inhibitoren - ähnlich der neuartigen Effekte auf Retroviren - ebenfalls die Produktion von infektiösen Hepatitisviren verhindern und dabei gleichzeitig das Absterben von Lebertumorzellen induzieren. Diese Mittel, die Proteasom-Inhibitoren, sind wegen der Möglichkeit, diese bevorzugt in die Leber zu transportieren, besonders für die selektive Therapie von viralen Lebererkrankumgen und Leberkarzinomen geeignet (Literaturverzeichnis hinter den Ausführungsbeispielen).

### 1. Funktion des Ubiquitin/Proteasom-Pathway

Proteasomen sind multikatalytische und multi-Subunit Enzymkomplexe, die ca. 1 % des Gesamt-Zellproteins darstellen und als die hauptsächliche proteolytische Komponente im Zellkern und Zytosol aller eukaryontischen Zellen vorkommen. Die wesentliche Funktion von Proteasomen ist die Proteolyse von missgefalteten, nicht-funktionellen oder für den schnellen Abbau bestimmten, in der Regel regulatorischen Proteinen. Eine weitere Funktion des proteasomalen Abbaus einer Vielzahl von zellulären oder viralen Proteinen ist die Generierung von Peptidliganden für Major Histocompatibilitäts (MHC) Klasse-I-Moleküle, welche für die T-Zellvermittelte Immunantwort notwendig ist (für Review siehe Rock und Goldberg, 1999).

Proteasom-Targets werden in der Regel für den proteasomalen Abbau durch die Anheflung von oligomeren Formen von Ubiquitin (Ub) markiert. Ub ist ein hochkonserviertes, 76 Aminosäuren langes Protein, das kovalent an Targetproteine *via* Isopeptidbindung zwischen dem COOH-Terminus und der ε-NH₂-Gruppe von Lysin-Seitenketten gekoppelt wird, entweder am Targetprotein oder an Ub-Moleküle, die bereits an das Targetprotein geheftet sind. Das Ergebnis der Konjugation von Ub-Molekülen ist die Formierung von sogenannten poly-Ub-Ketten. Allgemein sind Multimere von vier Ub-Molekülen notwendig, um als Signal für den Abbau durch das Proteasom zu fungieren. Die Ubiquitinierung selbst ist reversibel, und Ub-Moleküle können durch eine Vielzahl von Ub-Hydrolasen von dem Targetmolekül wieder entfernt werden. Die Verbindung zwischen der Ubiquitinierung von Targetproteinen und der proteasomalen Proteolyse wird allgemein als Ubiquitin-Proteasom-System (UPS) bezeichnet (für Review siehe Hershko und Chiechanover, 1998; Baumeister *et al.,* 1998).

Das 26S Proteasom ist ein 2.5 Megadalton (MDa) großer Multienzymkomplex, welcher aus ca. 31 Untereinheiten besteht (für Review siehe Voges *et al.,* 1999). Die proteolytische Aktivität des Proteasom-Komplexes wird durch eine core-Struktur, dem 20S Proteasom, realisiert. Das 20S Proteasom bildet einen aus 14 nicht identischen Proteinen (im Molekulargewichtsbereich von 21 bis 31 kDa) bestehenden komplizierten Multienzymkomplex, der in zwei α-und zwei β-Ringen in einer αββα-Reihenfolge angeordnet ist (für Review siehe Voges *et al.,* 1999). Die Substratspezifität des 20S Proteasom umfasst drei wesentliche proteolytische Aktivitäten: Trypsin-, Chymotrypsin- und Postglutamyl-Peptid hydrolysierende- (PGPH), oder auch Kaspaseähnliche Aktivitäten, welche in den β-Untereinheiten Z, Y und Z lokalisiert sind. Die enzymatische Aktivitäten des 20S Proteasoms werden durch Anlagerung der 19S regulatorischen Untereinheiten gesteuert, welche zusammen das aktive 26S Proteasom-Partikel bilden. Die 19S regulatorischen Untereinheiten sind bei der Erkennung von poly-ubiquitinierten Proteinen sowie bei der Entfaltung von Targetproteinen beteiligt. Die Aktivität des 26S Proteasom ist ATP-abhängig und degradiert fast ausschließlich nur poly-ubiquitinierte Proteine. Die katalytisch aktiven β-Untereinheiten des 20S-Proteasoms (X, Y und Z) können durch γ-Interferon induzierbare MHC-kodierte Untereinheiten ausgetauscht werden, die dann das sogenannte "Immuno-Proteasom" bilden (Gaczynska *et al.,* 1993).

### 1.1. Bedeutung des Ubiquitin-Proteasom-Systems in der Pathogenese klinisch relevanter Krankheiten

Die enge Verknüpfung des UPS (Ubiquitin-Proteasom-System) mit zellulären Mechanismen erklärt die Bedeutung dieses Systems für zahlreiche pathologische Mechanismen, von denen bislang nur ein geringer Teil bekannt ist (für Review siehe Schwartz und Ciechanover, 1999). Eine zentrale Funktion übt das UPS bei Erkrankungen des Immunsystems aus. Zum einen ist der 26S Proteasom-Komplex die hauptsächliche Protease in der MHC-I-Antigenprozessierung, und zum anderen kann die Aktivität des Proteasoms selbst sowohl durch γ-Interferon induzierbare katalytische β-Untereinheiten manipuliert werden. Viele entzündliche und immunologische Krankheiten stehen im Zusammenhang mit dem Transkriptionsfaktor NF-κB, welcher verschiedene Gen-Funktionen in der Immunantwort reguliert. Die Aktivierung von NF-κB, die durch Ubiquitinierung und spezifische Spaltung eines Vorläuferproteins durch das Proteasom gesteuert wird, führt zur erhöhten Expression von verschiedenen Zytokinen, Adhäsionsmolekülen, entzündlichen und Stress-Response-Proteinen sowie Immunrezeptoren (für Review siehe Chiechanover *et al.,* 2000; Schwartz und Ciechanover, 1999).

### 1.2. Proteasom-Inhibitoren

Verschiedene Substanzklassen sind als Proteasom-Inhibitoren bekannt. Es sind zum einen chemisch modifizierte Peptidaldehyde wie der Tripeptidaldehyd N-carbobenzoxyl-L-leucinyl-L-leucinyl-L-leucinal (zLLL), das auch als MG132 bezeichnet wird sowie das um den Faktor 10 wirksamere Borsäure-Derivat MG232. Das zLLL und davon abgeleitete Derivate blockieren das Proteasom reversible durch Ausbildung einer transienten Hemiacetal-Struktur mit der katalytisch aktiven Threonin-Hydroxyl-Seitenkette in Position 1 der β-Untereinheit des 26S Proteasoms (für Review siehe Coux *et al.,* 1996). Ähnlich zu zLLL wurde eine weitere Klasse von modifizierten Peptiden als Proteasom-Inhibitoren, Peptid-Vinyl-Sulfone, beschrieben (Bogyo *et al.,* 1997).
Natürlich vorkommende Substanzen, isoliert aus Mikroorganismen, sind Lactacystin (LC) (Fenteany *et al.,* 1995) aus Streptomyceten sowie Epoxomicin aus Aktinomyzeten (Meng *et al.,* 1999a,b). LC ist ein hoch spezifischer und wirksamer Proteasom-Inhibitor, welcher das Proteasom durch Transesterifizierung und Alkylienmg der Threonin-Seitenkette in der β-Untereinheit irreversibel inaktiviert (Fenteany *et al.,* 1995). LC ist daher ein irreversibler, kovalent wirkender Proteasom-Inhibitor, welcher hauptsächlich die Chymotrypsin- und die Trypsin-ähnlichen Aktivitäten des 26S Proteasom-Partikels blockiert (Fenteany *et al.,* 1995). LC hat keine Peptid-Grundstruktur, sondern besteht aus einem γ-Lactam-Ring, einem Cystein und einer Hydroxy-butyl-Gruppe. LC selbst inhibiert nicht das Proteasom. Vielmehr wird in wässriger Lösung der N-Acetyl-Cystein-Rest hydrolysiert. Das Resultat ist die Bildung eines Clastolactacystein β-Lactons. Diese Lacton-Struktur ist in der Lage, Zellmembranen zu penetrieren. Nach Zellaufnahme kommt es zum nukleophilen Angriff des β-Lacton-Rings und anschließender Transesterifizierung der Threonin¹-Hydroxyl-Gruppe der β-Untereinheit.
Ein weiterer Proteasom-Inhibitor ist das natürlich vorkommende Epoxyketon Epoxomicin. Hinsichtlich der Spezifität für das 26S Proteasom und Wirksamkeit ist Epoxomicin der bislang wirksamste von allen bekannten natürlich vorkommenden Proteasom-Inhibitoren (Meng *et al.,* 1999a,b). Weiterhin zeichnet sich Epoxomicin durch eine vergleichsweise geringe Toxizität in Zellkulturen aus (Hanada *et al.,* 1992).

Eine weitere und sehr potente Klasse an synthetischen Proteasom-Inhibitoren sind Borsäure-Peptid-Derivate, insbesondere die Verbindung Pyranozyl-Phenyl-Leuzinyl-Borsäure mit dem Namen "PS-341 ". PS-341 ist sehr stabil unter physiologischen Bedingungen und ist bioverfügbar nach intravenöser Applikation (Adams und Stein, 1996; Adams *et al.,* 1998). Borsäure-Peptid-Derivate sind allgemein bekannt als Inhibitoren verschiedenster eukaryotischer Proteasen, wie zum Beispiel Thrombin, Elastase, Dipeptidylprotease IV (für Review siehe Adams und Stein, 1996). Die besondere Wirksamkeit von PS-341 als Proteasom-Inhibitor wird durch die sehr stabile Bindung zwischen Borsäure- und Hydroxyl-Gruppe der katalytisch aktiven Seitenkette von Thr¹ in der aktiven β-Untereinheit des 20S-Proteasoms mit einer inhibitorischen Konstante (Ki) von 0.6 nM realisiert (Adams und Stein, 1996). Außer dem Proteasom ist bislang keine zelluläre Protease bekannt, welche durch PS-341 beeinflusst wird.

Andere, zu PS-341 ähnliche Borsäure-Peptid-Derivate wurden als Proteasom-Inhibitoren beschrieben, wie zum Beispiel Benzoyl-Phenylanalin-Borsäure-Leucin (Gardner *et al.,* 2000). Ebenfalls wurde ein stark potenter Proteasom-Inhibitor mit der Bezeichnung "PS-273" (Morpholin-CONH-(CH-Naphtyl)-CONH-(CH-isobutyl)-B(OH)₂) beschrieben, welcher eine ähnliche Grundstruktur wie PS-341 hat, jedoch N-terminal eine Morpholin-Sturktur aufweist, dadurch mehr hydrophob ist und vermutlich somit leichter Membranen penetrieren kann als PS-341 (Adams *et al.,* 1999).

### Klinische Applikation von Proteasom-Inhibitoren

Der Proteasom-Komplex übt essentielle Zellfunktionen aus und ist für die Zellvitalität unverzichtbar. Die permanente Hemmung der Proteasom-Aktivität kann daher zu Veränderungen in der Regulation des Zellzyklus, der Transkription, der gesamten zellulären Proteolyse sowie der MHC-I Antigenprozessierung führen (für Review siehe Hershko und Ciechanover, 1998). Vollständige Inhibierung der Proteasom-Aktivität führt allgemein zu Zellzyklusarrest und Zelltod. Eine dauerhafte Inhibierung aller enzymatischen Aktivitäten des Proteasoms ist mit dem Leben einer Zelle und somit des Gesamtorganismus nicht vereinbar.

Es zeigt sich jedoch, dass neuartige reversibel wirkende Proteasom-Inhibitoren selektiv einzelne proteolytische Aktivitäten des 26S Proteasoms inhibieren, ohne dabei andere zelluläre Proteasen zu beeinflussen. Die Zytotoxizität solcher Inhibitoren ist daher wesentlich geringer im Vergleich zu den relativ unspezifisch wirkenden Proteasom-Inhibitoren, wie zum Beispiel dem Peptidaldehyd zLLL. Diese Tatsache erlaubt sowohl die *in vivo* Anwendung solcher neuartigen Proteasom-Inhibitoren (Meng *et al.,* 1999a) als auch die Etablierung von permanenten Zelllinien, welche relativ hohe Konzentrationen an Proteasom-Inhibitoren tolerieren (Glas *et al.,* 1998; Geier *et al.,* 1999).

Der Anspruch, dass bestimmte Proteasom-Inhibitoren in einem bestimmten Dosis-Regime in vivo verträglich sein können, wurde mehrfach gezeigt. Beispielsweise wurde die Selektion von Maus-Thymus-Zelllinien beschrieben, welche die ständige Anwesenheit von 10 µM des Proteasom-Inhibitors z-Leuzinyl-Leuzinyl-Leuzinyl-vinylsulfon (NLVS) tolerieren und dabei normales Zellwachstum und Zellmetabolismus mit gleichzeitig eingeschränkter MHC-I-Antigenpräsentation besitzen (Glas *et al.,* 1998). Ähnliche Ergebnisse wurden mit dem sehr potenten Proteasom-Inhibitor LC erzielt, der bis zu 6 µM in Zellkultur toleriert wurde (Geier *et al.,* 1999).

Epoxomicin, ein Epoxy-β-aminoketon modifiziertes Peptid, wurde als eine völlig neuartige Klasse von Proteasom-Inhibitoren aus *Aktinomyceten* isoliert (Hanada *et al.,* 1992). Epoxomicin besitzt eine starke zytotoxische Wirkung gegen verschiedene *in vitro* kultivierte Tumorzelllinien und zeigte im Maus-Modell *in vivo* inhibitorische Aktivität gegen Melanom- und Leukämie-Modelltumore (Hanada *et al.,* 1992).

Die Bedeutung von Proteasom-Inhibitoren als ein neues therapeutisches Prinzip hat in den vergangenen Jahren eine zunehmende Aufmerksamkeit erfahren, insbesondere bei Behandlung von Krebs und entzündlichen Erkrankungen (für Review siehe Murray und Norbury, 2000; Rivett und Gardner, 2000 - Kategory XXX = Literatur-Stellen nach dem Prioritätsdatum). Bislang ist der Einsatz von Proteasom-Inhibitoren für die breite klinische Anwendung am Menschen noch nicht zugelassen. Es mehren sich jedoch Berichte in der Fachliteratur, dass in jüngster Zeit die pharmazeutische Industrie intensiv an der Entwicklung von neuen Medikamenten auf der Basis von *in vivo*-verträglichen Proteasom-Inhibitoren arbeitet. Einige Beispiele seien hierzu angeführt: Die Firma "Millennium Pharmaceuticals, Inc." (Cambridge, MA 02139, USA) arbeitet nach Übernahme der Firma "ProScript, Inc." an der Entwicklung von Proteasom-Inhibitoren für entzündungshemmende, immunmodulatorische und antineoplasitsche Therapien, insbesondere an Borsäure-Derivaten von Di-Peptiden. Dabei spielen die Verbindungen PS-341 (Gardner *et al.,* 2000 Kategory XXX), PS-519 und PS-273 (Adams *et al.,* 1998, 1999) eine besondere Rolle.

Die orale Applikation von PS-341 hat im Ratten-Modell eine entzündungshemmende Wirkung in Streptokokken-induzierter Polyarthritis und Leber-Entzündung (Palombella *et al.,* 1998). Im Maus-Modell zeigt PS-341 anti-neoplastische Wirkung gegen Lungenkatzinom und hat außerdem eine additive Wirkung in Verbindung mit Zytostatika (Teicher *et al.,* 1999). *In vitro* Versuche demonstrieren eine sehr gute Wirksamkeit gegenüber soliden humanen Ovarien- und Prostata-Tumorzellen (Frankel *et al.,* 2000). Phase I klinische Studien an PS-341 beweisen eine gute Bio-Verfügbarkeit und pharmakokinetisches Verhalten (Lightcap *et al.,* 2000 Kategory XXX).

PS-341 ist der bislang einzige klinisch erprobte Proteasom-Inhibitor. Dazu wurden Phase I und Phase II klinische Studien in Patienten mit unterschiedlichen Krebserkrankungen, wie zum Beispiel hämatologischen Malignancies als soliden Tumoren bereits abgeschlossen. Die Informationen dazu wurden in verschiedenen Pressemitteilungen von Millennium Pharmaceuticals *Inc.* vorgestellt:
- Zum Beispiel wurden Ergebnisse über Phase II klinische Studien in Multiple Myeloma-Patienten berichtet (Presse-Mitteilung von Millennium Pharmaceuticals, Inc. von 01.03.01: "Millennium Initiates Phase II Clinical Trials of LDP-341 in Multiple Myeloma." Publiziert auf Webseite
   http:biz.yahoo.com/prnews/010301/neth003.html, Kategory XXX).
- Erste vorklinische Studien über die Wirkung von Proteasom-Inhibitoren PS-341 als neue anti-Krebsbehandlung in Myelom-Patienten wurde auf dem 42. Meeting der Amerikanischen Hämatologischen Gesellschaft, Dezember 2000, San Francisco, CA, USA, vorgestellte (Presse-Mitteilung von Millennium Pharmaceuticals, *Inc.* von 04.12.00: Millennium's LDP(PS)-341 inhibits growth and induces death of cancer cells, apperars to overcome chemotherapy resitance. Publiziert auf Webseite
   http:biz.yahoo.com/prnews/010301/neth003.html, Kategory XXX).
- Auf dem Meeting der Amerikanischen Gesellschaft für Klinische Onkologie, im Mai 2000, wurden Daten über Phase I klinische Studien mit PS-341 in Patienten mit fortgeschrittenen malignen Tumoren (einschließlich Melanoma, Nierenkarzimon, Lungenkarzinom, ProstataKrebs, Ovarien-Krebs, Blasen-Krebs, Cervix-Krebs, Endometrialen und Gallen-Tumoren) vorgestellt. Es wurde keine Dosis-limitierende Toxizität durch die Behandlung mit PS-341 beobachtet. Aufgrund der erstaunlichen Wirkung von PS-341 in Bezug auf anti-neoplastische Wirkung und Induktion von Apoptose konnten therapeutische Effekte in verschiedenen Tumor-Patienten beobachtet werden (Presse-Mitteilung von Millennium Pharmaceuticals, Inc. von 23.05.00: "Millennium presents clinical trial data on LDP-341 for advanced malignancies." Publiziert auf Webseite
   http://www.mlnm.com.releases.pr052300_l.shtml).
- Weitere Informationen über das klinische Protokoll von Phase I Studien in Patienten mit fortgeschrittenen Tumoren (solide Tumore als auch Lymphoma), welche auf Standard-Chemotherapie nicht mehr angesprochen haben, wurden im "CancerNet" online publiziert ("Phase I study of PS-341 in patients with advanced solid tumors or lymphomas". Publiziert am 11.09.00 auf Webseite http://cancernet.nci.nih.gov/, Kategory XXX).
- Ergebnisse über Phase I klinische Studien mit PS-341 in Patienten mit akuter Leukämie, Myelodysplastischem Syndrom und chronischer Myelider Leukämie wurden berichtet, dabei insbesondere die synergistische Wirkung von PS-341 mit Standard Chemotherapeutika (Presse-Mitteilung von Millennium Pharmaceuticals, *Inc.*" vom 09.11.00: "Phase I study of PS-341 in acute leukemias, myelodysplastic syndromes and chronic myeloid leukemia in blast phase." Publiziert online in Leukemia Insights Newsletter auf Webseite httw//www3ananderson.org/leukemia/insight/letter52.html).

In einem Maus-Modell konnte gezeigt werden, dass der Proteasom-Inhibitor PS-519 (ein β-Lacton-Derivat), entwickelt von der Firma Millennium Pharmaceuticals, *Inc.*, eine starke antiinflammatorische Wirkung ausübt, und zwar in beiden, der verzögerten als auch der übersensitiven Entzündungsreaktion. In niedrigen Dosen ist PS-519 auch wirksam in Kombination mit Steroiden. PS-519 wurde daher als ein neues Medikament für die Asthma-Behandlung vorgeschlagen (Elliott *et al.,* 1999). Eine weitere Anwendung für PS-519 ergibt sich im Infarkt-Modell: Die inflammatorische Reaktion nach cerebralen Verletzungen wurde durch PS-519 dramatisch reduziert. Danach scheint PS-519 ebenfalls ein interessantes Pharmakon für die Behandlung von Gehirnschlag zu sein (Phillips *et al.,* 2000, Kategory XXX).

Da Proteasom-Inhibitoren einen essentiellen Pathway im Zellmetabolismus treffen, ist ein strenges Dosis-Regime notwendig, um toxische Neben-Effekte zu unterdrücken. Im Rahmen der Entwicklung von *in vivo* verträglichen Proteasom-Inhibitoren wurden verschiedene Peptid-Borsäure-Derivate getestet, welche sowohl in der Zellkultur als auch im Tiermodell anti-Tumor Wirkung zeigten (Adams und Stein, 1996; Adams *et al.,* 1998, 1999, 2000). *In vitro* besitzt PS-341 eine selektive zytotoxische Aktivität gegen ein breites Spektrum an humanen Tumorzelllinien (Adams *et al.,* 1999). Diese Aktivität ist mit der Akkumulation von p21 und Zellzyklus-Arrest in der G2-M-Phase mit nachfolgender Apoptose verbunden (Adams *et al.,* 1999). Direkte Injektion von PS-341 bewirkte das Absterben von 70% der untersuchten Tumoren im Maus-Modell. Nach intravenöser Verabreichung von PS-341 verteilte sich die Substanz in allen Organen und Geweben und hatte anti-neoplastische Aktivität in humanen Xenograft-Modellen (Adams *et al.,* 1999).

Über den Einsatz von Proteasom-Inhibitoren jeglicher Substanzklasse mit dem Ziel, virale Infektionen zu blockieren, wurde bislang nicht berichtet.

### 2. Biologie von Hepatitisviren

### 2.1. Humane und Animale Hepatitis-B-Viren

Es sind neben dem humanen HBV eine Vielzahl von verwandten Tierviren bekannt, die zusammen die Familie der sogenannten Hepadnaviren bilden (für Review siehe Schäfer *et al.,* 1998). Gemeinsam ist diesen Viren die Synthese einer pregenomischen RNA von einer zirkulären supergecoilten Form des Genomes (cccDNA) im Zellkern, Verpackung einer prägenomischen RNA im Zytoplasma in Nukleokapside, Überschreibung der prägenomischen RNA innerhalb des Kapsids in eine zirkuläre partiell doppelsträngige DNA-Form (ocDNA) mit Hilfe der Virus-kodierten reversen Transkriptase mit DNA-Polymerase Aktivität während der Virusreifung und -ausschleusung. Wegen der zahlreichen Gemeinsamkeiten mit HBV werden die animalen Hepadnaviren sehr häufig als Tiermodelle zur Erforschung der Biologie, Pathogenese und Evaluation von antiviralen Substanzen für die Therapie der humanen Hepatitis B verwendet (für Review siehe Schäfer *et al.,* 1998). Deshalb wurde in der vorliegenden Erfindungsbeschreibung insbesondere das Entenliepatitis-B-Virus-Tiermodell für Studien mit Proteasom-Inhibitoren eingesetzt.

### 2.1.1. Hepadnavirale Viruspartikel und Komponenten

Im Serum von virämischen Patienten und Tieren findet man neben dem infektiösen Virion (ca. 42 nm Durchmesser) in der Regel 1.000 bis 10.000 mehr subvirale, nicht infektiöse Partikel von sphärischer oder filamentöser Form. Die viralen Partikel bestehen aus einer Lipidhülle, in welche HBV-Genom-kodierte Oberflächenproteine (HBs oder S, PreS1 oder large S, PreS2 oder middle S) eingelagert sind. Das Nukleokapsid besteht aus dem Nukleokapsidprotein und enthält das partiell doppelsträngige Virusgenom sowie zelluläre Proteine. Dazu gehören unter anderem zelluläre Kinasen.

### 2.1.2. Die frühen Infektionsvorgänge bei Hepadnaviren

Nach Bindung der viralen Partikel an Oberflächenmoleküle der Hepatozyten und andere Zellen hepatischen und nicht-hepatischen Ursprungs wird das Virus über wenig verstandene Mechanismen in die Zellen und das Virusgenom in den Zellkern transportiert. Jeder der Vorgänge der frühen Schritte der Infektion, bei denen die Wechselwirkung mit zellulären Komponenten eine Rolle spielt, könnte in Proteasom-behandelten Zellen gestört sein und so die fehlende oder geringere Infektiosität von Virionen erklären, welche in der vorliegenden Erfindung überraschenderweise festgestellt wurde. Während oder nach Eintritt des viralen Genoms in den Zellkern wird es zu einem komplett doppelsträngigen supergecoilten DNA-Genom (cccDNA) umgewandelt. Von der cccDNA werden alle viralen RNAs synthetisiert. n subgenomischen RNA synthetisiert. Die Genomüberlängen-RNA ist terminal redundant und wird aAußer der Prägenom-RNA werden subgenomische RNAs synthetisiert, von denen die Struktur- und Nicht-Struktur-Proteine translatiert werden.

### 2.1.3. Die späten Schritte der Hepadnavirus-Replikarion

Phosphorylierung und Dephosphorylierung des Coreproteins spielen eine wichtige Rolle für den Zusammenbau des Nukleokapsids, für die DNA-Synthese, für die Assoziation der Nukleokapsidproteine an die Kemmembran und deren Transport in den Zellkern, sowie für den Zerfall des Nukleokapsids, welcher notwendig ist, um das Genom in den Zellkern zu transportieren. Veränderungen in der Phosphorylierung des Nukleokapsids können mit der Infektiosität der Hepadnaviren und dem Infektionsvorgang interferieren. Hat die DNA-Synthese einen bestimmten Reifezustand errreicht, kommt es zur Umhüllung des Virus. Ein Teil der Nukleokapside wandert zur Kernmembran und sorgt so für die notwendige Erhöhung der Kopienzahl der cccDNA.

### 2.1.4 Das Problem der Heterogenität von Hepadnaviren

Wegen des Schrittes der reversen Transkription des HBV-Genoms in DNA und des Fehlens einer Proofreadingfunktion der reversen Transkriptase entstehen während der Vermehrung von HBV häufig Mutationen, ähnlich wie es für HIV- und andere RNA-Viren einschließlich HCV bekannt ist (für Review siehe Günther *et al.,* 1998). Deshalb sind Patienten immer mit einer sehr heterogenen Population von HBV und HCV infiziert. Die Heterogenität beeinflußt die Pathogenese, die Virusresistenz, das Ansprechen auf die Therapie mit Interferonen (IFN) und antiviralen Substanzen (Nukleosidanaloge und andere) sowie die Erkennung der infizierten Zellen durch das Immunsystem. Diese Befunde belegen, dass neue antivirale Strategien für die Verhinderung einer *de novo* HBV-Infektion und vor allem die Therapie einer chronischen Infektion notwendig und sinnvoll sind, trotz der Verfügbarkeit einer Vakzine und prophylaktisch verabreichbarer Antikörper.

### 2.2. Möglichkeiten der Therapie des Immunschutzes einer HBV-Infektion

Eine der wenigen Therapiemöglichkeiten einer chronischen HBV-Infektion ist die Behandlung mit Interferonen (IFN). Diese ist anerkannt, zugelassen, aber nur zumindest teilweise wirksam. Bei Koinfektionen mit HIV und HCV, die relativ häufig vorkommen, ist die Erfolgsrate einer IFN-Behandlung noch geringer als bei alleiniger HBV- oder HCV-Infektion. Alle Reservoirs an HBV und HCV werden selbst bei klinisch erfolgreicher IFN-Behandlung so gut wie nie eliminiert und können zu einer Reaktivierung der Infektion führen (Rehermann *et al.,* 1996). Wesentlicher Nachteil einer anti-HBV-Therapie auf der Basis von IFN-Gabe ist, dass diese häufig mit negativen Nebenwirkungen assoziiert ist (für Review siehe Trautwein und Manns, 2001).
Die für HBV eingesetzten Nukleosidanaloga hemmen das Umschreiben der prägenomischen RNA in DNA durch die Virus-kodierte Poylmerase. Ein großer Nachteil von Nukleosidanaloga (z.B. Lamivudine, Famcyclovir, Adevofir und Entecavir) ist, dass es so gut wie immer zur Selektion von Medikamenten-resistenten HBV-Stämmen kommt. Außerdem bergen Nukleosidanaloga die Gefahr, dass sie chromosomale Mutationen und daher Krebs auslösen können. Die im Rahmen der hier vorgestellten Erfindung genannten neuen Medikamente sind nicht mit diesem Risiko und den Nebenwirkungen behaftet.

### 2.3. Biologie und Behandlung von HCV-Infektionen

Auch die Infektion mit Hepatitis-C-Virus (EICV) ist eines der großen Weltgesundheitsprobleme. Mit diesem Virus sind ca. 170 Millionen Menschen (d.h. ca. 3%) der Weltbevölkerung infiziert. Manche Länder haben eine Durchseuchung mit HCV von über 10% der Bevölkerung. Im Gegensatz zu HBV gibt es bisher keine effektiven Impfstoffe für HCV. Die HCV-Infektion verläuft in ca. 80% der Fälle chronisch mit unterschiedlichem Schweregrad von Leberentzündungen. Ähnlich wie bei HBV ist auch die chronische HCV-Infektion mit einem sehr hohen Risiko der Entstehung von Leberzirrhose und Leberkarzinom verbunden. Für beide Erkrankungen gibt es kaum Heilungschancen außer einer erfolgreichen Lebertransplantation. HCV gehört zu den Flaviviren und kodiert ca. 10 Genprodukte. Die Infektion wird in der Regel durch Bestimmung der spezifischen anti-HCV-Antikörper, der viralen Antigene und RNA diagnostiziert. Ähnlich wie bei HBV ist die Pathogenese, die sich durch unterschiedliche Grade der Leberentzündung bis hin zum Leberversagen, der Entstehung einer Lebeizirrhose/Fibrose und eines Leberkarzinoms sowie Begleiterkrankungen auszeichnet. Auch die Therapie beruht ähnlich wie bei HBV vorwiegend auf der Behandlung mit Interferon alpha und Derivaten sowie mit Nukleosidanalogen und weiteren Substanzen unbekannter Wirkweise (für Review siehe Trautwein und Manns, 2001). Seit 1999 ist für die Therapie der chronischen HCV das Guanosinanalogon Ribavirin in Kombination mit Interferonen zugelassen. Die Wirkungsweise dieses Medikaments ist jedoch nur unvollständig geklärt. Eine vollständige Eliminierung von HCV ist bei der Gabe von Ribavirin nicht zur erwarten. Ribavirin hat außerdem häufig eine Reihe von Nebenwirkungen (für Review siehe Trautwein und Manns, 2001). Für alle gegenwärtig zugelassenen Medikamente für HBV und HCV sowie HDV (Hepatitis-D-Virus) gilt, dass es eine Vielzahl von Nonrespondem gibt, denen nur grundsätzlich neue Medikamente helfen können, wie sie in dieser Erfindung beschrieben werden.

### 2.4. Verbindung zwischen dem UPS und dem Replikationszyklus von Hepadnaviren

Für eines der regulatorischen Proteine von HBV, das HBx Protein, wurde gezeigt, dass es mit einer Untereinheit des 26S Proteasom-Komplexes interagiert und dass diese Interaktion für die Funktion von HBx essentiell ist (Hu *et al.,* 1999). Weiterhin wurde berichtet, dass die Präsentation von HBV und HCV antigenen Determinanten in Form von MHC Klasse I Peptid-Komplexen auch in Gegenwart von Proteasom-Inhibitoren effizient erfolgen kann (López *et al.,* 2000). Die Erkennung von HBV-infizierten Leberzellen durch zytotoxische T_{CD8+}-Zellen und damit die zelluläre natürliche Immunität gegen eine HBV-Infektion wäre somit durch eine Therapie mit Proteasom-Inhibitoren nicht beeinflusst, wie dies in der vorliegenden Erfindungsbeschreibung vorgeschlagen wird.

### 2.4.1. Funktion des UPS in der Replikation von HCV

Die Expression von HCV-Proteinen beeinflusst nicht wie bei HBV die Aktivität des UPS (Moradpour *et al.,* 2001). Diese Ergebnisse lassen vermuten, dass die MHC-Klasse I Antigenpräsentation und die Prozessierung von viralen Antigenen von HCV nicht beeinflusst werden und somit andere Immunescape-Mechanismen für die Etablierung einer persistenten HCV notwendig sind. Eine Fraktion des HCV core-Proteins selbst wird *via* UPS abgebaut, und eine mono-ubiquitinierte Form des core-Proteins wurde beobachtet (Suzuki *et al.,* 2001).

### 2.4.2. Funktion des UPS in der Replikation von HCC

Bisher wurde nicht getestet, ob Proteasom-Inhibitoren einen Einfluss auf die Proliferation oder den Transformationstatus von hepatozellulären Karzinomen (HCC) haben. Lediglich bekannt ist, dass eine Proteasom-Untereinheit in den meisten HCCs überexprimiert wird (Higashitsuji *et al.,* 2000).
Anhand des bekannten Standes kann festgehalten werden, dass die überraschenderweise festgestellte anti-virale Wirkung von Proteasom-Inhibitoren auf späte Prozesse der Retrovirusreplikation, wie zum Beispiel die Gag-Prozessierung, die Assemblierung und das Budding von HTV-1 oder HIV-2 Virionen sowie für die Produktion von infektiösen Nachkommen-Viren bzw. für den gesamten Virusreplikationszyklus bisher nicht berichtet wurde. Ebenso liegen keine Berichte über die Verwendung von Proteasom-Inhibitoren zur Behandlung von Infektionen mit HIV oder anderen Retroviren vor. Weiterhin kann festgestellt werden, dass in keiner der bislang in der Fach- oder Patentliteratur publizierten Studien oder anderen bis zum gegenwärtigen Zeitpunkt publizierten Arbeiten getestet oder berichtet wurde, dass Proteasom-Inhibitoren einen Einfluss auf die Freisetzung und die Infektiosität von Hepatitisviren ausüben, wie dies in der vorliegenden Erfindungsbeschreibung dargestellt wird. Insbesondere wurden keinerlei anti-virale Effekte durch Proteasom-Inhibitoren auf die Prozesse des HBV-Replikationszyklus ermittelt. Weiterhin wurde nicht berichtet, dass Proteasom-Inhibitoren bevorzugt durch Heptatis-Infektionen hervorgerufene Leberkarzinomzellen abtöten und dadurch für die Therapie von Leberkarzinomen geeignet sind. Die erfindungsgemäß dargestellten Wirkungen von Proteasom-Inhibitoren auf frühe und späte Prozesse der HBV-Replikation wie auch auf die Entwicklung von sekundärer Leberzirrhose und Leberkarzinomen stellen somit völlig neuartige Prinzipien der anti-viralen Behandlung von HBV-Infektionen dar. Die Verwendung von Proteasom-Inhibitoren in der anti-viralen Therapie von Hepatitis-Infektionen, speziell zur Verhinderung der Etablierung oder der Aufrechterhaltung einer akuten und chronischen HBV- und HCV-Infektion wurde bislang nicht berichtet.

Folgende Patentschriften, welche die vorliegende Erfindung nicht unmittelbar betreffen, wurden veröffentlicht: Eine Erfindung, welche Mittel zur Interferenz von HBV-Infektionen auf der Basis von HBV-Protein X Wechselwirkung mit Proteasom-Untereinheiten beschreibt (US 5872206); ein Verfahren zur Bestimmung der Proteasom-Aktivität in biologischen Proben (WO 00/23614); die Verwendung von Proteasom-Inhibitoren als Mittel zur Behandlung von Krebs, Entzündungen und Autoimmunerkrankungen (WO 99/22729); die Verwendung von Inhibitoren des UPS als Mittel zur Behandlung von Entzündungen und Autoimmunerkrankungen (WO 99/15183).

### Wesen der Erfindung

Der Erfindung liegt die Aufgabe zugrunde, Mittel zur Verfugung zu stellen, die zur Behandlung. Therapie und Hemmung einer Virushepatitis verwendet werden können.
Die Aufgabe wurde durch den Einsatz von mindestens einem Proteasom-Inhibitor gelöst. Es sind erfindungsgemäß Mittel zur Behandlung von Virus-Infektionen entwickelt worden, die als wirksame Komponenten Proteasom-Inhibitoren in pharmazeutischen Zubereitungen enthalten. Die Erfindung bezieht sich auf Virus-Infektionen insbesondere solcher Viren, die im Rahmen des Replikationszyklus von der Zelloberfläche freigesetzt werden. Gemäß einer bevorzugten Ausführungsform der Erfindung werden als Proteasom-Inhibitoren Substanzen eingesetzt, welche die Aktivitäten des Ubiquitin/Proteasom-Pathway hemmen, regulieren oder anderweitig beeinflussen.
Es ist auch möglich, dass als Proteasom-Inhibitoren Substanzen eingesetzt werden, die speziell die enzymatischen Aktivitäten des kompletten 26S Pmteasom-Komplexes und der freien, nicht mit regulatorischen Untereinheiten assemblierten 20S katalytisch aktiven Proteasom-Struktur beeinflussen. Diese Inhibitoren können entweder eine oder mehrere oder alle drei hauptsächlichen proteolytischen Aktivitäten des Proteasoms (die Trypsin-, die Chymotrypsin- und die Postglutamyl-Peptid hydrolysierenden Aktivitäten) innerhalb des 26S- oder auch des 20S-Proteasom-Komplexes hemmen.

Eine Variante der Erfindung besteht darin, als Proteasom-Inhibitoren Substanzen einzusetzen, die von Zellen höherer Eykaryoten aufgenommen werden und nach Zellaufnahme mit der katalytischen beta-Untereinheit des 26S-Proteasoms in Wechselwirkung treten und dabei alle oder einzelne der proteolytischen Aktivitäten des Proteasom-Komplexes irreversibel oder reversibel blockieren.

Als eine weitere Form der Erfindung kommen Mittel zum Einsatz, welche die Aktivitäten der Ubiquitin-konjugierenden wie auch der Ubiquitin-hydrolysierenden Enzyme hemmen. Dazu gehören auch zellulären Faktoren, die mit Ubiquitin - als Mono- oder auch als Poly-Ubiquitin - in Wechselwirkung treten. Poly-Ubiquitinierung gilt allgemein als ein Erkennungssignal für die Proteolyse durch das 26S-Proteasom, und die Beeinflussung des Ubiquitin-Pathway kann ebenfalls die Aktivität des Proteasoms regulieren.
Erfindungsgemäß werden als Proteasom-Inhibitoren auch Substanzen eingesetzt, die in verschiedenen Formen *in vivo* oral, intravenös, intramuskulär, subkutan, in verkapselter Form mit oder ohne Zellspezifität-tragende Veränderungen, oder anderweitig verabreicht werden, aufgrund der Anwendung eines bestimmten Applikations- und Dosis-Regimes eine geringe Zytotoxizität und/oder hohe Selektivität für bestimmte Zellen und Organe aufweisen, keine oder unbedeutende Nebenwirkungen auslösen, eine relativ hohe metabolische Halbwertszeit und eine relativ geringe Clearence-Rate im Organismus aufweisen.
Als Proteasom-Inhibitoren werden des weiteren Substanzen eingesetzt, die in natürlicher Form aus Mikroorganismen oder anderen natürlichen Quellen isoliert werden, durch chemische Modifikationen aus natürlichen Substanzen hervorgehen oder total-synthetisch hergestellt werden oder durch gentherapeutische Verfahren *in vivo* synthetisiert oder durch gentechnische Verfahren in vitro oder in Mikroorganismen hergestellt werden. Dazu gehören:
a) natürlich vorkommende Proteasom-Inhibitoren:
   - Epoxomicin (Epoxomycin) und Eponemycin,
   - Aclacinomycin A (auch bezeichnet als Aclarubicin),
   - Lactacystin und dessen chemisch modifizierte Varianten, insbesondere die Zellmembranpenetrierende Variante "Clastolactacystein β-Lacton",
b) synthetisch hergestellte :
   - modifizierte Peptidaldehyde wie zum Beispiel N-carbobenzoxy-L-leucinyl-L-leucinyl-L-leucinal (auch bezeichnet als MG132 oder zLLL), dessen Borsäure-Derivat MG232; N-carbobenzoxy-Leu-Leu-Nva-H (bezeichnet als MG115); N-Acetyl-L-Leuzinyl-L-Leuzinyl-L-Norleuzinal (bezeichnet als LLnL); N-carbobenzoxy-Ile-Glu(OBut)-Ala-Leu-H (auch bezeichnet als PSI);
   - Peptide, die C-terminal α,β-Epoxyketone (auch bezeichnet als Epoxomicin / Epoxomycin oder Eponemycin), Vinyl-sulphone (zum Beispiel Carbobenzoxy-L-Leueinyl-L-Leucinyl-L-Leucin-vinyl-sulfon oder 4-Hydroxy-5-iodo-3-nitrophenylactetyl-L-Leucinyl-L-Leucinyl-L-Leucin-vinyl-sulfon, auch bezeichnet als NLVS), Glyoxal oder Borsäure-Reste (zum Beispiel Pyrazyl-CONH(CHPhe)CONH(CHisobutyl)B(OH)₂), auch bezeichnet als "PS-431" oder Benzoyl(Bz)-Phe-boroLeu, Phenacetyl-Leu-Leu-boroLeu, Cbz-Phe-boroLeu); Pinacol-Ester - zum Beispiel Benzyloxycarbonyl (Cbz)-Leu-Leu-boroLeu-Pinacol-Ester - tragen;
      und
   - als besonders geeignete Verbindungen werden Peptide und Peptid-Derivate eingesetzt, welche C-terminal Epoxyketon-Strukturen tragen, hierzu zählen beispielsweise Epoxomicin (Molekülformel: C₂₈H₈₆N₄O₇) und Eponemycin (Molekülformel: C₂₀H₃₆N₂O₅);
   - chemisch modifizierte Derivate auf der Basis von natürlich vorkommenden, insbesondere ein β-Lacton-Derivat mit der Bezeichnung PS-519 (1*R*-[1*S*,4*R*,5*S*]]-1-(1-Hydroxy-2-methylpropyl)-4-propyl-6-oxa-2-azabicyclo[3.2.0]heptane-3,7-dione, Molekülformel: C₁₂H₁₉NO₄), welches sich von dem natürlichen Proteasom-Inhibitor Lactacystin ableitet;
   - bestimmte Dipeptidyl-Bocsäure-Derivate, insbesondere Verbindungen, welche sich von dem Pyranozyl-Phenyl-Leuzinyl-Borsäure-Derivat mit dem Namen "PS-341" (N-Pyrazinecarbonyl-L-Phenylalanin-L-leuzin-Borsäure, Molekülformel: C₁₉H₂₅BN₄O₄) ableiten. Hierzu zählen weiterhin die Verbindungen "PS-273" (Morpholin-CONH-(CH-Naphtyl)-CONH-(CH-isobutyl)-B(Offl₂) und dessen Enantiomer PS-293, die Verbindung PS-296 (8-Quinolyl-sulfonyl-CONH-(CH-Naphthyl)-CONH(-CH-isobutyl)-B(OH)₂); die Verbindung PS-303 (NH₂(CH-Naphtyl)-CONH-(CH-isobutyl)-B(OH)₂); die Verbindung PS-321 (Morpholin-CONH-(CH-Naphthyl)-CONH-(CH-Phenylalanin)-H(OH)₂); die Verbindung PS-334 (CH₃-NH-(CH-Naphthyl-CONH-(CH-Isobutyl)-B(OH)₂); die Verbindung PS-325 (2-Quinol-CONH-(CH-*homo*-Phenylalanin)-CONH-(CH-isobutyl)-B(OH)₂); die Verbindung PS-352 (Phenyalanin-CH₂-CH₂-CONH-(CH-Phenylalanin)-CONH-(CH-isobutyl)1-B(OH)₂); die Verbindung PS-383 (Pyridyl-CONH-(CH*p*F-Phenylalanin)-CONH-(CH-isobutyl)-B(OH)₂. Alle diese Verbindungen wurden bereits beschrieben, unter anderem in Adams *et al.* (1999).
Als besonders geeignete Verbindungen haben sich, neben Epoxomicin und Eponemycin, die Proteasom-Inhibitoren PS-519, PS-341 und PS-273 (entwickelt von der Firma Millennium Pharmaceuticals Inc., Cambridge, MA 02139, USA) erwiesen. Diese Proteasom-Inhibitoren sind sehr potent, sehr spezifisch für das Proteasom, blockieren keine anderen zellulären Proteasen und haben daher so gut wie keine Nebenwirkungen. Die Proteasom-Inhibitoren PS-341 und PS-519 wurden außerdem sowohl in Tiermodellen für vorklinische als auch in Menschen (Krebspatienten) für klinische Studien getestet.
Mit den Proteasom-Inhibitoren werden erfindungsgemäß Mittel zur Verfügung gestellt, die überraschenderweise
- die Freisetzung von infektiösen Hepatitisviren, insbesondere von HBV und HCV, aus infizierten Zellen blockieren;
- die Ausbreitung einer akuten Infektion mit Hepatitisviren begrenzen;
- das bevorzugte Absterben von Leberkarzinomzellen bewirken;
- die Virämie sowohl bei einer Neuinfektion als auch bei chronischen Infektionen mit Hepatitisviren unterdrücken und den Erfolg einer Viruseliminierung durch das eigene Immunsystem und/oder durch bekannte Mittel mit ähnlicher oder anderer Wirkung erhöhen.

Die Blockierung der Virusreplikation in Zellkulturen kann mechanistisch als Ergebnis der im Rahmen der Erfindung beschriebenen neuartigen Aktivitäten von Proteasom-Inhibitoren wie folgt begründet werden:
- In Gegenwart der Proteasom-Inhibitoren werden weniger Virionen freigesetzt,
- die wenigen freigesetzten Virionen habe eine geringe oder gar keine Infektiosität
Dadurch wird die Neuinfektion von Zellen in der Kultur eingeschränkt, und die Ausbreitung der Infektion ist daher vermindert oder bricht vollständig ab.
Das Wesen der Erfindung liegt in der Verwendung bekannter Mittel zu einem neuen Zweck und in einer Kombination bekannter Elemente - den Proteasom-Inhibitoren - und einer neuen Wirkung - ihrem Einsatz zur Beeinflussung von Hepadnaviren - die in ihrer neuen Gesamtwirkung einen Vorteil und den erstrebten Erfolg ergeben, der darin liegt, dass nunmehr Mittel zur Hemmung der Freisetzung und Reifung von Hepadnaviren sowie Mittel zur Behandlung, Therapie und Hemmung einer Virushepatitis zur Verfügung stehen.

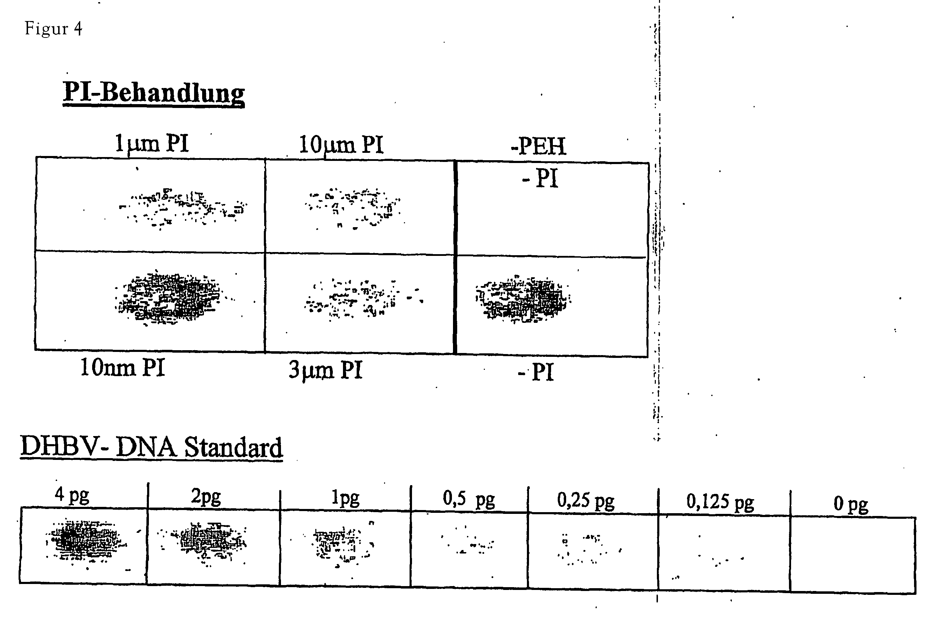

Es wird überraschenderweise festgestellt, dass ähnlich zu dem Effekt auf Retroviren, Proteasom-Inhibitoren späte Prozesse im Replikationszyklus von Hepadnaviren hemmen. Dabei wurde spezifisch beobachtet, dass sich die erfindungsgemäße Verwendung von Proteasom-Inhibitoren dazu eignet, die Produktion von infektiösen Virionen von chronisch mit HBV infizierten Zellen weitgehend oder vollkommen zu unterbinden. Nach Behandlung von HBV-produzierenden Zellen mit Proteasom-Inhibitoren tritt sowohl eine Hemmung der Freisetzung von Virionen als auch eine nahezu vollständige Vergleich zu zLLL ist der sehr spezifische Proteasom-Inhibitor Epoxomicin sehr viel potenter in seiner Wirkung auf die Proteasom-Aktivität. Dies widerspiegelt sich in der erfindungsgemäß erhaltenen Beobachtung, dass 100 nanoM Epoxomicin eine vollständige sowie 10 nanoM Epoxomicin eine fast vollständige Blockade der HIV-1 Replikation bewirken (Fig. 4, Panel 4. Experiment in A3.01).
Die Blockierung der Virusreplikation in Zellkulturen kann mechanistisch als Ergebnis der im Rahmen der Erfindung beschriebenen neuartigen Aktivitäten von Proteasom-Inhibitoren wie folgt begründet werden:
- In Gegenwart der Proteasom-Inhibitoren werden weniger Virionen freigesetzt,
- die wenigen freigesetzten Virionen habe eine geringe oder gar keine Infektiosität.
Dadurch wird die Neuinfektion von Zellen in der Kultur eingeschränkt, und die Ausbreitung der Infektion ist daher vermindert oder bricht vollständig ab.
Das Wesen der Erfindung liegt in der Verwendung bekannter Mittel zu einem neuen Zweck und in einer Kombination bekannter Elemente - den Proteasom-Inhibitoren - und einer neuen Wirkung - ihrem Einsatz zur Beeinflussung von Retroviren und von Hepadnaviren - die in ihrer neuen Gesamtwirkung einen Vorteil und den erstrebten Erfolg ergeben, der darin liegt, dass nunmehr Mittel zur Hemmung der Freisetzung und Reifung von Retroviren sowie Mittel zur Behandlung, Therapie und Hemmung einer Virushepatitis zur Verfügung stehen.
Die Erfindung richtet sich ferner auf die Verwendung von Proteasom-Inhibitoren zur Herstellung von Mitteln zur Hemmung der Freisetzung, Reifung und Replikation von Retroviren. Dazu gehört ihre Verwendung zur Herstellung von Arzneimitteln zur Behandlung und Prophylaxe von AIDS und damit verwandter pathologischer Erscheinungen einer HIV-Infektion, wie zum Beispiel der HIV-induzierten Demenz, der HIV-induzierten Störungen im Lipidstoffwechsel, speziell dem HLS Syndrom (HIV-associated lipodystrophy syndrome) sowie von HIV-induzierten Störungen der Nierenfunktionen, speziell dem HIVAN Syndrom (IUV associated nephropathy).

In einem weiteren Teil, dem zweiten Schwerpunkt der Erfindung, wird überraschenderweise festgestellt, dass ähnlich zu dem Effekt auf Retroviren, Proteasom-Inhibitoren späte Prozesse im Replikationszyklus von Hepadnaviren hemmen. Dabei wurde spezifisch beobachtet, dass sich die erfindungsgemäße Verwendung von Proteasom-Inhibitoren dazu eignet, die Produktion von infektiösen Virionen von chronisch mit HBV infizierten Zellen weitgehend oder vollkommen zu unterbinden. Nach Behandlung von HBV-produzierenden Zellen mit Proteasom-Inhibitoren tritt sowohl eine Hemmung der Freisetzung von Virionen als auch eine nahezu vollständige Reduktion der Infektiosität der freigesetzten Virionen ein. In Folge dieser neuartigen Aktivitäten können Proteasom-Inhibitoren die Virusreplikation und somit die Neuinfektion von Hepatozyten und damit die Ausbreitung einer HBV-Infektion *in vivo* in dem Lebergewebe eines HBV-Infizierten unterdrücken.
Ebenfalls wurde festgestellt, dass die Behandlung von chronisch mit HBV infizierten Hepato-Karzinomzellen mit Proteasom-Inhibitoren bevorzugt das Absterben (hauptsächlich durch Induktion von Apoptose) dieser Krebszellen induziert, während gesunde, primäre Hepatozyten. und die anderen nicht proliferierenden Leberzellen sehr viel resistenter gegenüber einer Behandlung mit Proteasom-Inhibitoren sind. Leberkarzinome sind medikamentös kaum behandelbar und führen in der Regel ohne Lebertransplantation oder Leberresektion zum Tod. Proteasom-Inhibitoren erlangen dadurch ein weiteres therapeutisches Potential für die Behandlung von Hepatitisvirus-Infektionen: Durch die Behandlung mit Proteasom-Inhibitoren kann nicht nur die Ausbreitung der Infektion (durch Blockierung der Produktion infektiöser Virionen), sondern auch die mit der Infektion verbundene Entstehung von Leberzellkarzinomen unterdrückt, verhindert oder ein bereits etabliertes Leberzellkarzinom geheilt werden. Dieser Anspruch beruht auf der Tatsache, dass die Behandlung mit Proteasom-Inhibitoren - ähnlich der bereits bekannten anti-neoplastischen Wirkung von Proteasom-Inhibitoren auf eine Vielzahl von Tumoren - eine spezifische Eliminierung von Leberkarzinomzellen *in vivo* bewirken kann. Die anti-neoplastische Wirkung von Proteasom-Inhibitoren wurde bislang nicht für Leberzellkarzinome gezeigt und stellt daher ein neuartiges therapeutisches Prinzip dar. Proteasom-Inhibitoren können somit zur Behandlung / Bekämpfung / Verhinderung von HBVinduzierter Leberzirrhose, insbesondere primären Leberzellkarzinomen eingesetzt werden.
Weiterhin können Proteasom-Inhibitoren unter Verwendung der neuartigen anti-viralen Wirkung für die Behandlung von folgenden symptomatischen und symptomlosen Hepatitisvirus-Infektionen eingesetzt werden: Hepatitis-A-Virus (HAV), Hepatitis-C-Virus (HCV), Hepatitis-Delta-Virus (HDV), Hepatitis-E-Virus (HEV), Hepatitis-F-Virus (HFV), Hepatitis-G-Virus (HGV). Die Behandlung der Hepatitis B und C mit Proteasom-Inhibitoren ist wegen der weiten Verbreitung, der besonders hohen Pathogenität sowie wegen der Assoziation der chronischen Infektion mit der Entwicklung eines Leberkarzinoms von besonderer Bedeutung.

Die Proteasom-Inhibitoren können auch in Kombination mit anderen anti-Hepatitis-Medikamenten und sonstigen Therapieschemata eingesetzt werden, z.B. Interferon alpha/beta/gamma und Varianten hiervon (zum Beispiel pegylierte Interferone), Interleukine, Nukleosidanaloga (Lamivudine, Cidovir, Ribavirin und andere), Steroide, Plasma-Austausch, Thymosin alpha 1, Impfstoffe, passive und aktive Vakzinierung, therapeutische und prophylaktische Vakzinierung, Glycyrrhizin, Stammzell-transplantation, Organtransplantationen, Nahrungstherapie, Immunsuppressiva, Cyclosporine und Derivate hiervon, Amanditin und Derivate, Interleukine und andere Cytokine, nicht Proteasom-selektive Protease-Inhibitoren, Azathoprin, Hämodialyse, sowie hoch aktive antiretrovirale Therapie (highly active antiretroviral therapy, "HAART") bei Co-Infektionen von HBV mit Humanen Immundefizienzviren (HIV). Da Proteasom-Inhibitoren auch anti-virale Wirkung auf HIV ausüben, ist eine Behandlung von HBV/HIV-Koinfektionen, insbesondere in Kombination mit einer HAART-Therapie, ein Anwendungsschwerpunkt der Erfindung.

Eine Verhinderung des Krankheitsausbruches und eine Reduzierung der Infektionsausbreitung im Organismus von symptomlosen HBV-infizierten Personen ist erfindungsgemäß ebenfalls mit Proteasom-Inhibitoren möglich.

Eine weitere Verwendung von Proteasom-Inhibitoren ist die Verhinderung der Etablierung einer systemischen Hepatitisvirus-Infektion unmittelbar nach Kontakt mit infektiösem Virus (zum Beispiel bei Nadel-Stich-Verletzungen mit Virus-kontaminiertem Blut oder Blutprodukten).
Eine weitere Verwendung von Proteasom-Inhibitoren ist die Vorbeugung einer Hepatitisvirus-Iinfektion bei Personen mit hohem Risiko einer Neuinfektion, zum Beispiel bei Ärzten, Risiko-Personal in Häusern mit hohem Besucherverkehr, Drogenabhängigen, Reisenden in hochendemische Gebiete für Hepatitis-Viren, in der Krankenbehandlung, für Familienangehörige von chronischen Virusträgern.
Eine weitere Verwendung von Proteasom-Inhibitoren ist die Verhinderung einer Re-Infektion mit HBV bei Leber- und anderen Organtransplantationen sowie bei Zelltherapien durch Gabe der Mittel vor, während und einige Zeit nach der Transplantation. Die Gabe dieser Mittel ist angezeigt sowohl für die Hochrisikosituation bei der Transplantation von virusfreien Organen auf chronische Virusträger, die immer Restvirus haben, welche die neuen Organe infizieren können, als auch für die Übertragung von Virus-haltigen Organen von Spendern auf virusfreie Patienten.
Die Prinzip-Lösung der Aufgabe wird am Beispiel von DHBV gezeigt. Es wird dargestellt, dass nach Zugabe von verschiedenen Substanzklassen von Proteasom-Inhibitoren die Produktion von infektiösen Viruspartikeln aus bereits infizierten Zellen gehemmt wird.

### Proteasom-Inhibitoren blockieren Freisetzung von infektiösen Viruspartikeln aus chronisch DHBV-infizierten Zellen

Erfindungsgemäß wird dieses Phänomen sowohl am Beispiel der Nichtinfizierbarkeit von primären Hepatozyten (Ente, Murmeltier, Tupaia und Mensch), von Gallengangszellen, von Mischkulturen aus Hepatozyten und Nichthepatozyten, von Zellen des hämatopoetischen System, als auch von etablierten Hepatomazellen mit Hepatitis B-, C- und D-Viren aus Proteasom-Inhibitoren-behandelten Zellen dargestellt. Darüber hinaus wird dies durch analoge Infektionsversuche in Tiermodellen *in vivo* dargestellt (uPA/RAG2-Mausmodell repopuliert mit Leberzellen aus Mensch, Murmeltier und Tupaia; mit Enten, Murmeltieren und Tupaias). Die Vorgehensweise ist, dass HBV-, HCV-, HDV-Viruspartikel und Kombinationen hiervon aus den Medien von Produzentenzellen, welche für unterschiedliche Zeiten und Dosen mit Proteasom-Inhibitoren behandelt wurden, geerntet werden und die Infektiosität der Viruspartikel durch Infektion von Hepatitisvirus-freien Zellen getestet wird. Hierzu werden die oben genannten Zellen mit den Viruspartikeln inkubiert und anschließend die Infektion oder das Ausbleiben der Infektion durch Analyse der intra- und extrazellulären Komponenten von Nachkommenviren überprüft. Konkret wird der Status der Neusynthese von viralen Antigenen wie zum Beispiel Oberflächenproteine und Nukleokapsidprotein mittels Immunoblot, ELISA und metabolische Markierung getestet. Ebenfalls werden die Virusnukleinsäuren (RNA durch Northern, DNA durch Southern blots sowie PCR- und RT-PCR-Analysen) analysiert. Außerdem werden die viralen Antigene durch indirekte Immunfluoreszenzfärbung mit Hilfe von Virus-spezifischen Antikörpern mikroskopisch nachgewiesen.
Erfindungsgemäß wird am Beispiel von chronisch mit DHBV infizierten Entenhepatozyten gezeigt, dass nach Behandlung mit Proteasom-Inhibitoren die Lebensfähigkeit der primären Hepatozyten nicht wesentlich beeinträchtigt ist, ebenso ist die Proteinsynthese in diesen primären Zellen nicht wesentlich blockiert. Jedoch wird im Rahmen der Erfindungsbeschreibung festgestellt, dass die freigesetzten Virionen faktisch nicht infektiös sind und daher keine Neuinfektion in primären Hepatozyten auslösen können. Im Ergebnis der Erfindung wird damit gezeigt, dass die Inhibierung des Proteasom-Pathway mittels Proteasom-Inhibitoren die Produktion von infektiösen DHB-Virionen blockiert, ohne dass dabei die Proteinsynthese von Leberzellen signifikant beeinträchtigt wird.
Die Prinzip-Lösung wird am Beispiel von primären Entenhepatozyten gezeigt, welche mit DHBV-Präparationen infiziert werden.
Chronisch mit DHBV infizierte primäre Entenhepatozyten, gewonnen durch Kollagenase-Behandlung aus der Leber von Entenembryonen (ausgebrütet aus kommerziell erwerblichen Enteneiern), die häufig bereits mit DHBV infiziert sind, werden mit verschiedenen Klassen von Proteasom-Inhibitoren behandelt, inklusive solcher Proteasom-Inhibitoren, welche sich bereits in klinischer Testung für die Behandlung von Kiebspatienten befinden (zum Beispiel die Proteasom-Inhibitoren PS-341, PS-273, PS-519). Nach der Behandlung mit Proteasom-Inhibitoren wird im Rahmen der Erfindung festgestellt, dass die behandelten Hepatozyten ihre Fähigkeit weitgehend oder vollständig verloren haben, infektiöse Viruspartikel zu produzieren. Aufgrund dieser neuartigen Aktivitäten von Proteasom-Inhibitoren ist davon auszugehen, dass die Applikation von *in vivo* verträglichen Proteasom-Inhibitoren die Infektionsausbreitung von Hepadnaviren im infizierten Organismus unterdrückt. Es ist weiterhin im Rahmen der Erfindung davon auszugehen, dass mit der neuartigen anti-viralen Strategie das Virusreservoir bei einer Hepadnavirus-Infektion vollständig eliminiert und damit eine teilweise oder vollständige Heilung einer viralen Hepatitis erzielt werden kann. Dieser Anspruch begründet sich insbesondere auf der Tatsache, dass mit dieser neuartigen Behandlung die Infektionsausbreitung und damit die Neuinfektion von Leberzellen verhindert werden kann. Auch wenn die intrazelluläre Synthese von viralen Proteinen und Nukleinsäuren in bereits infizierten Zellen nicht nachweisbar in der Menge reduziert wird, kann die Behandlung zur vollständigen Eliminierung des Virus führen, da Hepatitis-infizierte Zellen i.d.R. nur eine begrenzte Lebenszeit haben. Die Leberzellregeneration ist bei Patienten mit Leberentzündung besonders hoch. Deshalb sollten die in diesen Patienten häufiger entstehenden zunächst virusfreien Hepatozyten wegen der Behandlung mit Proteasom-Inhibitoren nicht *de novo* infiziert werden können.
Erfindungsgemäß wird gezeigt, dass der hemmende Effekt von Proteasom-Inhibitoren auf die Virusvermehrung von Hepadnaviren folgende Mechanismen beinhaltet:
1) Blockierung/Reduktion der Freisetzung von neuen Virionen;
2) Blockierung/Reduktion der Infektiosität von freigesetzten Virionen,
3) Blockierung/Reduktion der Infektionsausbreitung in Kultur von primären Hepatozyten;
4) Blockierung/Reduktion der Infektionsausbreitung in der Leber *in vivo.*

Zur Lösung der Aufgabe wurden im Rahmen der Erfindung verschiedene proteinchemische, molekular-virologische und immunhistologische Studien an HBV-infizierten Zellen durchgeführt. Erfindungsgemäß wird der durch Proteasom-Inhibitoren ausgelöste Defekt in der Infektiosität von Hepadnaviren mittels biochemischer Methoden dargestellt. Dazu wurden Western blot-Studien an DHBV-Proteinen durchgeführt.

Ebenfalls wird im Rahmen der Erfindungsbeschreibung die Blockierung der Neuinfektion von primären Hepatozyten mit Virus, welches auf Zellen behandelt mit Proteasom-Inhibitoren isoliert wurden, mittels Immunfluoreszenz-Messung ermittelt. Die dabei gewonnenen Informationen ermöglichen die Darstellung des hemmenden Effektes von Proteasom-Inhibitoren auf die Infektion von kultivierten primären Hepatozyten. Mit Hilfe dieser Methoden wird gezeigt, dass nach Behandlung von infizierten Zellen mit Proteasom-Inhibitoren faktisch keine infektiösen Viruspartikel von der Zelle mehr freigesetzt werden können. Durch diese *in vitro* Infektionsstudien wird gezeigt, dass verschiedene Substanzklassen von Proteasom-Inhibitoren alle den gleichen Effekt auslösen: die Blockierung der Produktion von infektiösen Hepadnaviren.
Ferner wird erfindungsgemäß die durch die Wirkung der Proteasom-Inhibitoren reduzierte Infektiosität freigesetzter unreifer Hepatitis-B-Virionen mittels End-Punkt-Titrationsstudien in primären Hepatozyten dargestellt. Dabei wird gezeigt, dass allein eine Inkubation mit Proteasom-Inhibitoren für 48 Stunden (etwa der Zeit eines HBV-Replikationszyklus) zu einer totalen Blockierung der Infektiosität führt, denn mit den verwendeten Methoden konnte faktisch kein Virus-Titer in den Zellkulturüberständen von Zellen beobachtet werden, welche mit Proteasom-Inhibitoren behandelt wurden.

Erfindungsgemäß wird am Beispiel DHBV gezeigt, dass unter der Behandlung mit Proteasom-Inhibitoren die biochemische Modifizierung des Nukleokapsidproteins verändert ist. Überraschenderweise wird festgestellt, dass core-Proteine, welche unter Proteasom-Inhibitoren-Behandlung exprimiert werden, eine Veränderung des Molekulargewichtes zeigen. Diese neuartige Beobachtung lässt auf eine Veränderung der Phosphorylierung des Nukleokapsidproteins rückschließen. Im Ergebnis dieser Beobachtung wird daher festgestellt, dass die Veränderung in der Modifizierung des Nukleokapsidproteins die mechanistische Grundlage für die reduzierte Infektiosität der aus den mit Proteasom-Inhibitoren behandelten Zellen sekretierten DHBV ist.
Im Rahmen der Erfindung wird erstmalig gezeigt, dass überraschenderweise nach Inaktivierung des UPS durch Behandlung von chronisch mit DHBV infizierten primären Hepatozyten die Freisetzung von infektiösen DHBV-Partikeln blockiert wird. Die spezifische Infektiosität der freigesetzten Virionen ist dramatisch erniedrigt. Mit allgemein üblichen Methoden der Infektiositätsbestimmung kann kein spezifischer Titer an neuproduzierten Viruspartikeln in den Zellkulturüberständen nachgewiesen werden, deshalb wurde ein Einzelinfektionstest angewandt (siehe hierzu Ausführungsbeispiel 7). Erfindungsgemäß werden hierzu primäre Entenhepatozyten, welche aus Lebern von Entenembryonen gewonnen und *in vitro* kultiviert wurden, mit DHBV-haltigen Zellkulturüberständen infiziert. Diese DHBV-haltigen Zellkulturüberstände wurden von chronisch mit DHBV infizierten primären Entenhepatozyten gewonnen, welche für zwei Tage mit 10 microM Proteasom-Inhibitoren behandelt wurden. Zu einer parallelen Kultur wurden keine Inhibitoren zugesetzt. Zum Zeitpunkt der maximalen Virusproduktion (ca. 90% der Hepatozyten einer primären Leberzellkultur befinden sich in der akuten Infektionsphase) wurde die Behandlung der Virus-produzierenden Kulturen begonnen. Die Menge an freigesetzten DHB-Virionen wird mittels DNA-dot-blot-Analyse der extrahierten DNA aus Viruspartikeln sowie mittels Western blot-Analyse des viralen core-Proteins untersucht. Dabei wurde eine 5- bis 10-fache Reduktion der freigesetzten DHB-Virionen beobachtet.
Die spezifische Infektiosität der neu produzierten DHB-Virionen wird mittels Titration in permissiven Kulturen an primären Entenhepatozyten ermittelt. Die Feststellung der Anzahl an infizierten Zellen erfolgt durch core- und preS-Immunfärbung. Bei Verwendung von verschiedenen Verdünnungen der Zellkulturüberstände als Inokkulum wird festgestellt, dass in Zellen, welche mit Zellkulturüberständen von den mit Proteasom-Inhibitoren behandelten Kulturen inkubiert wurden, kein einziges Infektionsereignis nachweisbar ist. In keiner der für die Titration verwendeten Kulturen konnte eine neu-infizierte Zelle mittels Immunfluoreszenz für DHBV core- oder preS-Proteine festgestellt werden. Erfindungsgemäß wird mit diesem Ergebnis eine neuartige Aktivität von Proteasom-Inhibitoren beansprucht (siehe hierzu Ausführungsbeispiel 8).

Diese neuartige Aktivität der Proteasom-Inhibitoren kann nicht auf rein unspezifische Beeinträchtigungen des Zellmetabolismus durch Abschaltung des UPS zurückgeführt werden, und zwar aus folgenden Gründen: Dieser Totalausfall an infektiösen DHB-Virionen ist nicht auf eine allgemeine Inhibierung der Proteinsynthese und DHBV-Expression in der Produzentenzelle zurückzuftihren, da zum einen während der 2-tägigen Behandlung mit Proteasom-Inhibitoren keine toxischen Effekte in den primären Entenhepatozyten beobachtet wurden, und zum anderen die Analyse der Expression von intrazellulären DHBV-Proteinen mittels Western blot-Technik keinen signifikanten Effekt der Proteasom-Inhibierung auf die Expression des DHBV-core-Proteins zeigte (siehe Ausführungsbeispiel 8).
Die Effizienz der Proteasom-Inhibitoren hinsichtlich der Blockierung des Proteasom-Pathway in den behandelten Hepatozyten wurde mittels Western blot-Analyse nachgewiesen unter Verwendung von Antikörpern, welche poly-ubiquitinierte Proteine erkennen. Es ist deutlich, dass bei allen getesteten Proteasom-Inhibitoren eine deutliche Akkumulation von poly-ubiquitinierten Proteinen erfolgt. Somit kann festgestellt werden, dass diese Inhibitoren in primären Hepatozyten voll wirksam sind.

In einer weiteren Ausführungsform der Erfindung wird gezeigt, dass die Behandlung von chronisch mit HBV infizierten humanen Hepatom-Zelllinien mit Proteasom-Inhibitoren die Freisetzung von HBV-spezifischen Proteinen, insbesondere von HBs- und HBe-Antigen bewirkt (siehe hierzu Ausführungsbeispiel 10). Ähnlich der Wirkung von Porteasom-Inhibitoren auf DHBV wurde eine deutliche Inhibierung der Sekretion von HBV-Proteinen beobachtet
In einer weiteren Form der Erfindung wird gezeigt, dass aufgrund der neuartigen Wirkung auf Freisetzung und Infektiosität von Hepatitis-Viren Proteasom-Inhibitoren die Ausbreitung einer HBV-Infektion in kultivierten Hepatozyten verhindern. Erfindungsgemäß wird dabei nachgewiesen, dass durch die Sekundärinfektion, das heißt die Übertragung einer bereits etablierten Infektion auf benachbarte Zellen, komplett verhindert wird. Dieser inhibitorische Effekt auf die Sekundärinfektion wurde in primären Entenhepatozyten nachgewiesen, welche nach Primärinfektion für mehrere Tage mit Proteasom-Inhibitoren behandelt wurden. Entsprechend der neuartigen Effekte von Proteasom-Inhibitoren wurde in den behandelten primär infizierten Zellen sowohl eine geringere Expression der viralen Antigene als auch wegen der Blockade der Sekundärinfektion eine geringere Zahl an neu infizierten Zellen festgestellt (siehe hierzu Ausführungsbeispiel 8).
Erfindungsgemäß ist dieser inhibitorische Effekt der Proteasom-Inhibitoren auf die DHBV-Sekundärinfektion vergleichbar mit der pharmakologischen Wirkung der Droge Suramin, von der bekannt ist, dass sie selektiv eine HBV-Sekundärinfektion blockiert, ohne dabei mit der bereits etablierten primären Infektion zu interferieren. Im Unterschied zu den verwendeten Proteasom-Inhibitoren ist Suramin jedoch eine sehr toxische Substanz, welche *in vivo* für die Behandlung von Hepatitis-Infektionen nicht verwendet werden kann. Darüber hinaus - und im Gegensatz zu Suramin - führt die Hemmung der Proteasom-Aktivität additiv zu einer Reduktion der HBV-Genexpression und bewirkt erfindungsgemäß die Überlagerung zweier antiviraler Effekte von Proteasom-Inhibitoren, die Inhibierung sowohl der Primär- als auch der Sekundärinfektion.

### Proteasom-Inhibitoren induzieren Zelltod von Leberkarzinomzellen, während primäre Hepatozyten relativ resistent gegenüber Proteasom-Inhibitoren sind

In einem weiteren Teil der Erfindung wird gezeigt, dass Proteasom-Inhibitoren den Zelltod von Leberkarzinomzellen induzieren. Erfindungsgemäß wird am Beispiel von kultivierten Entenhepatozyten gezeigt, dass primäre Leberzellen relativ resistent gegenüber Proteasom-Inhibitoren bis zu einer Konzentration von ca. 10 microM sind, während Leberkarzinomzellen bereits bei 100-fach niedrigeren Konzentrationen von Proteasom-Inhibitoren abgetötet werden.
Die Induktion des Zelltodes (ausgelöst durch apoptotische, nekrotische oder andere Prozesse) in HBV-produzierenden Leberkarzinom-Zellen nach Behandlung mit Proteasom-Inhibitoren wird erfindungsgemäß am Beispiel von humanen Leberkarzinomzelllinien (zum Beispiel HepG.2.2.15) dargestellt. Nachgewiesen wird dieser neuartige Mechanismus mittels Trypanblau-Ausschlussfärbung (lebende Zellen werden nicht gefärbt, tote Zellen nehmen den Farbstoff auf), durch Nachweis der Annexin-V Oberflächenexpositon durch Immunfluoreszenz, durch Detektion der DNA-Fragmentierung, durch Immunoblot-Nachweis und Fluoreszenzfärbungs-Nachweis von prozessierten und aktivierten Caspasen und enzymatischen Nachweis der Caspaseaktivität. In einer weiteren Ausführungsform der Erfindung werden eine Hühnerhepatomazelllinie (LMH) und primäre Hepatozyten aus Enten (DHBV-infiziert und nicht-infiziert) sowie Hühnern (nicht DHBV-infiziert) auf selektive Toxizität hinsichtlich Proteasom-Inhibitoren für Hepatomazellen im Verbund mit nicht transformierten Hepatozyten getestet.
Das bevorzugte Absterben von Leberkarzinomzellen begründet sich erfindungsgemäß in der anti-neoplastischen Wirkung von Proteasom-Inhibitoren. Beispielsweise besitzt der bekannte Proteasom-Inhibitor PS-341 eine selektive zytotoxische Aktivität gegen ein breites Spektrum an humanen Tumorzellen, eine Aktivität, welche mit der Akkumulation von p21 und Zellzyklus-Arrest in der G2-M-Phase mit nachfolgender Apoptose verbunden ist. Die Expression, Modifizierung und Aktivität des Tumorsuppressor-Proteins p53 wird ebenfalls durch die Wirkung von Proteasom-Inhibitoren beeinträchtigt. Erfindungsgemäß kann durch diese und andere Mechanismen die gezielte Eliminierung von Leberkarzinomzellen bewirkt werden, welche infolge einer HBV-Infektion und HCV-Infektion oder entsprechender Koinfektion mit beiden Viren oder mit HDV/HBV-Koinfektion mehr als 100 mal häufiger entstehen als bei nicht-infizierten Zellen.
In einer weiteren Ausführungsform der Erfindung wird erstmals festgestellt, dass Proteasom-Inhibitoren relativ geringe toxische Effekte in primären Hepatozyten auslösen, jedoch ein bevorzugtes Absterben von Leberkarzinomzellen bewirken. Erfindungsgemäß wird hierzu gezeigt, dass primäre Hepatozyten tagelange Behandlung mit Proteasom-Inhibitoren bis zu Konzentrationen von 10 micoM tolerieren. Im Unterschied dazu sterben humane Leberkarzinomzellen bereits bei 1000-fach niedrigeren Konzentrationen an Proteasom-Inhibitoren (siehe hierzu Ausführungsbeispiel 9). Die unterschiedliche Toxizität von Proteasom-Inhibitoren auf primäre Hepatozyten im Vergleich mit humanen Hepatoblastoma-Zellen wurden mittels Dosis-Limitierungsstudien mit Proteasom-Inhibitoren untersucht. Die Vitalität der Zellen wurde lichtmikroskopisch überprüft. Parallele Kulturen wurden mit steigenden Konzentration an Proteasom-Inhibitoren (10 microM, 3 microM, 1 microM, 10 nanoM und 1 nanoM) für mehrere Tage behandelt. Zusätzlich wurde die Funktionalität der Hepatozyten mittels Fluoreszenzvitalfärbung bestimmt. Dabei wurde erfindungsgemäß festgestellt, dass primäre Entenhepatozyten relativ hohe Konzentrationen von Proteasom-Inhibitoren bis zu ca. 10 microM tolerieren können, während proliferierende Leberkarzinomzellen sehr viel sensitiver gegenüber der toxischen Wirkung von Proteasom-Inhibitoren sind.

### Verwendung von Proteasom-Inhibitoren in der Behandlung von Infektionen mit Hepatitisviren

Das in der Erfindungsbeschreibung dargestellte Prinzip der Verwendung von Proteasom-Inhibitoren zur Blockierung einer Infektion mit Hepadnaviren ist neuartig in Hinsicht auf die Verwendung einer bereits bekannten Substanzklasse (den Proteasom-Inhibitoren) für eine neue Aktivität, welche sich in folgenden therapeutischen Konzepten zusammenfassen lässt:
- die Blockierung der Produktion von infektiösen Hepadnaviren und damit die Verhinderung der Infektionsausbreitung *in vivo,* dem Lebergewebe einer infizierten Person;
- die Induktion des Absterbens von Leberkarzinomzellen, welche in direkter oder indirekter Folge einer Infektion mit Hepadnaviren entstanden sind.
Gleichzeitig ist die Verwendung von Proteasom-Inhibitoren auch neuartig hinsichtlich des Anwendungsprinzips. Bislang sind keine Substanzen / Prinzipien / Methoden bekannt, welche späte Prozesse der Replikation von Hepadnaviren, speziell der Freisetzung von infektiösen Virionen beeinflussen. Weiterhin ist neu, dass die Verwendung von Proteasom-Inhibitoren zur Blockierung der Replikation von Hepatitisviren führt. Dieser Inhibitionsmechanismen ist in allen Wirtszellen des Virus, den Leberhepatozyten, konserviert. Im Vergleich zu bisherigen anti-viralen Methoden der Behandlung von Hepatitis-Infektionen, welche essentielle Komponenten des Virus direkt treffen, ist die Wahrscheinlichkeit der Entstehung von Resistenzmechanismen bei Applikation von Proteasom-Inhibitoren in der Behandlung von Hepadnavirus-Infektionen um Größenordnung geringer. Die Neuartigkeit dieses Wirkprinzips von Proteasom-Inhibitoren zeigt sich auch in der Tatsache, dass Proteasom-Inhibitoren ein breites Wirkungsspektrum gegenüber unterschiedlichen Hepatitisviren (HAV, HBV, HCV, HDV, HEV, HGV) besitzen. Der inhibitorische Effekt wurde im Rahmen der Erfindung mit gleicher Intensität bei verschiedenen primären als auch klonierten Hepatitisviren beobachtet.
Neuartig ist weiterhin das Prinzip der Wirkung von Proteasom-Inhibitoren, die die Produktion von infektiösen Viruspartikeln von bereits mit Hepadnaviren infizierten Zellen verhindern. Dadurch wird wesentlich die Menge an infektiösen Virionen (Viruslast) und somit die Infektionsausbreitung *in vivo* reduziert.
In der Summe dieser neuartigen Mechanismen lässt sich feststellen, dass die verminderte Freisetzung von noch dazu wenigen oder gar nicht infektiösen Viruspartikeln im Netto-Effekt bei gleichzeitigem Zelltod von Virus-produzierenden Karzinomzellen im Falle einer *in vivo* Anwendung von Proteasom-Inhibitoren die Menge an infektiösen Virionen in einem mit Hepadnaviren infizierten Organismus verringert. Somit wird insgesamt die Zahl infizierter Produzentenzellen im Leberzellgewebe reduziert. Dies macht die Anwendung von Proteasom-Inhibitoren allein oder in Kombination mit bereits in der anti-viralen Therapie von Hepadnaviren verwendeten Therapeutika attraktiv.
Im Rahmen der Erfindung wird erstmals festgestellt, dass Proteasom-Inhibitoren sowohl die Erhaltung und Persistenz einer bereits etablierten Infektion hemmen als auch die Sekundärinfektion und somit die Ausbreitung einer Infektion mit Hepatitisviren *in vivo,* in Hepatozyten komplett blockieren. Entsprechend der vorliegenden Erfindung sind Proteasom-Inhibitoren geeignete Substanzen, um die Ausbreitung einer HBV-Infektion *in vivo* zu blockieren.
Der wesentliche Vorteil der Erfindung besteht darin, dass mit der Behandlung von Proteasom-Inhibitoren zwei für die Bekämpfung viraler Hepatitis-Infektionen wesentliche Effekte ausgelöst werden können: Zum einen wird die Produktion von infektiösen Viruspartikeln und damit die Infektionsausbreitung im Organismus gehemmt. Zum anderen wird die Entstehung, das Wachstum und die Metastasierung von Leberzelltumoren verhindert, welche infolge einer Hepatitisvirus-Infektion nach einer Latenzphase sehr häufig auftritt. Darüber hinaus werden bereits bestehende Leberkarzinome durch die Proteasom-Inhibitoren zerstört, nicht aber die wenig oder nicht proliferierenden normalen Zellen der Leber.
Aufgrund dieser neuartigen Behandlungsmethode können daher vielfältige therapeutische Effekte durch den Einsatz von Proteasom-Inhibitoren bei Infektionen mit Hepadnaviren ausgelöst werden. Neben der Blockierung der Infektiosität der freigesetzten Viren sowie der Verhinderung von Leberzellkarzinomen besteht ein weiterer Vorteil dieser Behandlungsmethode darin, dass mit dieser Strategie zelluläre Faktoren getroffen werden, welche für die Replikation von Hepadnaviren essentiell sind, jedoch eine sehr viel höhere genetische Stabilität im Vergleich zu viralen Faktoren beinhalten. Aufgrund dieser genetischen Stabilität der Zielstruktur dieser neuartigen anti-viralen Strategie ist mit dem Auftreten von Resistenz-Erscheinungen, wie sie für viele der bislang bekannten Inhibitoren einer HBV-Infektion bekannt sind, nicht zu rechnen. Insbesondere gilt dies für die Polymerasemutanten von Hepatitis B und C Viren, die bei Nukleosidanaloga-Behandlung so gut wie immer nach einer relativ kurzen Zeit auftreten. Dies gilt auch für Immunescape-Varianten, wie sie bei passiv und aktiv Vakzinierten sowie natürlicherweise auftreten. Gleiches gilt für Interferon-resistente Stämme von HBV und HCV. Auf diesem neuartigen Effekt von Proteasom-Inhibitoren beruht der erfinderische Anspruch, dass mit der Behandlung von Proteasom-Inhibitoren nicht nur die Ausbreitung einer HBV-Infektion verhindert, sondern auch durch HBV verursachte Leberzellkarzinome therapiert werden können.

Die Erfindung soll anhand von Ausführungsbeispielen näher erläutert werden,

### Ausführungsbeispiele

### Beispiel 1: Hemmung der Proteasom-Aktivität hat keinen wesentlichen Einfluss auf die intrazelluläre virale Genexpression, blockiert jedoch drastisch die Freisetzung von Viruspartikeln aus chronisch DHBV-infizierten Hepatozyten.

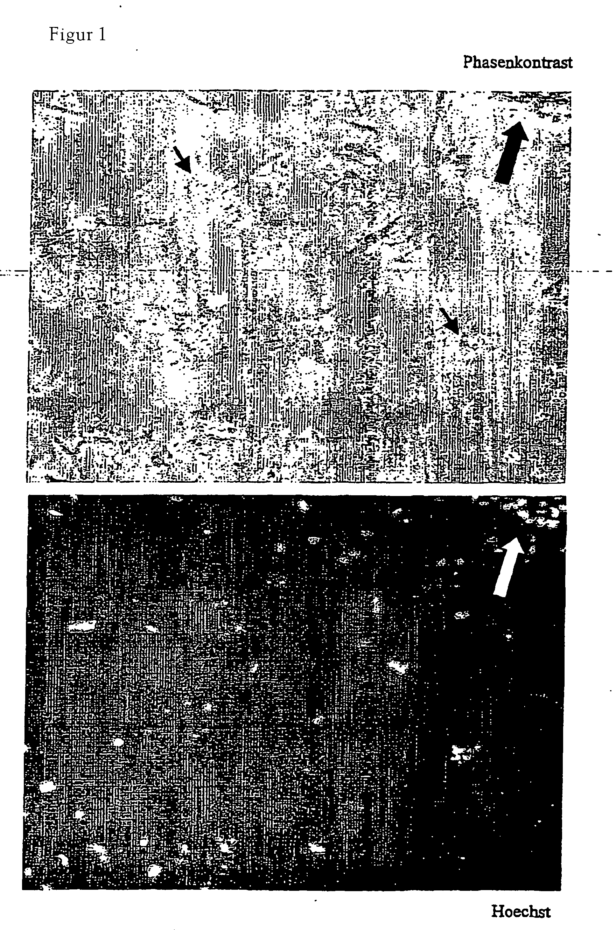

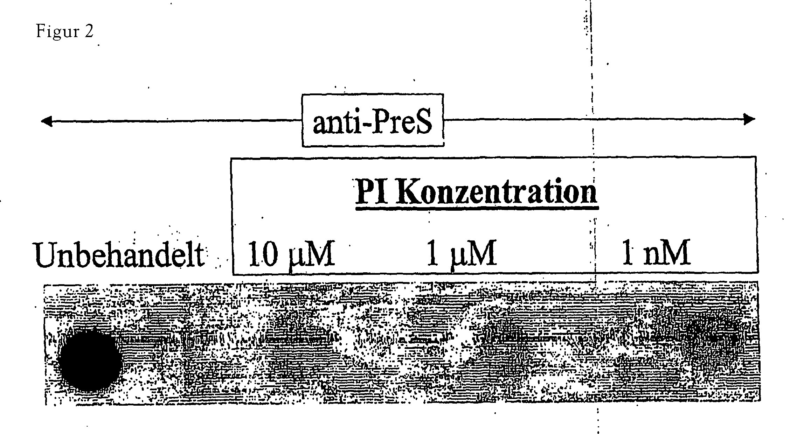

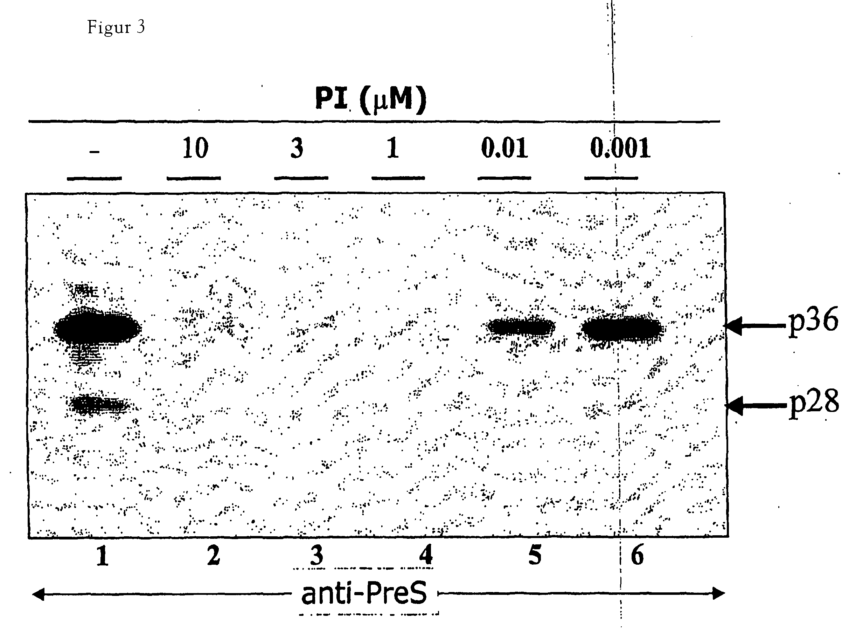

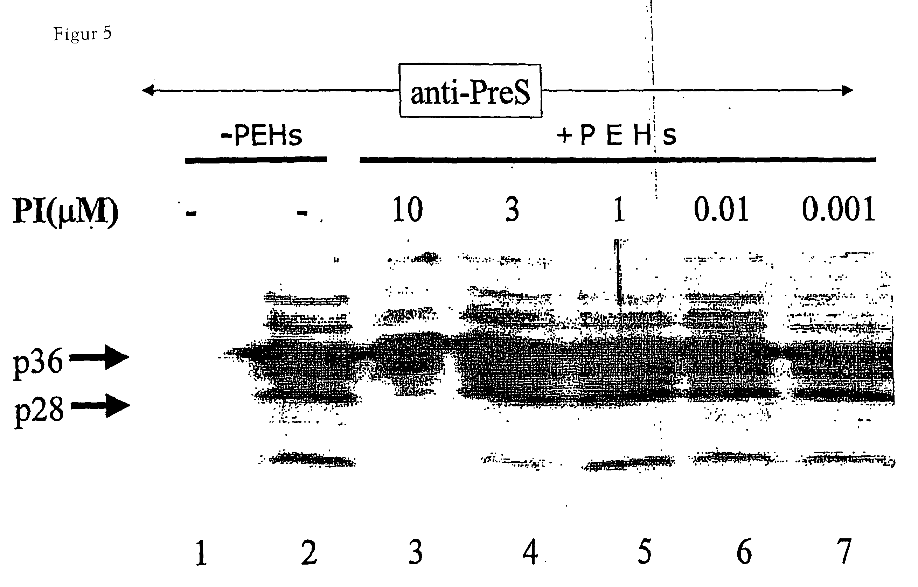

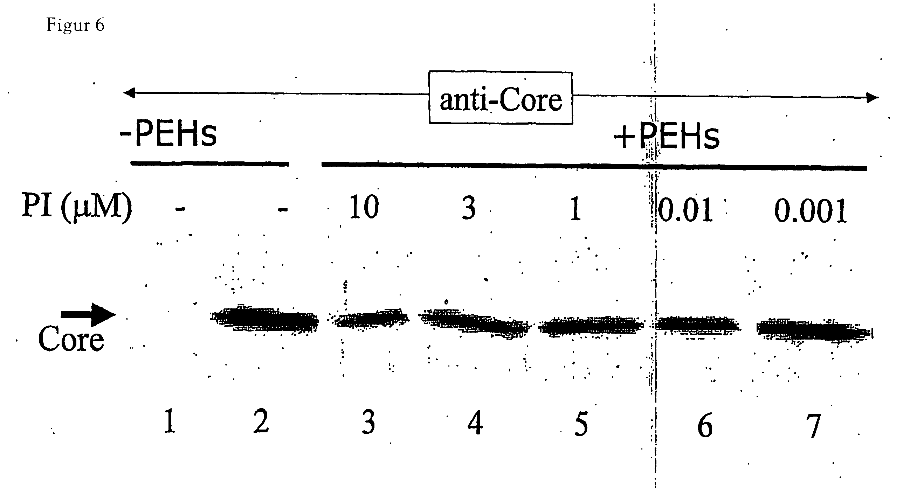

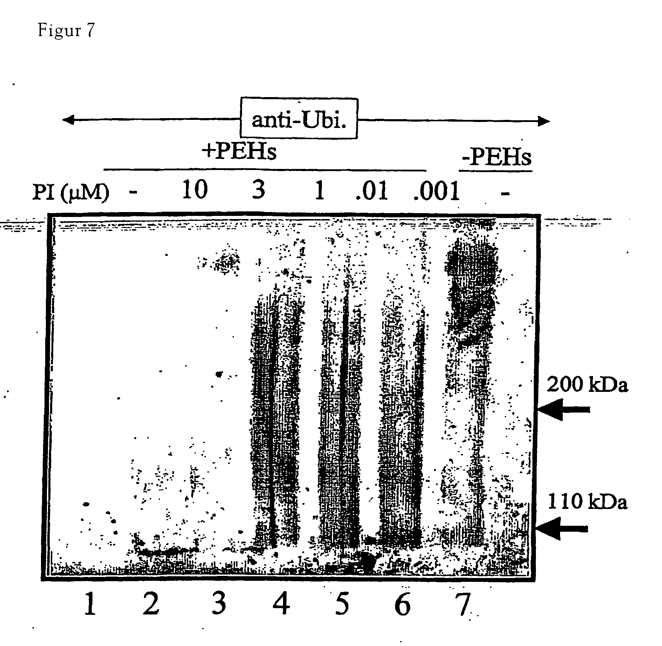

Primäre Entenhepatozyten und anderen Nichtparenchymzellen der Leber wurden aus Entenembryonen durch Kollagenase-Behandlung im wesentlichen nach einer bereits beschriebenen Methode gewonnen (Köck *et al.,* 1993). Befruchtete Pekingenteneier wurden von einer Zuchtfuma (Wichmann, Deutschland) käuflich erworben. Die Bebrütung der Eier erfolgte in einem automatisierten Brutschrank bei 37°C und 50% Humidität für mindestens 21 Tage. Nach Eröffnung des Eies und der Entnahme des Embryos, erfolgte dessen Sakrifizierung und Blutentnahme. Anschließend wurde die Leber unter Exklusion der Gallenblase entnommen, mechanisch zerkleinert und in 3 ml
Williams E Medium (GIBCOBRL, Paisley, Scotland) mit 0.5% Kollagenase (Sigma, Deisenhofen, Deutschland) aufgenommen und für 1 h bei 37°C verdaut. Die Zellen wurden zweimal mit nativem Williams E Medium gewaschen, die vitalen und dissozüerten Zellen durch 10%ige Percoll-Dichte-Zentrifugation (Sigma, Deisenhofen, Deutschland) von größeren Zellaggregaten und Zelltrümmem getrennt. Parallel wurden die entnommenen Blutseren auf die Präsenz einer natürlichen kongenitalen DHBV-Infektion mittels Protein-dot-blot-Analyse mit PreS-Antiserum getestet (Sunjach *et al.,* 1999). Hepatozyten aus DHBV-positiven Tieren wurden gepoolt und in diesem Experiment eingesetzt. Die Zellen wurden in Williams E Medium (GIBCOBRL, Paisley, Scotland), supplementiert mit 2 milliM L-Glutamin (GIBCOBRL, Paisley, Scotland), 100 Einheiten ml⁻¹ Penicillin, und 100 microg ml⁻¹ Streptomycin (Biochrom, Berlin, Deutschland) 15mM HEPES (N-[2-hydroxyethyl]piperazine-N'-[2-ethanesulfonic acid]) (pH 7.2) (alle von GIBCOBRL, Paisley, Scotland), 10⁻⁵ Hydrocortison, 10⁻⁹ M Insulin und 1.5% DMSO (alle von Sigma, Deisenhofen, Deutschland) resuspendiert und mit einer Dichte von ca. 8x10⁵ pro well in 12-well-Mikrotiterplatten (Greiner, Solingen, Deutschland) ausgesät und bei 37°C kultiviert. Ca. 4 h nach der Ausplattierung wurde das Medium gewechselt, und ein weiterer Mediumwechsel fand 24 h später statt. Wenn nicht anderes angegeben, wurde das Medium im folgenden alle 2 bis 3 Tage gewechselt. Eine Woche nach Aussaat des Gemisches aus primären Hepatozyten und anderen Leberzellen wurde das Kulturmedium erneut gewechselt und die Zellen mit neuem Medium, welches nun unterschiedliche Konzentration an PI (10 microM, 3 microM, 1 microM, 10 nanoM, und 1 nanoM) bzw. keinen Inhibitor enthielt, inkubiert. PI wurde in PBS gelöst wie beschrieben (Adams und Stein, 1996; Adams *et al.,* 1996) und ein Aliquot der 1000-fach konzentierten Stock-Lösung dem Medium zugesetzt. Die Morphologie der Zellen während der Behandlungsphase wurde alle 12 h (Stunden) lichtmikroskopisch überprüft. Nur bei einer Konzentration von 10 microM PI wurden die ersten, auf Toxizität deutenden morphologischen Veränderungen beobachtet. Diese traten nach etwa 48 h Behandlung auf und beinhalteten eine feingranuläre Vakuolisierung, Verlust der makrovakuolären Fetttröpfchen sowie Abflachung der Hepatozyten, während bei nichtparenchymalen Zellen Abrundung, Schrumpfung und die Ablösung vom Boden der Zellkulturschale das Bild bestimmte. Bei Konzentrationen kleiner als 10 microM PI wurden selbst nach 48 h Behandlungszeit keine signifikanten morphologischen Veränderungen bei Hepatozyten beobachtet, welche auf PI-bedingte Veränderungen des Zellmetabolismus schließen lassen könnten.
Im Unterschied dazu war eine kontinuierliche Behandlung für 72 h mit 1 microM PI nicht vereinbar mit der Vitalität der nicht-parenchymalen Zellen. Das Phasenkontrastbild (Figur 1) zeigt einen relativ großen Bereich mit einem ehemaligen Cluster von nicht-parenchymalen Zellen, wo nach Behandlung mit 1 microM PI fast keine adharenten Zellen mehr vorhanden sind. Die Hepatozyten haben dagegen ihre normale Morphologie beibehalten (Figur 1, Blockpfeil). Die Hörhstfärbung (Figur 1) belegt, dass die restlichen nicht-parenchymalen Zellen abgerundet und pyknotisch sind (Pfeile).
Nach der Behandlung der chronisch DHBV-infizierten Hepatozyten für 48 h mit unterschiedlichen Dosen an PI (10 microM, 3 microM, 1 microM, 10 nanoM, und 1 nanoM) bzw. keinen Inhibitor wurden die Zellkulturüberstände geerntet und zentrifugiert (4000 rpm für 5 min). Die Menge der freigesetzten viralen Partikeln wurde mittels eines PreS-Antigen spezifischen dot-blots (Figur 2) und eines Immunoblots (Figur 3) bestimmt (Bruns *et al.,* 1998). Für dot-blot-Analyse wurden Aliquots von 300 microl mittels eines Apparates (Schleicher & Schuell, Dassel, Deutschland) auf Nitrozellulosemembran (Schleicher & Schuell, Dassel, Deutschland) aufgedottet, luftgetrocknet und anschließend mit PBS (phosphate buffered saline) gewaschen. Für Western blot-Analyse wurden 5 µl Überstand nach Zugabe von SDS-PAGE Probenpuffer durch Kochen für 5 min denaturiert. Anschließend wurden die Proben in 12.5 % SDS-PAGE aufgetrennt und mittels Semi-Dry Transfertechnik (Biorad, München, Deutschland) auf Nitrozellulose elektro-transferiert.
Nach Blocken mit 5% Magermilch erfolgte die Inkubation der Membranen mit PreSspezifischem Antiserum (Kaninchen anti-preS Kpn; Femholz *et al.,* 1993). Die markierten Proteine wurden mit enhanced Chemolumineszenzmethode (ECL) (Pierce, Rockford, USA) sichtbar gemacht. Figuren 2 und 3 zeigen, dass die Proteasom-Inhibitor-Behandlung von DHBV-infizierten Hepatozytenkulturen zu einer drastischen dosisabhängigen Verminderung an PreS-Protein im Medium führt. Selbst bei einer relativ niedrigen Konzentration von 0.01 microM PI wurde eine fast 10-fache Inhibierung der Freisetzung der subviralen Partikel beobachtet (Figur 3). Da PreS-Protein ein struktureller Bestandteil aller viraler Partikel ist (leere subvirale Partikel und DNA-haltige infektiöse Virionen), reflektiert die PreS-Proteinmenge im dot- und Western blot unmittelbar die Menge an umhüllten Viruspartikeln, welche während der Behandlungszeit in das Medium sezenüert wurden.
Der Effekt von Proteasom-Inhibitoren auf die Menge der ins Medium freigesetzten, Genom enthaltenden viralen Partikel wurde mittels DNA-dot-blot analysiert (Sunjach *et al.,* 1999). Nach der Behandlung der chronisch DHBV-infizierten Hepatozyten für 48 h mit unterschiedlichen Dosen an PI (10 microm, 3 microM, 1 microM und 10 nanoM) bzw. keinen Inhibitor wurden die Zellkulturüberstande geerntet und zentrifugiert (4,000 rpm für 5 min). Nach Aufdotten von Proben (jeweils 200 microl) auf Nitrozellulose-Membran, wurde sie mit einem alkalischen Puffer (0.5 M NaOH und 1.5 M NaCl) 2 Mal jeweils 1.5 min auf 3MM Whatman-Papier (Schleicher & Schuell, Dassel, Deutschland) denaturiert und anschließend mit einem neutralen Puffer (0.5M Tris·HCl, pH 7.4 und 3M NaCl) 4 Mal für jeweils 1 min auf 3MM Whatman renaturiert. Nach Lufttrocknung und Crosslinking der Membran erfolgte anschließend deren Hybridisierung mit ³²P-markiertem kloniertem DHBV-Genom als Sonde. Die Signale wurden durch Autoradiographie nach einem Standardprotokoll (Sunjach *et al.,* 1999) sichtbar gemacht. Die Figur 4 zeigt, dass PI die Menge der freigesetzten kompletten Viruspartikel mindestens um den Faktor von ca. 4 reduziert. Das Ergebnis der DNA-Bestimmung korreliert somit mit der in ähnlicher Weise reduzierten Menge an sezerniertem PreS-Antigen (Figur 2 und 3).
Um den Einfluss der Behandlung mit Proteasom-Inhibitoren auf die intrazelluläre DHBV-Expression zu testen, wurden die parallel behandelten und unbehandelten Zellen mit 200 microl Puffer (50 mM Tris HCl pH 7.5, 150 mM NaCl, 1% Triton X-100, 1% Natrium-Deoxycholat), komplementiert mit einem Protease-Inhibitor-Cocktail (Complete, Roche, Mannheim) lysiert Die Lysate wurden von der unlöslichen Zellfraktion durch Zentrifugation (14,000 rpm, 10 min, 4°C) getrennt, und jeweils 20 microl der geklärten Gesamtlysate wurden nach Zugabe von Laemmli-Puffer durch Kochen für 5 min denaturiert. Anschließend wurden die Proben in 12.5 % SDS-PAGE aufgetrennt und mittels Semi-Dry Transfertechnik (Biorad, München, Deutschland) auf Nitrozellulose transferiert. Die Membranen wurden mit 5% Magermilch (Biorad, München, Deutschland) geblockt und nach Waschen mit TBS (tris buffered saline) mit Antikörpern gegen PreS- (Fernholz *et al.,* 1993) oder gegen Core-Protein (Breiner *et al.,* 2001) inkubiert (Figur 5 und 6. Die markierten viralen Proteine wurden anschließend mittels enhanced Chemolumineszenz (Pierce, Rockford, USA) sichtbar gemacht.
Die Figuren 5 und 6 zeigen, dass es unter Behandlung mit Proteasom-Inhibitoren zu keiner signifikanten Änderung der steady state Mengen an intrazellulärem PreS- (Figur 5) und Core-Protein (Figur 6) kommt. Lediglich bei der höchsten Konzentration von 10 microM PI ist eine geringfügige Reduktion an viralen Proteinen zu beobachten. Es ist bemerkenswert, dass zum Beispiel 10 nanoM PI überhaupt keinen Effekt auf die intrazelluläre virale Proteinexpression hat, aber immer noch einen deutlichen Effekt auf die Freisetzung ausübt.
In der Summe dieser Ergebnisse wird festgestellt, dass die Behandlung von chronisch infizierten primären Entenhepatozyten mit PI bis zu einer Konzentration von 10 microM über einen 48 h Behandlungszeitraum nur einen geringen oder gar keinen Einfluss auf die intrazelluläre Expression von Core- und PreS-Antigen hat, während die Freisetzung von viralen Partikeln jedoch drastisch reduziert ist. Darüber hinaus kann geschlussfolgert werden, dass Vitalität und Proteinsynthese der primären Entenhepatozyten nicht wesentlich beeinträchtigt sind.
Um die Wirksamkeit der Behandlung von primären Entenhepatozyten mit Proteasom-Inhibitoren hinsichtlich der Blockade des UPS zu demonstrieren, wurden jeweils 20 microl der Zelllysate in SDS-PAGE Probenpuffer aufgenommen, für 5 min gekocht, und in 6% SDS-PAGE aufgetrennt. Die Proteine wurden auf Nitrozellulose transferiert und mit polyklonalen Kaninchen-anti-Ubiquitin-Antikörpem (Sigma, Deisenhofen, Deutschland) inkubiert und mittels nachfolgender ECL-Reaktion sichtbar gemacht. Die Figur 7 zeigt ab einer Konzentration von 1 microM PI eine deutliche Akkumulation von poly-ubiquitinierten Proteinen relativ zu den niedermolekularen Banden. Es ist bekannt, dass lang andauernde Behandlung mit Proteasom-Inhibitoren zu einer Veränderung sowohl der Proteinsynthese als auch des -musters führt. Diese ist deutlich anhand des veränderten Profils der niedermolekularen Proteine in den PI-behandelten Zellen zu erkennen und daher ein weiterer Beleg für die Wirksamkeit von PI zur effizienten Blockierung der Proteasom-Aktivität in primären Hepatozyten.

### Beispiel 2: Proteasom-Inhibitoren blockieren die Produktion / Freisetzung von infektiösen Viren aus chronisch DHBV-infizierten primären Hepatozyten.

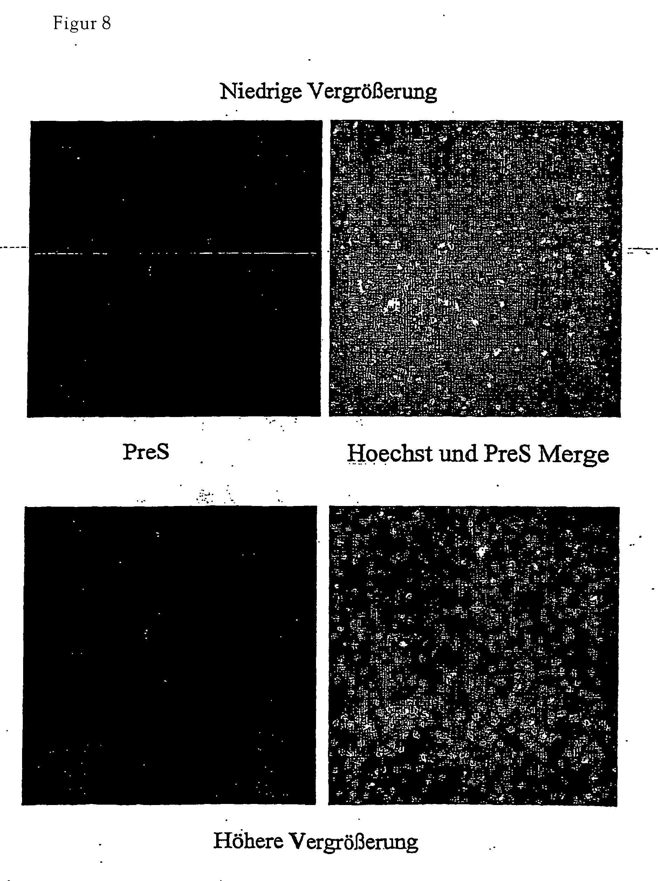

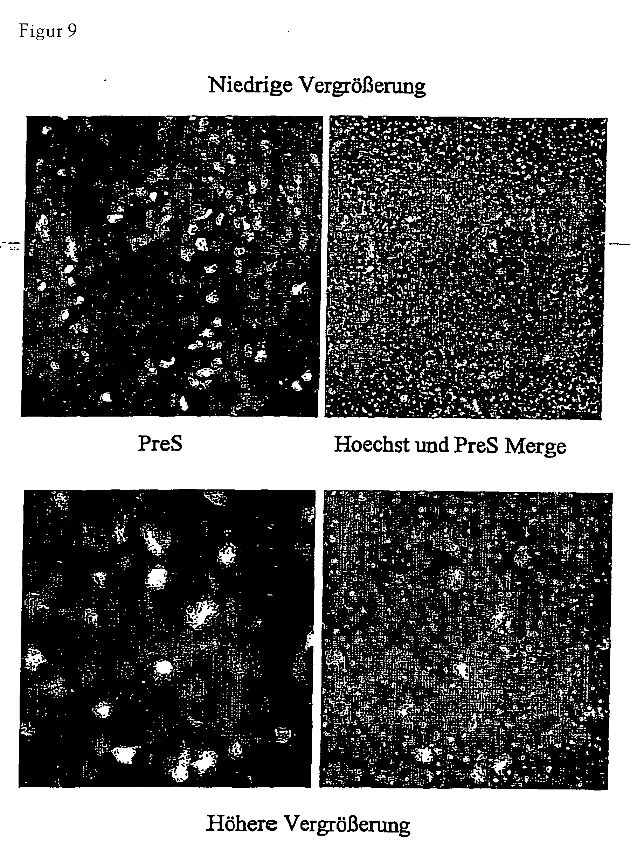

In den vorhergehenden Experimenten (Beispiel 1) wurde festgestellt, dass eine Inhibition der zellulären Proteasom-Aktivität die Freisetzung von DHBV-Proteinen wesentlich einschränkt, ohne jedoch signifikant die intrazelluläre virale Proteinexpression zu beeinflussen. Es blieb daher weiterhin zu klären, ob die Behandlung mit Proteasom-Inhibitoren nicht nur die Sekretion von Viruspartikeln um einen Faktor von ca. 4 reduziert, sondern darüber hinaus auch noch einen negativen Effekt auf den Grad der Infektiosität der Nachkommenviren hat. Es wurde daher untersucht, ob und wie viele infektiöse Virionen in den Überständen von mit Proteasom-Inhibitoren behandelten chronisch DHBV-infizierten primären Entenhepatozyten enthalten sind im Vergleich zu nicht behandelten Zellen. Hierzu wurden ca. 7 Tage alte, chronisch DHBV-infizierte primäre Entenhepatozyten, deren Herstellung bereits unter Beispiel 1 beschrieben ist, für 48 h mit 10 microM PI behandelt. Anschließend wurden die Zellkulturüberstände, wie bereits unter Beispiel 1 beschrieben, geerntet und für die Neuinfektion von primären Leberzellen benutzt. DHBV-negative, primäre Entenhepatozyten wurden wie im Beispiel 1 beschrieben präpariert und mit einer Dichte von ca. 8x10⁵/well in 12 well-Mikrotiterplatten ausgesät. Eine Woche nach der Ausplattierung wurden die Zellen durch Zugabe von 1 ml zollfreier Zellkulturüberstände von PI-behandelten beziehungsweise nicht behandelten DHBV-positiven Zellkulturen infiziert. Als Kontrolle wurde eine parallele Kultur mit 20 microl DHBV-positivem Serum/well infiziert (entspricht einer multiplicity of infection (MOI) von 20). Nach 16 h Inkubation bei 37°C wurde das Virus-Inokkulum entfemt, die Zellen mit PBS gewaschen und anschließend in neuem Medium weiterkultiviert. Die Etablierung einer produktiven Infektion wurde mittels indirekter Immunofluoreszenz und SDS-PAGE für die viralen Proteine Core und PreS nach 2.5 Tagen bestimmt. Für Immunfluoreszenzfärbung wurden die Zellen mit PBS gewaschen und anschließend mit 1 ml einer eiskalten 1:1 Mischung aus Methanol und Aceton für 10 min bei Raumtemperatur fixiert. Die fixierten Zellen wurden mit dem ersten Antikörper, Kaninchen-anti-PreS (Breiner *et al.,* 2001) in einer Verdünnung von 1:800 in PBS für 1 h bei Raumtemperatur inkubiert. Nach Waschen mit PBS erfolgte eine 30-minütige Inkubation mit dem Alexa 488-gekoppelten Sekundätantrlcörper (Ziege-anti-Kaninchen, Molecular Probes, Leiden, Niederlande). Die Zellkerne wurden mit Hoechst (4 microg/ml) (Sigma, Deisenhofen, Deutschland) gefärbt Die Fluoreszenzen wurden mit einem inversen Epiffuoreszenzmikroskop (Axiovert S100, Carl Zeiss, Göttingen, Deutschland) analysiert und mittels des Bildverarbeitungssystems Openlab (Improvision, Coventry, UK) prozessiert. Die Immunfluoreszenzbilder zeigen, dass in Zellkulturen, welche mit DHBV aus Zellkulturüberständen von mit PI-behandelten Zellen infiziert wurden, keine PreSexprimierende Zelle nachgewiesen werden konnte (Figur 8, PreS), während ca. 1-5% der Hepatozyten in den Kulturen, welche mit Überständen von unbehandelten Zellen inkubiert wurden, eindeutig PreS-positiv und damit infiziert waren (Figur 9, PreS). Als Nachweis für die Anzahl der auf dem Objektträger vorhandenen Zellen wurde deren Zellkerne mit Hoechst gefärbt und dieses Bild mit dem Fluoreszenzbildern überlagert (für behandelte Zellen, siehe Figur 8 und für unbehandelte Zellen, siehe Figur 9, jeweils Hoechst und PreS merge).

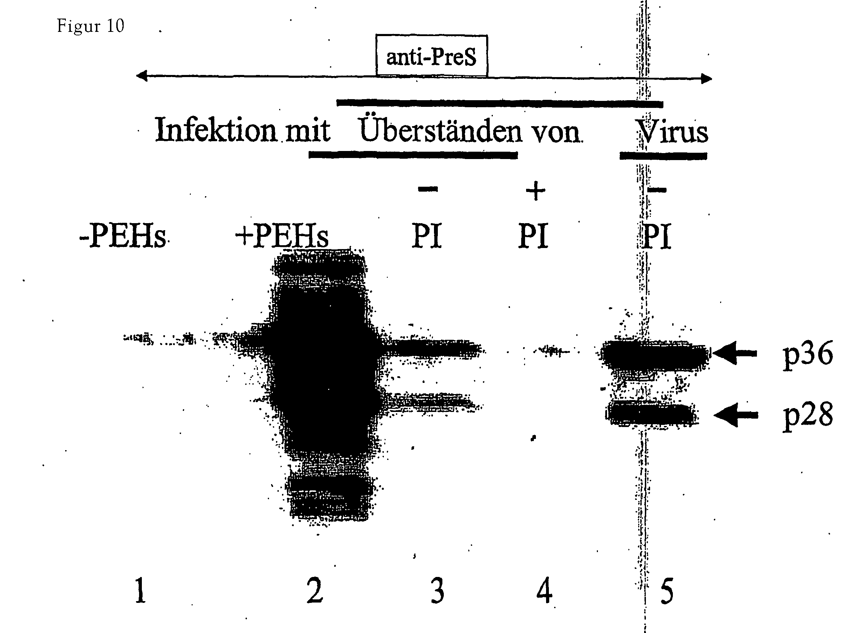

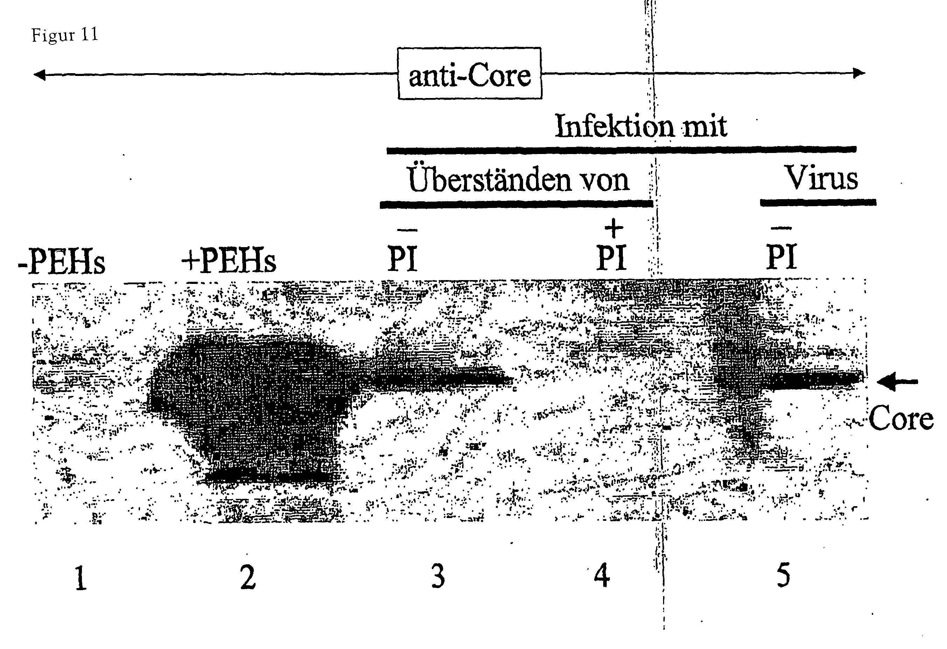

Für Western blot-Analysen wurden die Zellen aus parallelen Kulturen direkt mit 200 microl Laemmli-Puffer lysiert und für 5 min gekocht. Jeweils 20 µl der Zelllysate wurden in 12.5% SDS-PAGE aufgetrennt und auf Nitrozellulose transferiert. Die Membranen wurden mit Antikörpern gegen PreS- (Fernholz *et al.,* 1993) oder gegen Core-Protein (Breiner *et al.,* 2001) inkubiert und Proteine mit ECL sichtbar gemacht. Die blots zeigen deutlich sichtbare Signale für PreS- (Figur 10, Spur 3) und Core-Protein (Figur 11, Spur 3) in den Extrakten von Zellen, die mit DHBV aus nicht behandelten Zellen infiziert wurden, dagegen so gut wie keinen Hinweis für die Expression von DHBV-Proteinen in Zellen, welche mit Zellkultur-Überständen von PI behandelten Zellen inkubiert wurden (Figur 10, Spur 4 bzw. Figur 11, Spur 4).

### Beispiel 3: Proteasom-Inhibitoren blockieren sowohl die frühe Phase einer akuten DHBV-Infektion als auch die nachfolgende Sekundärinfektion und damit deren Ausbreitung in Zellkultur.

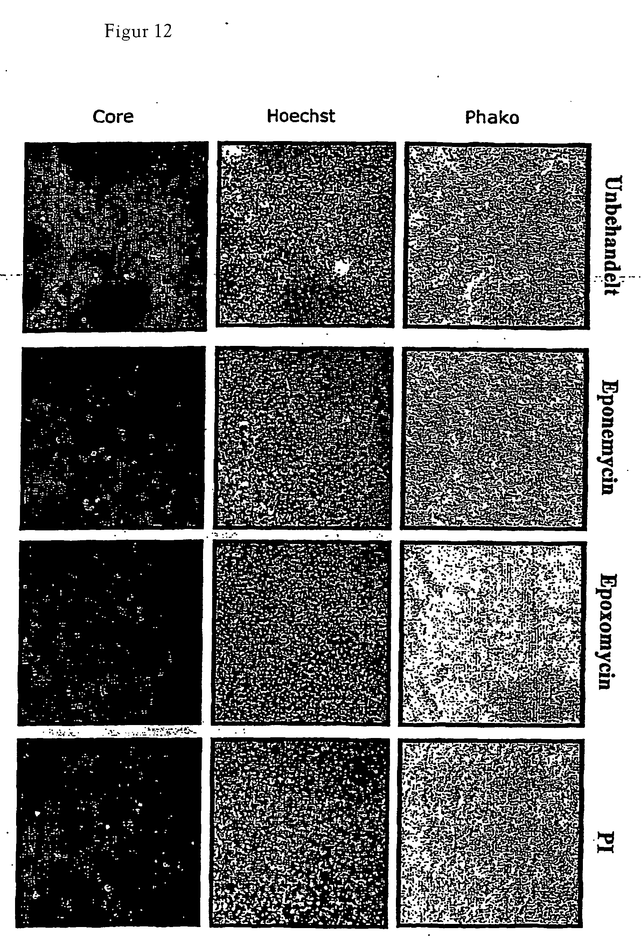

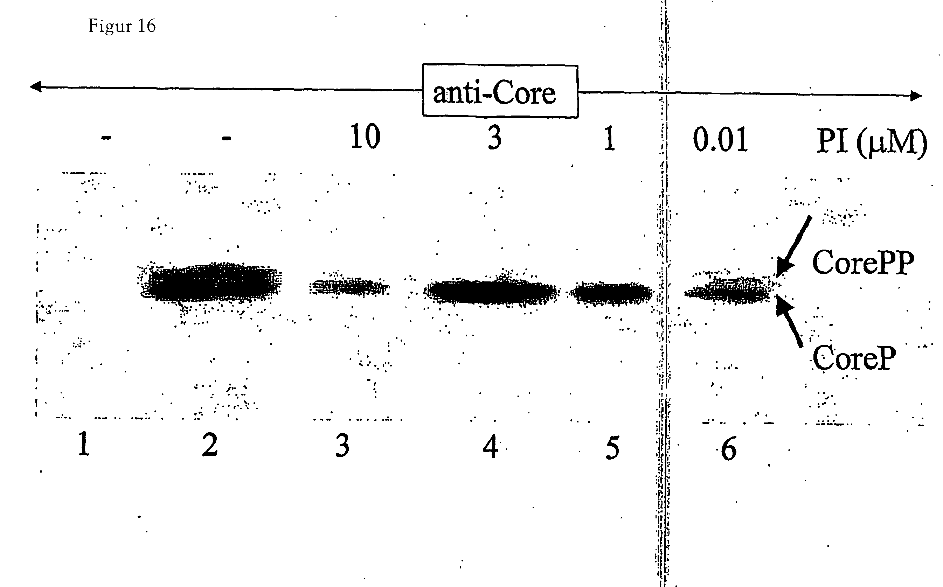

In den vorhergehenden Ausführungsbeispielen wurde gezeigt, dass Proteasom-Inhibitoren nicht nur die Sekretion neuer viraler und subviraler Partikel aus chronisch DHBV-infizierten Hepatozyten hemmen, sondern darüber hinaus auch die Infektiosität der wenigen, noch freigesetzten DHB-Virionen praktisch vollkommen blockieren. Daher kann angenommen werden, dass Proteasom-Inhibitoren die Ausbreitung einer bereits etablierten Infektion (durch freigesetzte Nachkommensviren aus bereits infizierten Hepatozyrten) auf nicht infizierte Zellen, die so genannte Sekundärinfektion, verhindern kann. Dieser Effekt wurde in folgendem Experiment untersucht: Primäre Hepatozyten aus DHBV-negativen Tieren wurden exakt nach der im Beispiel 1 beschriebenen Methodik präpariert 4 Tage nach Aussaat der Zellen in 12 well-Mikrotiterplatten (ca. 8x10⁵ Zellen/well) wurde das Kulturmedium erneut gewechselt und die Zellen mit einer MOI von 20 infiziert. Nach 16 h Inkubation wurden die Zellen mit PBS gewaschen und anschließend in neuem Medium für weitere 3 Tage weiter kultiviert. In diesem Zeitraum findet nur die Etablierung der Primärinfektion statt. Danach wurde das Medium erneut gewechselt, und zu parallelen Kulturen wurden jeweils 1 µM der Proteasom-Inhibitoren Eponemycin oder Expoxomicin zugegeben, und die Kulturen für weitere 3 Tage inkubiert. Entsprechend des oben beschriebenen Effektes von Proteasom-Inhibitoren sollten die behandelten primär infizierten Zellen im Vergleich zu nicht behandelten Zellen sowohl eine geringere Expression der viralen Antigene zeigen als auch (wegen der Blockade der Sekundärinfektion) die Zahl der infizierten Zellen deutlich verringert sein. Um dies zu prüfen, wurden die Zellkulturen fixiert und die Zahl der Core-positiven Zellen und deren Expressionshöhe auf Einzelzellebene durch indirekte Immunfluoreszenz mit anti-Core Antikörpern bestimmt. In der Tat hemmen die eingesetzten Proteasom-Inhibitoren sowohl die Erhaltung einer bereits etablierten, produktiven Infektion (offensichtlich in der Figur 16 (Core, PI, Epone- und Epoxomicin), welches an der sehr viel geringeren Anzahl von Core-positiven Zellen im Vergleich zu unbehandelten Kulturen (Figur 12, unbehandelt) ablesbar ist. Zum Vergleich sind die Phasenkontrsstaufnahmen (Figur 12, Phako) als auch die Hoechstfärbung (Figur 12, Hoechst) der gleichen Bildausschnitte gezeigt.

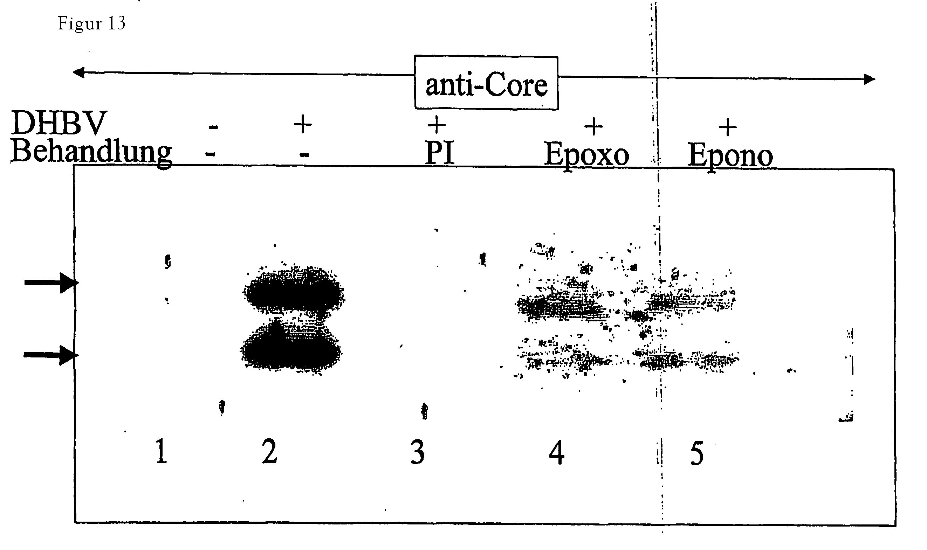

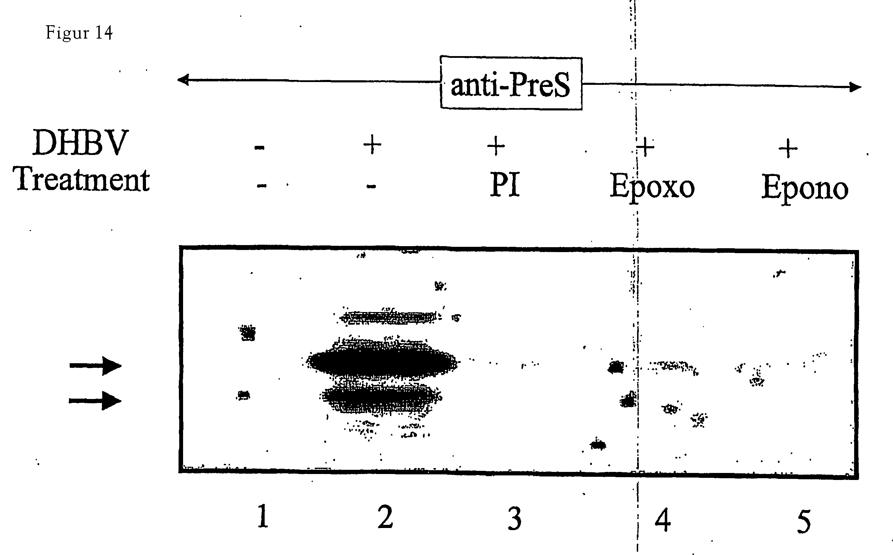

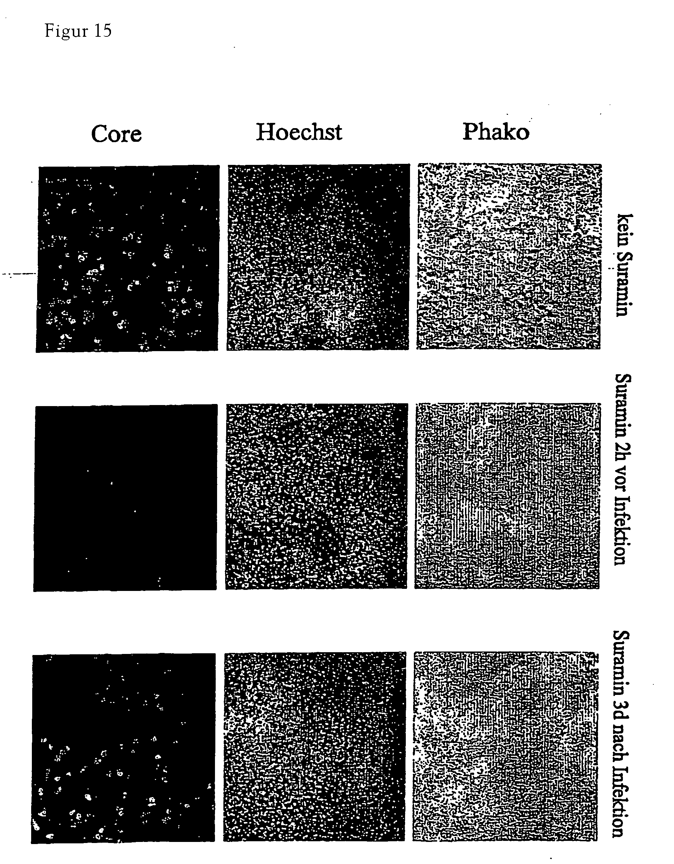

Um herauszufinden, ob Proteasom-Inhibitoren die Freisetzung des sogenannten e-Antigens, welches für die Etablierung einer chronischen Infektion notwendig ist (Chen *et al.,* 1992), hemmen, wurde die Menge des e-Antigens im Medium mittels Immunoblot bestimmt und mit dem ebenfalls getestetem PreS-Antigen korreliert. Hierzu wurden die Proteine von je einem Aliquot der Zellkulturmedien (20 microl) in 12.5% SDS-PAGE aufgetrennt, auf Nitrozellulose transferiert und die e- und PreS-Antigenproteine durch Kaninchenantikörper gegen Core-Protein (kreuzreagierend mit dem e-Antigen, Breiner *et al.,* 2001) bzw. PreS durch ECL nachgewiesen. Wie in Figur 13 und 14 zu sehen, führt die Proteasom-Inhibitor-Behandlung zu einer signifikanten Reduktion der Menge des e- bzw. des PreS-Antigens im Medium. Die Coomasieblaufärbung der Gele ergab, dass die Proteasom-Inhibitoren spezifisch nur die Sekretion der getesteten viralen Proteine hemmen, jedoch nicht die bestimmter zellulärer Proteine.
Zusammenfassend zeigen diese Ergebnisse, dass die Behandlung mit Proteasom-Inhibitoren zu einer drastischen Reduktion der Freisetzung des e- und PreS-Antigens fuhrt.
Um zu zeigen, dass die geringere Zahl DHBV-positiver Zellen in den mit Proteasom-Inhibitoren behandelten Zellen in der Tat durch Blockade der Sekundärinfektion bedingt ist, wurden die Zellen drei Tage nach Infektion für drei Tage mit 100 microg/ml Suramin (Sigma, Deisenhofen, Deutschland) behandelt. Suramin, eine *in vivo* sehr toxische Substanz, blockiert bekanntlich die Sekundärinfektion, ohne dabei mit der bereits etablierten primären DHBV-Infektioa zu interferieren (Pugh und Simmons, 1994). Andererseits sind die mit Suramin vorbehandelten Hepatozyten resistent gegenüber einer DHBV-Infektion. Dementsprechend ergibt sich drei Tage nach der Primärinfektion in den mit Suramin behandelten Zellen (Figur 15*,* Suramin 3 d post Infektion) eine ähnlich intensive Core-Färbung von Zellen wie in den unbehandelten Kulturen (Figur 15, unbehandelt), jedoch ist die Zahl der Core-exprimierenden Zellen sehr stark verringert. Die Vorbehandlung der Zellen mit Suramin blockiert wie erwartet die Etablierung der primären Infektion vollständig (Figur 15, Suramin 2 h prä Infektion). Die korrespondierenden Phasenkonbustaufimhmen (Figur 15, Phako) und Höchstfärbungen (Figur 15, Höchst) der gleichen Bildausschnitte sind zum Vergleich ebenfalls gezeigt. Zusammenfassend zeigen diese Experimente, dass Proteasom-Inhibitoren, ähnlich wie Suramin, durch Blockade der Sekundärinfektion die Ausbreitung der DHBV-Infektion verhindern können. Darüber hinaus und im Gegensatz zu Suramin führt die Hemmung der Proteasom-Aktivität additiv zu einer Reduktion der viralen Genexpression in primär infizierten Hepatozyten. Die Effekte von Proteasom-Inhibitoren sowohl auf die Primär- als auch auf die Sekundärinfektion korrelieren eng mit dem Potential der eingesetzten unterschiedlichen Substanzklassen.
Insgesamt demonstrieren diese Experimente, dass Proteasom-Inhibitoren sowohl die Etablierung als auch die Persistenz einer primären Infektion hemmen. Darüber hinaus verhindern sie die Ausbreitung der primären DHBV-Infektion durch Blockierung der Nachkommenviren. Damit sind Proteasom-Inhibitoren geeignete Substanzen, um die Ausbreitung einer HBV-Infektion in vivo zu blockieren.

### Beispiel 4: Proteasom-Inhibitoren induzieren Hypophosphorylierung des Core-Proteins in chronisch DHBV-infizierten primären Hepatozyten.

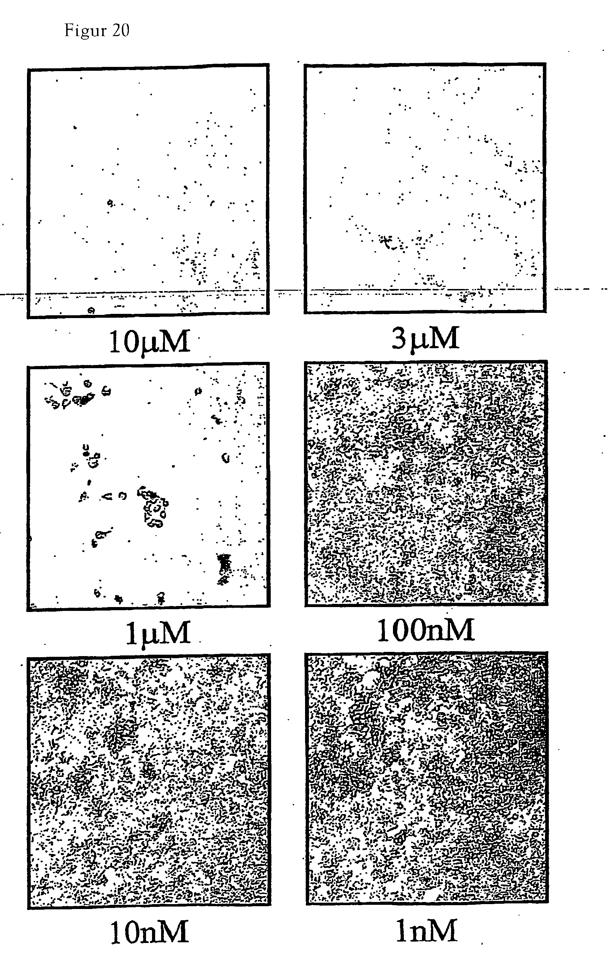

In den vorhergehenden Ausführungsbeispielen wurde gezeigt, dass Proteasom-Inhibitoren nicht nur die Sekretion neuer viraler und subviraler Partikel aus Hepatozyten hemmen, sondern darüber hinaus auch die Infektiosität der wenigen, noch freigesetzten DHB-Virionen praktisch vollkommen blockieren. Ein Grund für die massiv reduzierte Infektiosität von DHBV könnte auf die Proteasom-Inhibitor-vermittelten posttranslationalen Modifikationen der strukturellen Komponenten des Virus beruhen. Zum Beispiel würde eine Änderung des Phosphorylierungsgrades des Core-Proteins zu einem Defekt in der Destabilisierung der "incoming Cores" in der frühen Phase der Infektion führen.
Um den Einfluss der Behandlung mit Proteasom-Inhibitoren auf das intrazelluläre Phosphorylierungmuster des Core-Proteins zu testen, wurden ca. 7 Tage alte, chronisch DHBV-infizierte primäre Entenhepatozyten für 48 h mit steigenden Konzentrationen von PI 10 µM, 3 µM, 1 µM und 10 nM behandelt. Abschließend wurden die Zellen lysiert (50 mM Tris.HCl pH 7.5, 150 mM NaCl, 1% Triton X-100, 1% Natrium-Deoxycholat, komplementiert mit einem Protease-Inhibitor-Cocktail) (Complete, Roche, Mannheim)). Die Zelllysate wurden von der unlöslichen Fraktion durch Zentrifugation (14,000 rpm, 10 min, 4°C) getrennt und jeweils 20 microl der geklärten Fraktion wurden nach Zugabe von Laemmli-Puffer durch Kochen für 5 min denaturiert. Anschließend wurden die Lysate in 12.5% SDS-PAGE aufgetrennt und mittels Semi-Dry Transfertechnik (Biorad, München, Deutschland) auf Nitrozellulose elektro-transferiert. Die Membranen wurden mit 5% Magermilch geblockt und nach Waschen mit TBS mit Antikörpern gegen Core-Protein (Breiner *et al.,* 2001) inkubiert (Figur 16). Die markierten viralen Proteine wurden anschließend mittels Chemolumineszenz sichtbar gemacht. Die Figur 20 zeigt, dass in allen behandelten Proben das Verhältnis von hyperphosphoryliertem Core- zu weniger oder nicht phosphoryliertem Core-Protein zugunsten des letzteren verschoben ist. Es ist bemerkenswert, dass zum Beispiel 4 nanoM PI überhaupt keinen Effekt auf die intrazelluläre virale Proteinexpression hat (Figur 5, Spur 6; Figur 6, Spur 6), aber immer noch einen deutlichen Effekt auf den Phosphorylierungsgrad des Core-Proteins (Figur 16, Spur 6) ausübt.
Im Ergebnis dieser Untersuchung wird daher festgestellt, dass Proteasom-Inhibitoren zu einer Veränderung in der Modifizierung des Nukleokapsidproteins fuhren und dies wahrscheinlich die mechanistische Gmndlage für die reduzierte Infektiosität der aus den mit Proteasom-Inhibitoren behandelten Zellen sekretierten DHB-Viren ist

### Beispiel 5: Primäre Entenhepatozyten sind relativ resistent gegenüber den toxischen Effekten von Proteasom-Inhibitoren.

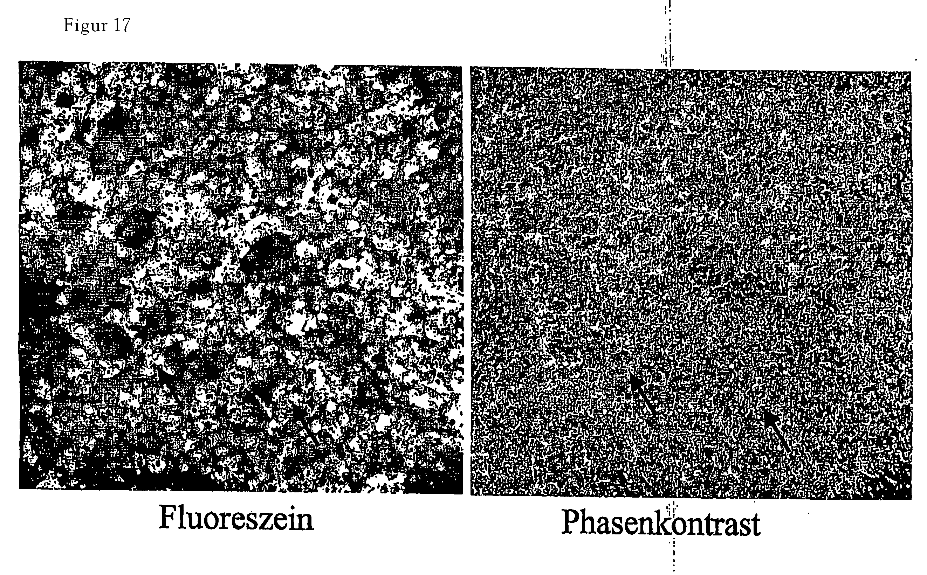

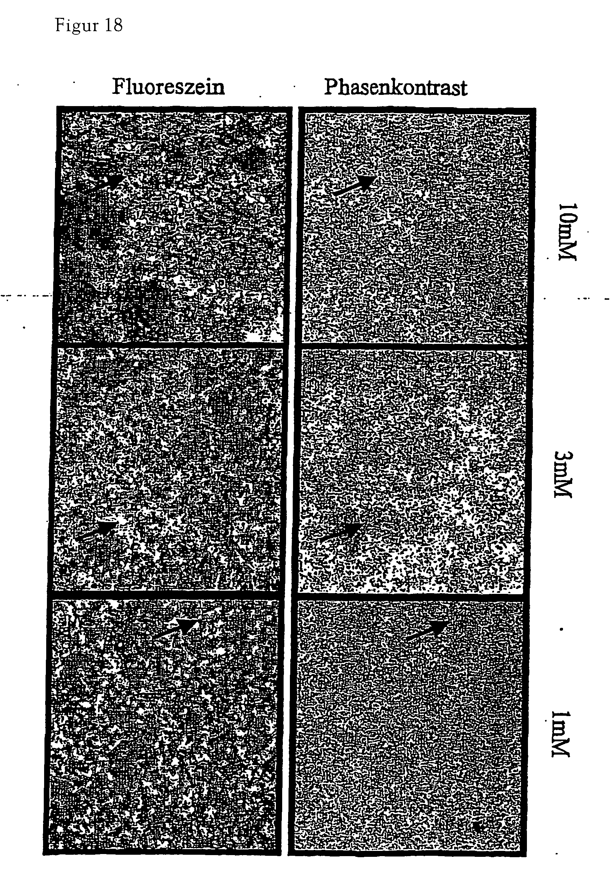

Da das UPS in zahlreichen zellulären Mechanismen involviert ist, ist eine vollständige Inhibition der Proteasom-Aktivität auf längere Zeit nicht mit der Vitalität einer Zelle vereinbar. Jedoch zeigen verschiedene Zelltypen unterschiedliche Sensitivität gegenüber der toxischen Wirkung von Proteasom-Inhibitoren. Dabei ist auffällig, dass sich rasch teilende und/oder aktivierte Zellen in der Regel eine höhere Sensitivität gegenüber Proteasom-Inhibitoren besitzen im Vergleich zu ruhenden und/oder nicht aktivierten Kulturen. Darauf beruht die anti-neoplastische Wirkung der Proteasom-Inhibitoren. Um die Toxizität von Proteasom-Inhibitoren in primären Entenhepatozyten im Vergleich zu humanen transformierten Hepatoblastoma-Zellen zu bestimmen (siehe Beispiel 6), wurden Dosis-Limitierungsstudien mit Proteasom-Inhibitoren durchgeführt. Hierzu wurden primäre Hepatozyten, wie im Beispiel 1 bereits beschrieben, aus Entenembryonen gewonnen, mit einer Dichte von ca. 8x10⁵/well in 12 Well-Mikrotiterplatten ausgesät und für 7 Tage kultiviert. Die Vitalität der Zellen wurde lichtmikroskopisch überprüft Parallel Kulturen wurden mit steigenden Dosen an PI (jeweils 10 microM, 3 microM, 1 microM, 10 nanoM und 1 nanoM) für 48 h behandelt. Etwa alle 12 h wurden die Zellen hinsichtlich Morphologie und Vitalität lichtmikroskopisch untersucht. Zusätzlich wurde deren Funktionalität mittels Fluoreszenzvitalfärbung mit Fluoreszeindiazetat (FDA) (Sigma, Deisenhofen, Deutschland) bestimmt (Yagi *et al.,* 2001). FDA wird aktiv und präferenziell von Hepatozyten aufgenommen und dort mittels einer Lipase, die exklusiv in Hepatozyten exprimiert wird, in Fluoreszein konvertiert und bewirkt die Fluoreszenzfärbung des Zytoplasmas, ein Zeichen für die Funktionsfähigkeit und Vitalität von Hepatozyten. In diesem Experiment wurden die behandelten und unbehandelten primären Entenhepatozyten mit FDA-haltigem Williams E Medium (5 microg/ml) für 5 min bei 37°C inkubiert. Anschließend wurden die Zellen mit PBS gewaschen, die vtaliät der Hepatozyten mittels eines inversen Epifluoreszenzmikroskops beurteilt und mit dem Bildverarbeitungssystems Openlab weiter prozxssiert. Figur 17 zeigt, dass alle Kulturen, die mit bis zu 1 microM PI behandelt wurden, weder eine morphologische Veränderung noch Hinweise auf eingeschränkte Vitalität zeigten, d.h. nicht zu unterscheiden waren von nicht behandelten Hepatozyten (Figur 18). Bei Behandlung mit 3 microM und 10 microM der PI wurden in den Nicht-Hepatozyten, die in Kulturen von primären Hepatozyten immer mit vorliegen, deutliche morphologische Veränderungen wie zum Beispiel abgerundete Zellen und intrazelluläre Vakuolenbildung beobachtet. Die Vitalität der Hepatozyten dagegen war nicht verändert gemäß visueller Beurteilung und der Fluoreszenzintensität. Erst ab einer Konzentration von 10 microM PI traten erste Zeichen einer toxischen Wirkung auch in Hepatozyten auf (Figur 17). Ähnliche Ergebnisse wurden mit unterschiedlichen PI, wie zum Beispiel Lactazystin und Epoxomicin, beobachtet
Zusammengefasst kann festgestellt werden, dass primäre Entenhepatozyten relativ hohe Konzentrationen von bis zu ca. 10 µM Proteasom-Inhibitor tolerieren können, während proliferierende Leberkarzinomzellen (siehe Beispiele 6) und andere proliferierende Zellen in der Hepatozyten-Kultur sehr viel sensitiver gegenüber der toxischen Wirkung von Proteasom-Inhibitoren sind (Figur 1, 17, 19, 20 und 21).

### Beispiel 6: Effekt von Proteasom-Inhibitoren auf das Zellwachstum und die Vitalität der humanen Hepatoma-Zellen, HepG2.

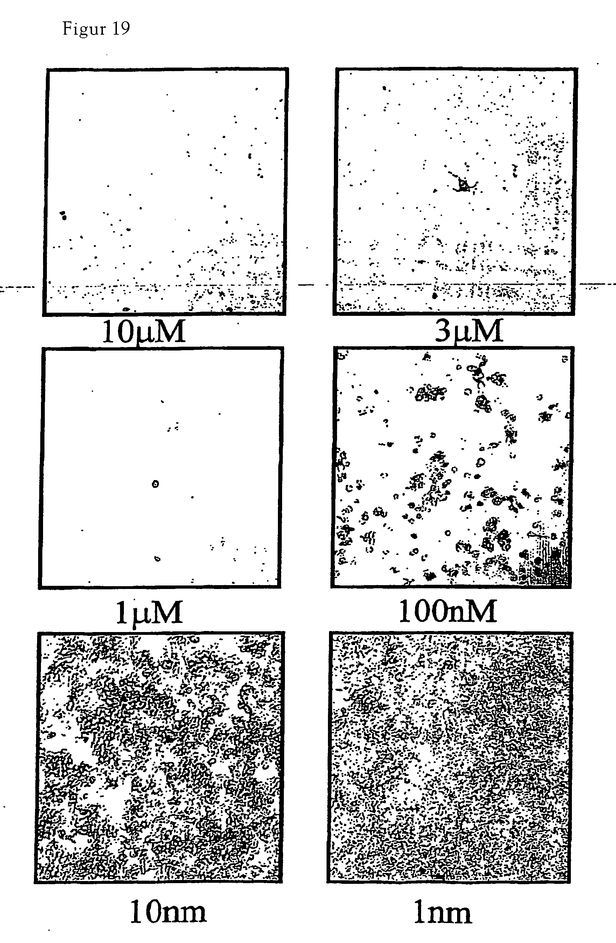

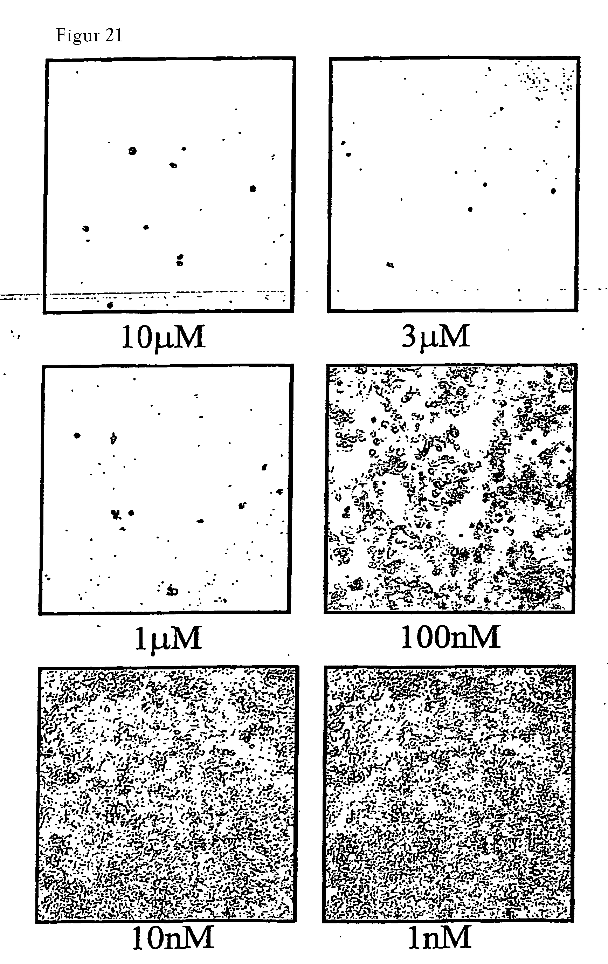

Die humane Hepatoma-Zelllinie HepG2 wurde in Dulbeccos' modifizierten Eagle's Medium (DMEM) (GIBCOBRL, Paisley, Scotland) suplementiert mit 10% fötalem Kälberserum (Biochrom, Berlin, Deutschland), 2 milliM L-Glutamin (GIBCOBRL, Paisley, Scotland), 100 Einheiten ml⁻¹ Penicillin und 100 microg ml⁻¹ Streptomycin (Biochrom, Berlin, Deutschland) bei 37°C kultiviert. Nach Passagierung mit 0.25% Trypsin und 1 milliM EDTA (GIBCOBRL, Paisley, Scotland) wurden die Zellen in einer Dichte von 0.5x10⁶ /well in 12 well-Mikrotiterplatten ausgesät. 24 h später wurde das Medium gewechselt, und die Zellen wurden mit PI, Eponemycin und Epoxomicin, jeweils in Konzentrationen von 10 microM, 3 microM und 1 microM sowie 100 nanoM, 10 nanoM, und 1 nanoM für 48 h behandelt und anschließend mit Trypanblaufärbung auf ihre Vitalität getestet und mittels Lichtmikroskopie morphologisch begutachtet. Bis zu einer Konzentration von 10 nanoM war keiner der eingesetzten Proteasom-Inhibitoren toxisch und anti-proliferativ (Figur 19, 20 und 21). Toxische Erscheinungen traten ab einer Konzentration von 100 nanoM bei PI (Figur 19) und Epoxo- (Figur 21) auf, während Eponemycin (Figur 20) noch gut vertragen wurde. Bei 1 microM und höher waren alle getesteten Substanzen hochtoxisch (Figur 19, 20 und 21). Dabei wurden Effekte wie Abrundung und Ablösung der Zellen, Degeneration sowie Apoptose beobachtet.

Zusammengefasst kann festgestellt werden, dass proliferierende Leberkarzinoma-Zellen sehr viel sensitiver gegenüber der toxischen Wirkung von Proteasom-Inhibitoren sind, während primäre Entenhepatozyten relativ hohe Konzentrationen von bis zu ca. 10 microM Proteasom-Inhibitoren tolerieren können (Figur 17, 19, 20 und 21).

### Beispiel 7: Effekt von Proteasom-Inhibitoren auf die Sekretion von HBV-spezifischen Antigenen in humanen Hepatoma-Zelllinien.

Primäre humane Hepatozyten wurden wie beschrieben aus menschlichen Lebern gewonnen (Dandri *et al.,* 2001) und mit humanem HBV infiziert, welche aus dem Medium von HepG2.2.15-Zellen, die infektiöses humanes Hepatitis-B-Virus produziert (Sells *et al.,* 1987), geerntet wurden. Parallele Kulturen der HepG2-Zellen wurden für zwei Tage mit PI behandelt, oder nicht behandelt. Die HepG2-Zellen wurden vor der Behandlung mit replikationskompeteten HBV-Genomen wie beschrieben (Kalinina *et al.,* 2001) transfiziert. Im Gegensatz zu zahlreichen HBV Core-positiven primären Hepatozyten (analysiert durch indirekte Immunfluoreszenzfärbung mit anti-Core Antikörpern), welche auf eine erfolgreiche Infektion von primären humanen Hepatozyten mit Viren aus den nicht behandelten Zellen schließen lassen, wurde keine Core-positive primäre humane Hepatozyten nach Infektion mit Nachkommenviren aus Proteasom-Inhibitor-behandelten HepG2 Zellen gefunden. Im Ergebnis dieses Experimentes kann damit festgestellt werden, dass Proteasom-Inhibitor-Behandlung ähnlich der Wirkung auf DHBV ebenfalls die Freisetzung und Infektiosität von HBV blockiert.
Generell beinhalten späte Prozesse des Replikatonszyklus die Neusynthese von viralen Strukturproteinen in der infizierten Zelle, die korrekte Faltung und Modifizierung sowie den Transport der Strukturprotein zu dem Ort der Virusassemblierung, gefolgt von Virusfreisetzung an der Zellmembran und der proteolytischen Prozessierung der Gag-Polyproteine im reifenden Viruspartikel durch die virale Protease. Am Beispiel der Humanen Immundefizienzviren wie auch an Hepatitis-B-Viren wurde gezeigt, dass die verschiedenen Inhibitoren des 26S Proteasoms sowohl die Freisetzung von Viruspartikeln als auch die Infektiosität der freigesetzten Viruspartikel reduzieren. Die biochemischen Untersuchungen zeigen, dass Proteasom-Inhibitoren spezifisch die Reifung und proteolytische Prozessierung der Gag-Proteine blockieren und bei Hepatitis-B-Viren auch die posttranslationale Modifikation des Nukleokapsids ändern und die Sekretion des e-Antigens reduzieren. Morphologische Untersuchungen zeigen, dass sich im Falle von HIV-1 in Gegenwart von Proteasom-Inhibitoren unreife Viruspartikel an der Zellmembran konzentrieren. Virologische Untersuchungen zeigen, dass Proteasom-Inhibitoren die Ausbreitung einer HTV-1-Infektion wie auch eine HBV-Infektion in der Zellkultur verhindern. Mechanistische Untersuchungen zeigen weiterhin, dass Proteasom-Inhibitoren keinen direkten Effekt auf die virale Protease von HIV-1 ausüben, sondern zelluläre Mechanismen beeinflussen, welche für die effiziente. Reifung und Freisetzung von Viruspartikeln notwendig sind.
Die Verwendung von Proteasom-Inhibitoren stellt eine neuartige Methode zur Intervention viraler Replikationszyklen dar. Da das Target dieser Methode konservierte zelluläre Mechanismen, dass heißt die enzymatische Aktivität des Proteasom-Komplexes selbst darstellt, sind bei einer *in vivo* Applikation von Proteasom-Inhibitoren keine durch virale Mutationen vermittelte Resistenzmechanismen zu erwarten.
Die Erfindung betrifft des weiteren die Verwendung in der Grundlagenforschung, z.B. die Analyse der Assemblierung, Freisetzung und Reifung von Retroviren, speziell späte Prozesse im HIV-Replikationszyklus; ferner
- die Grundlagenforschung und angewandten Forschung,
- das Verständnis der Retrovirusassemblierung,
- das Verständnis der Wirkungsweise der viralen Proteasen,
- das Verständnis der Gag-Prozessierung von Retroviren,
- das Verständnis zellulärer Mechanismen, involviert in den späten Prozess der RetrovirusAssemblierung, insbesondere von Faktoren des Ubiqutin-Proteasom-Systems, von Ubiquitinbindenden Faktoren,
- Fakoren, welche an ubiquitinierte Gag-Proteine von Retroviren binden,
- Fakoren, welche an mono-ubiquitinierte L (late assembly)-Domänen von retroviralen Gag Proteinen binden,
- zelluläre Faktoren, welche die mono-Ubiquitinierung von L-Domänen von Retroviren steuern, regulieren, beeinflussen und / oder hemmen,
- zelluläre Faktoren, welche die mono-Ubiqutinierung von L-Domänen in Gag-Proteinen von Retroviren durch De-Ubiquitinierung rückgängig machen,
- zelluläre Faktoren, welche späte Prozesse der Virusassemblierung, speziell der Ablösung von Viruspartikeln von der Zellmembran in Abhängigkeit der mono-Ubiquitinierung von L-Domänen in retroviralen Gag-Proteinen steuern, regulieren, beeinflussen und / oder hemmen sowie
- das Verständnis bei der Entwicklung von weiteren Substanzen, welche die retrovirale Gag-Prozessierung sowie die Assemblierung und die Freisetzung von Retroviren durch Beeinflussung der Wechselwirkung von retroviralen Gag-Proteinen mit dem Ubiquitin-Proteasom-System steuern, regulieren, beeinflussen und / oder hemmen können.

Weitere Anwendungsgebiete der vorliegenden Erfindung sind:
- die Vorbeugung und Behandlung/Therapie von Erkrankungen, die durch Retrovirusinfektion verursacht werden, speziell die anti-retrovirale Therapie und Vorbeugung bei Infektionen mit Immundefizienz verursachenden Lentiviren, vor allem die erworbene Immundefizienz bei Tieren und Menschen, wie die Behandlung von HIV-infizierten Individuen - symptomloser wie auch AIDS-Patienten, die Vorbeugung einer HIV-Infektion und die Verhinderung einer HIV-Infektion unmittelbar nach Kontakt mit HI-Viren;
- die Entwicklung von neuen Pharmaka, speziell auf der Basis von Substanzen, die das 26S Proteasom inhibieren, wie Proteasom-Inhibitoren, die *in vivo* applizierbar sind und eine geringe Toxizität aufweisen und dabei gleichzeitig die Replikation von Retroviren im Organismus unterdrücken und die Infektionsausbreitung verhindern;
- die Applikation von retroviralen Vektoren für den Einsatz bei Gentransfennethoden in der Gentherapie, speziell der Anwendung von Proteasom-Inibitoren zur Verhinderung der Entstehung und Ausbreitung rekombinanter und/oder ungewollter replikarionskompetenter Retroviren nach Gentransfer,
- die Virologie, Zellbiologie, Gentherapie, Pharmakologie, Naturstoffchemie, Peptidchemie, molekulare HIV- und angewandte AIDS-Forschung.

### Legende zu den Figuren

### Figur 1: Nichtparenchymale Zellen einer primären Hepatozytenkultur sind sensitiver gegenüber den toxischen Effekten von Proteasom-Inhibitoren.

Gezeigt ist eine primäre Hepatozytenkultur, welche mit 1 microM PI für 72 h behandelt worden ist. Anschließend wurden die Zellen fixiert und die Kerne mit Hoechst gefärbt (blau). Die korrespondierende Phasenkontrastaufnahme ist gegenüberstellt. Das obere Phasenkontrastbild zeigt eine ehemalige Insel aus Nicht-Hepatozyten, während das untere die gefärbten Kerne desselben Bildausschnittes wiedergibt. Die kleinen Pfeile zeigen auf die apoptotischen nichtparenchymalen Zellen, während der Blockpfeil auf eine intakte, kleine Hepatozytenkolonie zeigt.

### Figur 2: Proteasom-Inhibitoren hemmen konzentrationsabhängig die Freisetzung von PreS-haltigen Viruspartikeln in chronisch DHBV-infizierten primären Entenhepatozyten - PreS dot-blot- Nachweis.

Eine 7 Tage alte, chronisch DHBV-infizierte primäre Entenhepatozytenkultur wurde für 48 h mit steigenden Konzentrationen von PI (1 nanoM, 1 microM und 10 microM) behandelt bzw. nicht behandelt. 200 microl der jeweiligen Überstände wurden aufgedottet. Die Menge der freigesetzten viralen Partikeln wurde mittels eines PreS-Antigen spezifischen dot-blots bestimmt.

### Figur 3: Proteasom-Inhibitoren hemmen konzentrationsabhängig die Freisetzung von PreS-haltigen Viruspartikeln in chronisch DHBV-infizierten primären Entenhepatozyten - PreS Western blot-Nachweis.

Eine 7 Tage alte, chronisch DHBV-infizierte primäre Entenhepatozytenkultur wurde für 48h mit steigenden Konzentrationen von PI (1 nanoM, 10 nanoM, 1 microM, 3 microM und 10 microM) behandelt bzw nicht behandelt. Die 5 microL der jeweiligen Überstände wurden in 12.5% SDS-PAGE aufgetrennt. Die Darstellung der PreS-Protein-Banden (p36 und p28) erfolgte durch Western blot.

### Figur 4: Proteasom-Inhibitoren hemmen konzentrationsabhängig die Freisetzung von DNA-haltigen Viruspartikeln in chronisch DHBV-infizierten primären Entenhepatozyten (PEH) - DNA-dot-blot-Nachweis.

Eine 7 Tage alte chronisch DHBV-infizierte primäre Entenhepatozytenkultur wurde für 48 h mit steigenden Konzentrationen von PI (10 nanoM, 1 microM, 3 microM und 10 microM) behandelt bzw. nicht behandelt, der Überstand gesammelt und durch Zentrifugation geklärt. Danach wurde der Überstand auf Nitrozellulose aufgedottet und die Membran mit einer ³²P-markierten DHBV-DNA-Sonde hybridisiert und autoradiographiert. Dots von nicht infizierten PERs sind mit - gekennzeichnet, dots von infizierten Hepatozyten mit +. Die Standardisierung des DNA-dot-blots erfolgte durch Auftragung von bekannten Konzentrationen an klonierter DHBV-DNA (Standard DHBV-DNA).

### Figur 5: Proteasom-Inhibitoren haben keinen nennenwerten Einfluss auf die intrazelluläre PreS-Genexpression.

7 Tage alte chronisch DHBV-infizierte primäre Entenhepatozytenkulturen (PEHs) wurden für 48 h mit steigenden Dosen PI behandelt. Zelllysate wurden in 12.5% SDS-PAGE aufgetrennt und zum Nachweis von PreS-Antigen geblottet. In Spuren 1 und 2 sind unbehandelte, nicht infizierte (-PEHs) bzw chronisch DHBV-infizierte Hepatozyten (+PEHs) aufgetragen. Spuren 3 bis 7 entsprechen Aufträgen von mit unterschiedlichen Dosen an PI-behandelben Zellen. p36 und p28 entsprechen dem PreS-Protein voller Länge bzw. einem Degradationprodukt desselben.

### Figur 6: Proteasom-Inhibitoren haben keinen nennenswerten Einfluss auf die Core-Expression.

7 Tage alte chronisch DHBV-infizierte primäre Entenhepatozyteakulturen wurden für 48 h mit steigenden Dosen PI behandelt. Zelllysate wurden in 12.5% SDS-PAGE aufgetrennt und das Core-Protein (Pfeil) durch indirekte Chemolumineszenz dargestellt. In Spuren 1 und 2 sind nicht behandelte DHBV-negative (-PEHs) bzw. chronisch DHBV-infizierte Hepatozyten (+PEH) aufgetragen. Spuren 3 bis 7 entsprechen Aufträgen von mit unterschiedlichen Dosen an PI-behandelten Zellen.

### Figur 7: Akkumulation von poly-ubiquitinierten Proteinen in mit Proteasom-Inhibitoren behandelten primären Entenhepatozyten.

Eine Woche alte, chronisch DHBV-infizierte primäre Entenhepatozyten wurden für 48 h mit steigenden Konzentrationen von PI (Spuren 2 bis 6) behandelt. Als Kontrolle wurden DHBV infizierte (+PEHs, Spur 1) und Virus-freie Zellen ohne PI kultiviert (-PEHs, Spur 7). Anschließend wurden Zelllysate in 6% SDS-PAGE aufgetrennt und mono- sowie poly-ubiquitinierte Proteine mittels Kaninchen-anti-Ubiquitin nachgewiesen. Rechts ist die jeweilige Position der Proteinmarkerbanden in kDA angegeben. Polyubi. zeigt die hochmolekularen poly-ubiquitinierten Proteine an.

### Figur 8: Überstände der mit Proteasom-Inhibitoren behandelten chronisch DHBV-infizierten primären Entenhepatozyten-Kulturen (PEH) enthalten keine infektiösen Nachkommenviren.

7 Tage alte chronisch DHBV-infizierte primäre Entenhepatozyten wurden für 48 h mit 10 microM PI behandelt und anschließend der geerntete Zellkulturüberstand für die Neuinfektion von DHBV-negativen PEH-Kulturen benutzt. 60 h nach der Infektion wurden die Zellen gewaschen und fixiert. Abschließend erfolgte die Färbung der Zellen für PreS (grün). Die Darstellung der Zellkerne erfolgte mittels Hoechstfärbung (blau). Der obere bzw. untere Bereich der Figur ist eine niedrige bzw höhere Vergrößerung desselben Bildauschnittes.

### Figur 9: Nachkommenviren aus chronisch DHBV-infizierten primären Entenhepatozyten sind infektiös.

Zellkulturüberstände einer 7 Tage alten chronisch infizierten primären Entenhepatozytenkultur wurden gesammelt und für die Neu-Infektion von DHBV-negativen primären Entenhepatozyten benutzt. 60 h nach der Infektion wurden die Zellen fixiert. Abschließend erfolgte die Färbung der Zellen für PreS (grün). Die Darstellung der Zellkerne erfolgte mittels Hoechstfärbung (blau). Der untere Bereich der Figur ist die höhere Vergrößerung desselben oben gezeigten Bildauschnittes.

### Figur 10: Überstände der mit Proteasom-Inhibitoren behandelten DHBV-infizierten Hepatozyten enthalten keine infektiöse Nachkommenviren - Nachweis durch PreS-blot.

7 Tage alte chronisch DHBV-infizierte primäre Entenhepatozyten (PEH) wurden für 48 h mit 10 microM PI behandelt und anschließend der Überstand gesammelt, durch Zentrifugation geklärt und für de novo Infektion von PEHs benutzt. 60 h nach der Infektion wurden die Zellen lysiert und in 12.5% SDS-PAGE aufgetrennt. Die Darstellung der PreS-Proteinbanden erfolgte durch Western blot. In Spuren 1 und 2 sind DIHBV-negative bzw. chronisch DHBV-infizierte PEH aufgetragen. Spur 3 und 4 entsprechen PEHs, welche mit Überständen aus nicht behandelten bzw. PI-behandelten Kulturen infiziert wurden. Aufgetragen in Spur 5 sind PEHs, welche mit einer MOI von 20 infiziert wurden.

### Figur 11: Überstände der mit Proteasom-Inhibitoren behandelten DHBV-infizierten Hepatozyten enthalten keine infektiösen Nachkommenviren - Nachweis durch Core blot.

7 Tage alte chronisch DHBV-infizierte primäre Entenhepatozyten (PEH) wurden für 48 h mit 10 microM PI behandelt und anschließend der Überstand gesammelt, durch Zentrifugation geklärt und für Neuinfektion von PEH-Kulturen benutzt. 60 h nach der Infektion wurden die Zellen lysiert und das Lysat durch 12.5% SDS-PAGE aufgetrennt. Die Darstellung des Core-Proteins erfolgte durch Western blot. In Spuren 1 und 2 sind DHBV-negative bzw. chronisch DHBV-infizierte PEH aufgetragen. Spur 3 und 4 entsprechen PEH, welche mit Überständen aus nicht behandelten bzw. PI-behandelten Kulturen infiziert wurden. Aufgetragen in Spur 5 sind PEHs, welche mit einer MOI von 20 infiziert worden sind.

### Figur 12: Hemmung der Primär- und Sekundärinfektion durch Behandlung mit Proteasom-Inhibitoren von primären Entenhepatozyten (PEH) infiziert mit DHBV.

Vier Tage alte Kulturen von PEH wurden mit einer MOI von 20 für 16 h infiziert. Anschließend wurden die Zellen gewaschen und im neuen Medium für weitere drei Tage kultiviert. Am 3. Tag nach der Infektion wurden die Zellen entweder ohne Proteasom-Inhibitoren kultiviert (unbehandelt) oder mit jeweils 1 microM Eponemycin, Epoxomicin oder PI für weitere 3 Tage behandelt. Am 6. Tag der Infektion wurden die Zellen gewaschen und fixiert Anschließend erfolgte die Färbung der Zellen für Core-Protein (grün). Die Zellkerne wurden mit Hoechst gefärbt (blau). Die Phasenkontrastaufnahmen der jeweiligen Bildausschnitte sind ebenfalls zu sehen.

### Figur 13: Proteasom-Inhibitoren hemmen die Freisetzung des e-Antigens in DHBV-infizierten primären Entenhepatozyten.

Vier Tage alte primäre Entenhepatozyten (PEH) wurden mit einer MOI von 20 für 16 h infiziert Anschließend wurden die Zellen gewaschen und im neuen Medium für weitere drei Tage kultiviert. Am 3. Tag nach der Infektion wurden die Zellen mit jeweils 1 microM PI (Spur 3) Epoxomicin (Spur 4), Eponemycin (Spur 5) für weitere drei Tage behandelt. Am 6. Tag nach der Infektion wurde der Überstand gesammelt, durch Zentrifugation geklärt. Ein Aliquot der Überstände wurde in 12.5% SDS-PAGE aufgetrennt Die Darstellung der e-Antigen-Protein-Banden (Pfeile) erfolgte durch indirekte Chemoluminescenz. In Spuren 1 und 2 sind nicht behandelte negative bzw. chronisch DHBV-infizierte PEH, in Spuren 3 PI, 4 Epoxomicin und 5 Eponemycin behandelte, DHBV infizierte PEH aufgetragen.

### Figur 14: Proteasom-Inhibitoren hemmen die Freisetzung des PreS-Antigens in DHBV-infizierten primären Entenhepatozyten.

Vier Tage alte Hepatozyten-Kulturen wurden mit einer MOI von 20 für 16 h infiziert. Anschließend wurden die Zellen gewaschen und im neuen Medium für weitere drei Tage kultiviert. Am 3. Tag nach der Infektion wurden die Zellen mit jeweils 1 microM PI (Spur 3), Epoxomicin (Spur 4) und Eponemycin (Spur 5) für weitere drei Tage behandelt. Am 6. Tag nach der Infektion wurde der Überstand gesammelt und in 12.5% SDS-PAGE aufgetrennt. Die Darstellung der PreS-Protein-Banden (p36 und p28) erfolgte im Western blot. In Spuren 1 und 2 sind nicht behandelte negative bzw. chronisch DHBV-infizierte PEH aufgetragen, DHBV infizierte PEH wurden aufgetragen in Spur 3 (PI), in Spur 4 (Epoxomicin), und in Spur 5 (Eponemycin).

### Figur 15: Suramin hemmt sowohl die primäre als auch die sekundäre DHBV-Infektion in primären Entenhepatozyten.

Vier Tage alte PEH wurden 2 h vor der Infektion mit 100 microg/ml Suramin behandelt (Suramin Fräinfektion) oder zunächst unbehandelt belassen (kein Suramin und Suramin 3d post Infektion). Anschließend wurden alle Zellen mit einer MOI von 20 infiziert. Nach 16 h Inkubation wurden die Kulturen gewaschen und mit neuem Medium weiter kultiviert. Am 3. Tag nach der Infektion wurde das Medium erneut gewechselt und die Zellen in "kein Suramin" und "Suramin prä Infektion" wurden für weitere drei Tage ohne und die Zellen in "Suramin 3d post Infektion" mit 100 microg/ml Suramin behandelt. Am 6. Tag der Infektion wurden die Zellen gewaschen und fixiert. Anschließend erfolgte die Färbung der Zellen für Core-Protein (Core). Die Zellkerne wurden mit Hoechst gefärbt (Hoechst). Die Phasenkontrastaufnahmen der jeweiligen Bildausschnitte sind ebenfalls gezeigt (Phasenkontrast).

### Figur 16: Proteasom-Inhibitoren induzieren die Hypophosphorylierung des intrazellulären Core-Proteins in chronisch DHBV-infizierten primären Hepatozyten.

7 Tage alte chronisch DHBV-infizierte primäre Entenhepatozytenkulturen wurden für 48 h mit steigenden Dosen PI behandelt und anschließend lysiert. Die Lysate wurden in 12.5% SDS-PAGE aufgetrennt und das Core-Protein durch indirekte Chemolumineszenz dargestellt In Spuren 1 und 2 sind nicht behandelte negative bzw. chronisch DHBV-infizierte PEH aufgetragen. Spuren 3 bis 6 entsprechen den mit PI behandelten Zellen. CorePP entspricht hyperphosphoryliertem und CoreP hypophosphoryliertem Core-Protein.

### Figur 17: Behandlung von primären Entenhepatozyten mit Proteasom-Inhibitoren schränkt nicht deren Vitalität und Funktionalität ein.

7 Tage alte primäre Zellkulturen aus chronisch DHBV-infizierten Entenlebern wurden 48 h lang mit steigenden Konzentration von PI (10 microM, 3 microM und 1 microM) behandelt. Am Ende dieser Behandlungsperiode wurden die Zellen für 5 min mit FDA-haItigem Vinlliams E Medium (5 microg/ml) bei 37°C inkubiert. Anschließend wurden die Zellen mit PBS gewaschen und die erfolgreiche Konversion des FDA in Fluoreszein in Hepatozyten mittels eines Epifluoreszensmikroskops beurteilt. Zellen mit grüner Färbung im Zytoplasma wurden als vital und funktionell angenommen. Die jeweiligen Fluoreszenz- (Fluoreszein) und die korrespondierenden Phasenkontrastaufnahmen (Phasenkontrast) mit der Angabe der benutzten PI-Dosis werden gezeigt. Die weißen Pfeile mit Spitze zeigen beispielhaft auf Nester von nichtparenchymalen Zellen, welche im Vergleich zu Hepatozyten keine bzw. nur beschränkte FDA-Aufnahme und Konversion zeigen.

### Figur 18: Aufnahme und Konversion des Vitalmarkers Fluoreszeindiazetat in Fluoreszein erfolgt bevorzugt in Hepatozyten.

Eine Woche alte primäre Zellkulturen aus chronisch DHBV -infizierten Entenlebem wurden für 5 min mit FDA-haltigem Williams E Medium (5 microg/ml) bei 37°C inkubiert. Anschließend wurden die Zellen mit PBS gewaschen und die erfolgreiche Konversion des FDA in Fluoreszein in Hepatozyten mittels eines Epifluoreszenzmikroskops beurteilt. Zellen mit grüner Färbung im Zytoplasma wurden als vital und funktionell angenommen (Fluoreszein). Die zu dem selben Bildausschnitt korrespondierende Phasenkontrastaufnahme ist ebenfalls gezeigt (Phasenkontrast). Die weißen Pfeile mit Spitze zeigen beispielhaft auf Nester von nichtparenchymalen Zellen, welche im Vergleich zu den Hepatozyten keine bzw nur beschränkte FDA-Aufnahme und Konversion zeigen.

### Figur 19: Behandlung von Hepatomazellen (HepG2) mit Proteasom-Inhibitoren schränkt deren Vitalität und Funktionalität ein.

HepG2-Zellen wurden 48 h lang mit steigenden Konzentration (10 microM, 3 microM und 1microM, 100 nanoM, 10 nanoM und 1 nanoM) von PI (Abschnitt A), behandelt. Am Ende dieser Behandlungsperiode wurden die Zellen mit Trypanblau gefärbt und mit einem Durchlichtmikroskop begutachtet. Zellen mit dunkelblauer Färbung wurden als nicht-vital angenommen. Die jeweilige Konzentration des PI ist in den entsprechenden Phasenkontrastaufnahmen angegeben.

### Figur 20: Behandlung von Hepatomazellen (HepG2) mit Eponemycin schränkt deren Vitalität und Funktionalität ein.

HepG2-Zellen wurden 48 h lang mit steigenden Konzentration (10 microM, 3 microM und 1 microm, 100 nanoM, 10 nanoM und 1 nanoM) von Eponemycin (Abschnitt A), behandelt. Am Ende dieser Behandlungsperiode wurden die Zellen mit Trypanblau gefärbt und mit einem Durchlichtmikroskop begutachtet. Zellen mit dunkelblauer Färbung wurden als nicht-vital angenommen. Die jeweilige Konzentration des Eponemycin ist in den entsprechenden Phasenkonhastaufnahmen angegeben.

### Figur 21: Behandlung von humanen Hepatomazellen, HepG2 mit Epoxomicin schränkt deren Vitalität und Funktionalität ein.

HepG2-Zellen wurden 48 h lang mit steigenden Konzentration (10 microM, 3 microM und 1 microM, 100 nanoM, 10 nanoM und 1 nanoM) von Epoxomicin (Abschnitt A), behandelt. Am Ende dieser Behandlungsperiode wurden die Zellen mit Trypanblau gefärbt und mit einem Durchlichtmikroskop begutachtet. Zellen mit dunkelblauer Färbung wurden als nicht-vital angenommen. Die jeweilige Konzentration von Epoxomicin ist in den entsprechenden Phasenkontrastaufnahmen angegeben.

### Literatur

Adachi, A; Gentleman, HE; König, S, Folks; T, Willey, R; Rabson, A; Martin, MA (1986). Production of acquired immunodeficiency syndrome-associated retrovirus in human and nonhuman cells transfected with an infectious molecular clone. *J. Virol.* **59**:284-291.
Adams, J; Behnke, M; Chen, S; Cruickshank, AA; Dick, LR; Grenier, L; Klunder, JM; Ma, YT; Plamondon, L; Stein, RL (1998). Potent and selective inhibitors of the proteasome: dipeptidyl boronic acids. *Bioorg. Med. Chem. Lett.* **8**:333-338.
Adams, J; Palombella, VJ; Sausville, EA; Johnson, J; Destree, A; Lazarus, DD; Maas, J.; Pien, CS; Prakash, S; Elliott, PJ (1999). Proteasome inhibitors: a novel class of potent and effective antitumor agents. *Cancer Res.* **59**:261 -2622.
Adams, J; Palombella, VJ; Elliott, PJ (2000). Proteasome inhibition: a new strategy in cancer treatment. *Investigational New drugs* **18**:109-121.
Adams, J; Stein, R (1996). Novel inhibitors of the proteasome and their therapeutic use in inflammation. *Annu. Rep. Med. Chem.* **31**:279-288.
André, P; Gröttrup, M; Klenerman, P; de Giuli, R; Booth, BL Jr; Cerundolo, V; Bonneville, M; Jotereau, F; Zinkernagel, RM; Lotteau, V (1998). An inhibitor of HIV-1 protease modulates proteasome activity, antigen presentation, and T cell responses. *Proc. Natl. Acad. Sci.* USA **95**:13120-13124.
Baumeister, W; Walz, J; Zuhl, F; Seemuller, E (1998). The proteasome: paradigm of a selfcompartmentalizing protease. *Cell* **92**:367-380.
Bogyo, M; McMaster, JS; Gaczynska, M; Tortorella, D; Goldberg, AL; Ploegh, H (1997). Covalent modification of the active site threonine of proteasomal beta subunits and the Escherichia coli homolog Hs1V by a new class of inhibitors. *Proc. Natl. Acad. Sci.* USA **94**:6629-6634.
Bour, S; Schubert, U; Peden, K; Strebel, K (1996). The envelope glycoprotein of human immunodeficiency virus type 2 enhances particle release: a Vpu-like factor? *J. Virol.* **70**:820-829.
Breiner, KM; Urban, S; Glass, B; Schaller, H (2001). Envelope protein-mediated downregulation of hepatitis B virus receptor in infected hepatocytes. *J. Virol.* **75**:143-50.
Bruns, M; Miska, S; Chassot, S; Will, H (1998). Enhancement of hepatitis B virus infection by noninfectious subviral particles. *J. Virol.* **72**:1462-1468.
Bryant, JL; Gallo RC; Weichold FF (2000). Use of protease inhibitors to modulate cellular pathways, immunity and therapies associated therewith. Patent application WO 0033654.
Chen, HS; Kew, MC; Hornbuckle, WE; Tennant, BC; Cote, PJ; Gerin, JL; Purcell, RH; Miller, RL (1992). The precore gene of the woodchuck hepatitis virus genome is not essential for viral replication in the natural host. *J. Virol.* **66**:5682-5684.
Coux, O; Tanaka, K; Goldberg, AL (1996). Structure and functions of the 20S and 26S proteasomes. *Annu. Rev. Biochem.* **65**:801-847.
Dandri, M; Burda, MR; Torok, E; Pollok, JM; Iwanska, A; Sommer, G; Rogiers, X; Rogler, CE; Gupta, S; Will, H; Greten, H; Petersen, J (2001). Repopulation of mouse liver with human hepatocytes and in vivo infection with hepatitis B virus. Hepatology 33:981-988.
Doolittle, RF; Feng, DF; McClure, MA; Johnson, MS (1990). Retrovirus phylogeny and evolution. *Curr. Top. Microbiol. Immunol.* **157**:1-18.
Elliott, PJ; Pien, CS; McCormack, TA; Capman, ID; Adams, J (1999). Proteasome inhibition: a novel mechanism to combat asthma. *J. Allergy Clin. Immunol.* **104**:294-300.
Fenteany, G; Standaert, RF; Lane, WS; Choi, S; Corey, EJ; Schreiber, SL (1995). Inhibition of proteasome activities and subunit-specific amino-terminal threonine modification by lactacystein. *Science* **268**:726-731.
Fernholz, D; Wildner, G; Will, H (1993). Minor envelope proteins of duck hepatitis B virus are initiated at internal pre-S AUG codons but are not essential for infectivity. *Virology* **197**:64-73.
Frankel, A; Man, S; Elliott, P; Adams, J; Kerbel, RS (2000). Lack of multicellular drug resistance observed in human ovarian and prostate carcinoma treated with the proteasome inhibitor PS-341. *Clin. Cancer Res.* **6**:3719-3728.
Gaczynska M, Rock KL, Goldberg AL (1993). Gamma-interferon and expression of MHC genes regulate peptide hydrolysis by proteasomes. *Nature* **365**:264-267.
Gardner, RC; Assinder, SJ; Christie, G; Mason, GG; Markwell, R; Wadsworth, H; McLaughlin, M; King, R; Chabot-Fletcher, MC; Breton, JJ; Allsop, D; Rivett, AJ (2000). Characterization of peptidyl boronic acid inhibitors of mammalian 20 S and 26 S proteasomes and their inhibition of proteasomes in cultured cells. *Biochem. J.* **346**:447-454.
Geier, E; Pfeifer, G; Wilm, M; Lucchiari-Hartz, M; Baumeister, W; Eichmann, K; Niedermann, G (1999). A giant protease with potential to substitute for some functions of the proteasome. *Science* **283**:978-981.
Glas, R; Bogyo, M; McMaster, JS; Gaczynska, M; Ploegh, HL (1998). A proteolytic system that compensates for loss of proteasome function. *Nature* **392**:618-622.
Hanada, M; Sugawara, K; Kaneta, K; Toda, S; Nishiyama, Y; Tomita, K; Yamamoto, H; Konishi, M; Oki, T (1992). Epoxomicin, a new antitumor agent of microbial origin. *J. Antibiot.* (Tokyo) **45**:1746-1752.
Hershko, A; Ciechanover, A (1998). The ubiquitin system. *Annu. Rev Biochem.* **67**:425-479.
Higashitsuji, H; Itoh, K; Nagao, T; Dawson, S; Nonoguchi, K; Kido, T; Mayer, RJ; Arii, S; Fujita, J (2000). Reduced stability of retinoblastoma protein by gankyrin, an oncogenic ankyrinrepeat protein overexpressed in hepatomas. *Nat. Med.* **6**:96-99.
Hu, Z; Zhang, Z; Doo, E; Coux, O; Goldberg, AL; Liang, TJ (1999). Hepatitis B virus X protein is both a substrate and a potential inhibitor of the proteasome complex. *J. Virol.* **73**:7231-40.
Lightcap, ES; McCormack, TA; Pien, CS; Chau, V; Adams, J; Elliott, PJ (2000). Proteasome inhibition measurements: clinical application. *Clin. Chem.* **46**:673-683.
Meng, L; Kwok, BH; Sin, N; Crews, CM (1999a). Eponemycin exerts its antitumor effect through the inhibition of proteasome function. *Cancer Res.* **59**:2798-2801.
Meng, L; Mohan, R; Kwok, BH; Elofsson, M; Sin, N; Crews, CM (1999b). Epoxomicin, a potent and selective proteasome inhibitor, exhibits in vivo antiinflammatory activity. *Proc. Natl. Acad. Sci.* USA. **96**(18):10403-10408.
Moradpour, D; Grabscheid, B; Kammer, AR; Schmidtke, G; Gröttrup, M; Blum, HE; Cemy, A (2001). Expression of hepatitis C virus proteins does not interfere with major histocompatibility complex class I processing and presentation in vitro. *Hepatology* **33**: 1282-1287.
Murray, RZ; Norbury, C (2000). Proteasome Inhibitors as anti-cancer agents. *Anticancer Drugs* **11**:407-417.
Ott, DE; Coren, LV; Copeland, TD; Kane, BP; Johnson, DG; Sowder, RC 2nd; Yoshinaka, Y; Oroszlan, S; Arthur, LO; Henderson, LE (1998). Ubiquitin is covalently attached to the p6Gag proteins of human immunodeficiency virus type-1 and simian immunodeficiency virus and the p12Gag protein of moloney murine leukemia virus. *J. Virol.* **72**:2962-2968.
Palombella, VJ; Conner, EM; Fuseler, JW; Destree, A; Davis, JM; Laroux, FS; Wolf, RE; Huang, J; Brand, S; Elliott, PJ; Lazarus, D; McCormack, T; Parent, L; Stein, R; Adams, J; Grisham, MB (1998). Role of the proteasome and NF-kappaB in streptococcal cell wall-induced polyarthritis. *Proc. Natl. Acad Sci. USA* **95**:15671-15676.
Phillips, JB; Williams, AJ; Adams, J; Elliott, PJ; Tortella, FC (2000). Proteasome inhibitor PS-519 reduces infarction and attenuates leukocyte infiltration in a rat model of focal cerebral ischemia. *Stroke* **31**:1686-1693.
Pugh, JC; Simmons, H (1994). Duck hepatitis B virus infection of Muscovy duck hepatocytes and nature of virus resistance in vivo. *J. Virol.* **68**:2487-94.
Rehermann, B; Ferrari, C; Pasquinelli, C; Chisari, FV (1996). The hepatitis B virus persists for decades after patients' recovery from acute viral hepatitis despite active maintenance of a cytotoxic T-lymphocyte response. *Nat. Med.* **2**:1104-1108.
Rivett, AJ; Gardner, RC (2000). Proteasome inhibitors: from in vitro uses to clinical trials. J. *Pept. Sci.* **6**:478-488.
Rock, KL; Goldberg, AL (1999). Degradation of cell proteins and the generation of MHC class I-presented peptides. *Annu. Rev. Immunol.* **17**:739-779.
Schäfer, S; Tolle, T; Lottmann, S; Gerlich, WH (1998). Animal models and experimental systems in hepatitis B virus research. *In* R. C. Koshy, W. H. (ed.), Hepatitis B virus: Molecular mechanisms in disease and novel strategies for therapy. Imperial College Press, London p. 51-74.
Schmidtke, G; Holzhutter, HG; Bogyo, M; Kairies, N; Groll, M; de Giuli, R; Emch, S; Gröttrup, M (1999). How an inhibitor of the HTV-1 protease modulates proteasome activity. *J. Biol. Chem.* **274**:35734-35740.
Schubert, U; Antón, LC; Bacik, I; Cox, JH; Bour, S; Bennink, JR; Orlowski, M; Strebel, K; Yewdell, JW (1998). CD4 glycoprotein degradation induced by Human Immunodeficiency Virus type 1 Vpu protein requires the function of proteasomes and the ubiquitin conjugating pathway. *J. Virol.* **72**:2280-2288.
Schubert, U; Clouse, KA; Strebel, K (1995). Augmentation of virus secretion by the human immunodeficiency virus type 1 Vpu protein is cell type independent and occurs in cultured human primary macrophages and lymphocytes. *J. Virol.* **69**:7699-7711.
Schubert, U; Antón, LC; Gibbs, J; Norbury, CC; Yewdell, JW; Bennink, JR (2000) Rapid degradation of a large fraction of newly synthesized proteins by proteasomes. *Nature* **404**:770-774.
Schwartz, O; Marechal, V; Friguet, B; Arenzana-Seisdedos, F; Heard, J-M (1998). Antiviral activity of the proteasome on incoming human immunodeficiency virus type 1. *J*. *Virol.* **72**:3845-3850.
Schwartz, AL; Ciechanover, A (1999). The ubiquitin-proteasome pathway and pathogenesis of human diseases. *Annu. Rev. Med.* **50**:57-74.
Sells, MA; Chen, ML; Acs, G (1987). Production of hepatitis B virus particles in Hep G2 cells transfected with cloned hepatitis B virus DNA. *Proc. Natl. Acad. Sci. USA* **84**:1005-1009.
Sirma, H; Weil, R; Rosmorduc, O; Urban, S; Israel, A; Kremsdorf, D; Brechot, C (1998). Cytosol is the prime compartment of hepatitis B virus X protein where it colocalizes with the proteasome. *Oncogene* **16**: 2051-2063.
Suzuki, R; Tamura, K; Li, J; Ishii, K; Matsuura, Y; Miyamura, T; Suzuki, T (2001). Ubiquitinmediated degradation of hepatitis C virus core protein is regulated by processing at its carboxyl terminus. *Virology* **280**:301-309.
Swanstrom, R; Wills, J (1997). Synthesis, assembly, and processing of viral proteins. In Retroviruses, J. Coffin, S. Hughes and H. Varmus eds. (Plainview, NY: Cold Spring Harbor Press), pp. 263-334.
Teicher, BA; Ara, G; Herbst, R; Palombella, VJ; Adams, J (1999). The proteasome inhibitor PS-341 in cancer therapy. *Clin. Cancer Res.* **5**:2638-2645.
Theodore, TS; Englund, G; Buckler-White, A; Buckler, CE; Martin, MA; Peden, KWC (1996). Construction and characterization of a stable full-length macrophage-tropic HIV type 1 molecular clone that directs the production of high titers of progeny virions. *AIDS Res. Hum. Retrovir.* **12**:191-194.
Trautwein, C; Manns, M (2001). Antivirale Therapie der chronischen Virushepatitis. *Internist* **42**:913-923.
Voges, D; Zwickl, P; Baumeister, W (1999). The 26S proteasome: a molecular machine designed for controlled proteolysis. *Annu. Rev. Biochem.* **68**:1015-1068.
Yagi, T; Hardin, JA; Valenzuela, YM; Miyoshi, H; Gores, GJ; Nyberg, SL (2001). Caspase inhibition reduces apoptotic death of cryopreserved porcine hepatocytes. *Hepatology* **33**:1432-40.

### Abkürzungsverzeichnis

- A3.01: humane CD4⁺ T-Zelllinie
- AIDS: Aquired Immunodeficiency Syndrome
- Annexin-V: Zellrezeptor Annexin-Fünf
- ATP: Adenosin-5'-triphosphat
- BIV: Bovines Immundefizienzvirus
- BLV: Bovines Leukämievirus
- CA: capsid (HIV-1 p24^{CA} Antigen)
- cccDNA: komplett doppelsträngig supergecoiltes DNA-Genom
- CFTR: Cystis Fibrosis Transmembran Regulator
- d: Tag
- DHB(V): Enten-Hepatitis-B(-Virus)
- DHBV: Enten-Hepatitis-B-Virus
- DMEM: Dulbeccos' modifizierten Eagle's Medium
- DMSO: Dimethylsulfoxid
- DNA: desoxyribonucleic acid (Desoxyribonukleinsäure)
- ECL: enhanced chemiluminescence
- EDTA: Ethylendiamintetraessigsäure
- EIAV: Equine Infectious Anemia Virus
- ELISA: Enzyme Linked Immunosorbent Assay
- Env: Envelope
- ER: Endoplasmatisches Retikulum
- FDA: Fluoreszeindiazetat
- FIV: Feline Leukemia Virus
- Gag: Gruppenspezifisches Antigen, Core Portein von Retroviren
- h: Stunde(n)
- hr: Stunde(n)
- HAART: HAART-Therapie (highly active antiretroviral therapy)
- HAV: Hepatitis-A-Virus
- HBe: Hepatitis-B-Virus E-Antigen
- HBs: HBV-Oberflächenprotein
- HBV: Hepatitis-B-Virus
- HCC: Hepatozelluläres Karzinom
- HCV: Hepatitis-C-Virus
- HDV: Hepatitis-Delta-Virus
- HEPES: (N-[2-hydroxyeihyl]piperazine-N'-[2-ethanesulfonic acid])
- HEV: Hepatitis-E-Virus
- HFV: Hepatitis-F-Virus
- HGV: Hepatitis-G-Virus
- HIV: Humanes Immundefizienzvirus
- HIVAN: HIV associated nephropathy Syndrom
- HIV-1_{NL4-3}: Humanes Immundefizienzvirus, infektioeser Klon NL4-3
- HLS: HIV-associated lipodystrophy syndrome
- HPV: Humanes Papilloma-Virus
- HTLV: Humanes T-Zell Leukämie Virus
- i.d.R: in der Regel
- IFN: Interferon
- IL: Interleukin
- kb: Kilobasen
- IκB: inhibitorischen Faktor IκB
- kDa: Kilodalton (Maß für Molekulargewicht)
- Ki: inhibitorische Konstante
- LC: Lactacystin
- L-Domäne: late assembly domain, späte Assemblierungsdomäne in retroviralen Gag-Proteinen
- LLnL: Leuzinyl- Leuzinly-nor-Leuzinal
- MA: Matrix, (p17^{MA} Protein von HIV-1)
- MDa: Megadalton
- MG132: N-carbobenzoxyl-L-leucinyl-L-leucinyl-L-leucinal
- MG232: Borsäure-Derivat von MG132
- MHC: Major Histocompatibilitäts Komplex
- Mo-MuLV: Murines Leukämie-Virus
- MOI: multiplicity of infection
- MT-4-Zellen: humane CD4⁺ T-Zellinie, HTLVI transformiert
- nanoM: nano Molar (nM)
- NC: Nukleokapsid,
- NF-κB: Transkriptionsfaktor
- NLVS: 4-Hydroxy-5-iodo-3-nitrophenylactetyl-L-Leucinyl-L-Leucinyl-L-Leucin-vinylsulfon, auch bezeichnet als NLVS
- nM: nano Mol
- ocDNA: offen zirkuläre Form der DANN
- p27^{CA}: capsid (HIV-2 p27^{CA} Antigen)
- PBS: Phosphatpuffer, phosphate buffered saline
- PCR: polymerase chain reaction - Polymerase-Kettenreaktion
- PEH: Entenhepatozyten / Pekingentenhepatozyten
- PGPH: Postglutamyl-Peptid hydrolysierende
- PI: Proteasom-Inhibitor
- Pol: Polymerase (Retroviren)
- P-Protein: Hepatitis-B-Virus Polymerase Protein
- PR: Protease
- Pr55: Prekursor Gag Portein von HIV-1
- Pr58: Prekursor Gag Portein von HIV-2
- PreS: großes Hepatitis-B-Virus Hüllprotein, PräS
- PS: ProScript
- PS-273: Morpholin-CONH-(CH-Naphtyl)-CONH-(CH-isobutyl)-B(OH)₂
- PS-293: Enantiomer von PS-273
- PS-296: 8-Quinolyl-sulfonyl-CONH-(CH-Naphthyl)-CONH(-CH-isobutyl)-B(OH)₂
- PS-303: NH₂(CH-Naphtyl)-CONH-(CH-isobutyl)-B(OH)₂)
- PS-325: 2-Quinol-CONH-(CH-*homo*-Phenylalanin)-CONH-(CH-isobutyl)-B(OH)₂);
- PS-334: CH₃-NH-(CH-Naphtyl-CONH-(CH-Isobutyl)-B(OH)₂);
- PS-341: N-Pyrazinecarbonyl-L-Phenylalanin-L-leuzin-Borsäure, Molekülformel: C₁₉H₂₅BN₄O₄
- PS-383: Pyridyl-CONH-(CH*p*F-Phenylalanin)-CONH-(CH-isobutyl)-B(OH)₂
- PS-519: 1*R*-[1*S*, 4*R*, 5*S*]]-1-(1-Hydroxy-2-methylpropyl)-4-propyl-6-oxa-2-azabicyclo[3.2.0]heptane-3,7-dion, Molekülformel: C₁₂H₁₉NO₄
- PSI: N-Carbobenzoxy-Ile-Glu(OBut)-Ala-Leu-H
- RNA: Ribonucleinsäure (ribonucleic acid)
- RSV: Rouse Sarcoma Virus
- RT: Reverse Transkriptase
- SDS: Natriumdodecylsulfat (Sodium Dodecylsulfate)
- SDS-PAGE: SDS-Polyacrylamid Gelelektrophorese
- SIV: Simian immunodeficiency virus
- TBS: tris buffered saline
- TNF: Tumornekrosefaktor
- Thr: Threonin
- Tris: Tris-Puffer - Tris-(hydroxymethyl)-aminomethan
- Ub: Ubiquitin
- UPS: Ub-Proteasom-System
- Vpu: Virus Protein U
- WB: Western blot
- XXX: Kategory XXX = Literatur-Stellen nach dem Prioritätsdatum
- zLLL: N-carbobenzoxyl-L-leucinyl-L-leucinyl-L-leucinal

## Patentansprüche

1. Verwendung von Proteasom-Inhibitoren zur Herstellung eines Mittels zur Inhibierung der Freisetzung, Reifung und Replikation eines Hepatitis-Virus.

2. Verwendung nach Anspruch 1, **dadurch gekennzeichnet, dass** das Mittel als wirksame Komponente mindestens einen Proteasom-Inhibitor enthält, der aus natürlichen Quellen oder aus Mikroorganismen isoliert oder synthetisch hergestellt wird oder aus Verbindungen mit Epoxyketon- oder Lacton-Strukturen oder aus Peptidaldehyd-, Glyoxal-, Borsäure oder Pinacolester-Derivaten besteht.

3. Verwendung gemäß den Ansprüchen 1 und 2, **dadurch gekennzeichnet, dass** das Mittel gegen Viren eingesetzt wird, die im Rahmen des Replikationszyklus von der Zelloberfläche freigesetzt werden.

4. Verwendung gemäß den Ansprüchen 1 und 2, **dadurch gekennzeichnet, dass** das Mittel einen oder mehrere Proteasom-Inhibitoren als wirksame Komponente in einer pharmazeutischen Zubereitung enthält, welche die Aktivitäten des Ubiquitin/Proteasom-Pathway hemmen, regulieren oder anderweitig beeinflussen.

5. Verwendung gemäß Anspruch 4, **dadurch gekennzeichnet, dass** ein oder mehrere Proteasom-Inhibitoren als Substanzen eingesetzt werden, die speziell die enzymatischen Aktivitäten des kompletten 26S Proteasom-Komplexes und der freien, nicht mit regulatorischen Untereinheiten assemblierten 20S katalytisch aktiven Proteasom-Struktur beeinflussen.

6. Verwendung gemäß den Ansprüchen 4 und 5, **dadurch gekennzeichnet, dass** Substanzen als Mittel eingesetzt werden, die als Proteasom-Inhibitoren von höheren Eukaryoten aufgenommen werden und nach Zellaufnahme mit den katalytischen Untereinheiten des Proteasoms in Wechselwirkung treten und dabei alle oder einzelne der proteolytischen Aktivitäten des Proteasoms - die Trypsin-, die Chymotrypsin- und die Postglutamyl-Peptid hydrolysierenden Aktivitäten - innerhalb des 26S oder auch des 20S Proteasom-Komplexes irreversibel oder reversibel blockieren.

7. Verwendung nach einem der Ansprüche 4 bis 6, **dadurch gekennzeichnet, dass** die pharmazeutischen Zubereitungen neben Proteasom-Inhibitoren auch andere Mittel enthalten, welche das zelluläre Ubiquitin-System beeinflussen, regulieren oder hemmen.

8. Verwendung gemäß Anspruch 7, **dadurch gekennzeichnet, dass** die Aktivitäten
a) der Ubiquitin-konjugierenden Enzyme und/oder
b) der Ubiquitin-hydrolysierenden Enzyme; und/oder
c) von zellulären Faktoren, die mit Ubiquitin in Wechselwirkung treten, beeinflusst, reguliert oder gehemmt werden.

9. Verwendung gemäß Anspruch 8, **dadurch gekennzeichnet, dass** die Aktivitäten von zellularen Faktoren, die mit Ubiquitin als Mono-Ubiquitin oder als poly-Ubiquitin in Wechselwirkung treten, beeinflusst, reguliert oder gehemmt werden.

10. Verwendung gemäß einem der Ansprüche 1 bis 9, **dadurch gekennzeichnet, dass** als Proteasom-Inhibitoren Substanzen eingesetzt werden, die in verschiedenen Formen *in vivo* oral, intravenös, intramuskulär, subkutan oder in verkapselter Form mit oder ohne Zellspezifität-tragende Veränderungen verabreicht werden, aufgrund der Anwendung eines bestimmtes Applikations- und/oder Dosis-Regimes eine geringe Zytotoxizität aufweisen, keine oder unbedeutende Nebenwirkungen auslösen, eine relative hohe metabolische Halbwertszeit und eine relative geringe Clearence-Rate im Organismus aufweisen.

11. Verwendung gemäß einem der Ansprüche 1 bis 10, **dadurch gekennzeichnet, dass** als Proteasom-Inhibitoren Substanzen eingesetzt werden, die
a) in natürlicher Form aus Mikroorganismen oder anderen natürlichen Quellen isoliert werden; oder
b) durch chemische Modifikationen aus natürlichen Substanzen hervorgehen; oder
c) total-synthetisch hergestellt werden; oder
d) durch gentherapeutische Verfahren in vivo synthetisiert werden; oder
e) durch gentechnische Verfahren in vitro oder
f) in Mikroorganismen hergestellt werden.

12. Verwendung gemäß Anspruch 11, **dadurch gekennzeichnet, dass** als Proteasom-Inhibitoren Substanzen eingesetzt werden, die folgenden Substanzklassen angehören:
a) natürlich vorkommende Proteasom-Inhibitoren:
- Peptid-Derivate, welche C-terminal Epoxyketon-Strukturen enthalten; oder
- β-Lacton-Derivate; oder
- Aclacinomycin A (auch bezeichnet als Aclarubicin); oder
- Lactacystin und dessen chemische modifizierte Varianten, wie der Zellmembranpenetrierenden Variante "Clasto lactacystein β-Lacton"
b) synthetisch hergestellte Proteasom-Inhibitoren:
modifizierte Peptidaldehyde wie N-carbobenzoxy-L-leucinyl-L-leucinyl-L-leucinal (auch bezeichnet als MG132 oder zLLL), dessen Borsäure-Derivat MG232; N-Carbobenzoxy-Leu-Leu-Nva-H (bezeichnet als MG115; N-AcetylL-Leuzinyl-L-Leuzinyl-L-Norleuzinal (bezeichnet als LLnL), N-Carbobenzoxy-Ile-Glu(OBut)-Ala-Leu-H (auch bezeichnet als PSI);
c) Peptide, welche C-terminal eine α,β-Epoxyketon-Struktur tragen, ferner Vinylsulfone wie Carbobenzoxy-L-Leucinyl-L-Leucinyl-L-Leucin-vinyl-sulfon oder 4-Hydroxy-5-iodo-3-nitrophenylactetyl-L-Leucinyl-L-Leucinyl-L-Leucin-vinyl-sulfon (NLVS);
d) Glyoxal- oder Borsäure-Reste wie Pyrazyl-CONH(CHPhe)CONH(CHisobutyl)B(OH)₂) sowie Dipeptidyl-Borsäure-Derivate oder
e) Pinacol-Ester - wie Benzyloxycarbonyl(Cbz)-Leu-Leu-boroLeu-Pinacol-Ester.

13. Verwendung gemäß den Ansprüchen 11 und 12, **dadurch gekennzeichnet, dass** als besonders geeignete Proteasom-Inhibitoren die Epoxyketone Epoxomicin (Epoxomycin, Molekülformel: C₂₈H₈₆N₄O₇) und/oder Eponemycin (Eponemicin, Molekülformel: C₂₀H₃₆N₂O₅) werden.

14. Verwendung gemäß den Ansprüchen 11 und 12, **dadurch gekennzeichnet, dass** als besonders geeignete Proteasom-Inhibitoren aus der PS-Serie die Verbindungen
a) PS-519 als β-Lacton- sowie als Lactacystin-Derivat die Verbindung 1R-[1S, 4R, 5S]]-1-(1-Hydroxy-2-methylpropyl)-4-propyl-6-oxa-2-azabicyclo[3.2.0]heptane-3,7-dione-Molekülformel C₁₂H₁₉NO₄- und/oder
b) PS-314 als Peptidyl-Borsäure-Derivat die Verbindung N-Pyrazinecarbonyl-L-Phenylalanin-L-Leuzin-Borsäure - Molekülformel C₁₉H₂₅BN₄O₄ - und/oder
c) PS-273 (Morpholin-CONH-(CH-Naphthyl)-CONH-(CH-isobutyl)-B(OH)₂) und dessen Enantiomer PS-293 und/oder
d) die Verbindung PS-296 (8-Quinolyl-sulfonyl-CONH-(CH-Napthyl)-CONH(-CH-isobutyl)-B(OH)₂) und/oder
e) PS-303 (NH₂(CH-Naphthyl)-CONH-(CH-isobutyl)-B(OH)₂) und/oder
f) PS-321 als (Morpholin-CONH-(CH-Napthyl)-CONH-(CH-Phenylalanin)-B(OH)₂); - und/oder
g) PS-334 (CH₃-NH-(CH-Naphthyl-CONH-(CH-Isobutyl)-B(OH)₂) und/oder
h) die Verbindung PS-325 (2-Quinol-CONH-(CH-*homo*-Phenylalanin)-CONH-(CH-isobutyl)-B(OH)₂) und/oder
i) PS-352 (Phenyalanin-CH₂-CH₂-CONH-(CH-Phenylalanin)-CONH-(CH-isobutyl)1-B(OH)₂) und/oder
j) PS-383 (pyridyl-CONH-(CH*p*F-Phenylalanin)-CONH-(CH-isobutyl)-B(OH)₂)
eingesetzt werden.

15. Verwendung nach einem der Ansprüche 1 bis 14, **dadurch gekennzeichnet, dass** Proteasom-Inhibitoren
a) die Produktion von infektiösen Virionen von Hepatitisvirus-infizierten Zellen weitgehend oder vollkommen unterbinden und/oder
b) die Hemmung der Freisetzung von Virionen wie auch eine nahezu vollständige Reduktion der Infektiosität der freigesetzten Virionen bewirken und/oder
c) die Virusvermehrung und somit die Neuinfektion von Hepatozyten und damit die Ausbreitung einer Hepatitis-Infektion in vivo in dem Lebergewebe eines Infizierten unterdrücken.

16. Verwendung nach einem der Ansprüche 1 bis 14, **dadurch gekennzeichnet, dass** die Vermehrung von Hepadnaviren durch Proteasom-Inhibitoren nach den Mechanismen
a) Blockierung/Reduktion der Freisetzung von neuen Virionen
b) Blockierung/Reduktion der Infektiosität von freigesetzten Virionen
c) Blockierung/Reduktion der Infektionsausbreitung in Kulturen von primären Hepatozyten
d) Blockierung/Reduktion der Infektionsausbreitung in vivo in dem Lebergewebe eines Infizierten.
gehemmt wird.

17. Verwendung gemäß einem der Ansprüche 1 bis 14, wobei
a) das Absterbens von Hepato-Karzimonzellen induziert wird; und / oder
b) das Entstehens von Leberzellkarzinomen unterdrückt und / oder verhindert wird; und / oder
c) Patienten mit etablierten Leberzellkarzinomen therapiert werden.

18. Verwendung nach Anspruch 17, wobei
a) HBV-induzierte Leberzirrhose und/oder
b) HBV-induzierte Leberzellkarzinome und/oder
c) HCV-induzierte Leberkarzinome und/oder
d) Medikamenten-induzierte Leberkarzinome und/oder
e) genetisch bedingte Leberkarzinome und/oder
f) Umwelt-bedingte Leberkarzinome
behandelt / bekämpft / verhindert werden.

19. Verwendung nach den Ansprüchen 17 und 18, wobei Leberkarzinomzellen gezielt eliminiert werden, welche infolge einer
a) HBV-Infektion und/oder
b) HCV-Infektion oder
c) entsprechenden Koinfektion mit beiden Viren oder
d) HDV/HBV-Koinfektion
entstehen.

20. Verwendung nach den Ansprüchen 17 bis 19, wobei Entstehung, das Wachstums und / oder die Metastasierung von Leberzelltumoren verhindert wird sowie Leberkarzinomzellen in HBV- und HCV-infizierten Patienten und Tieren bevorzugt zerstört werden.

21. Verwendung nach einem der Ansprüche 11 bis 14, wobei
a) Expression, Modifizierung und Aktivität des Tumorsuppressor-Proteins p53 moduliert wird; und / oder
b) der Anzahl infizierter und Hepatitisviren produzierender Zellen im Leberzellgewebe reduziert wird; und / oder
c) sowohl die Erhaltung und Persistenz einer bereits etablierten Infektion mit Hepatitisviren wie auch einer Sekundärinfektion und somit der Ausbreitung einer Infektion, einschließlich der Blockierung der Ausbreitung einer Infektion mit Hepatitisviren *in vivo* gehemmt wird

22. Verwendung gemäß einem der Ansprüche 1 bis 14, **dadurch gekennzeichnet, dass** Proteasom-Inhibitoren die Phosphorylierung von Hepatitisvirus spezifischen Nukleokapsid-Proteinen verändern und **dadurch** die Freisetzung und Infektiosität von Hepatitis-B- und C-Viren herabsetzen oder blockieren.

23. Verwendung von Proteasom-Inhibitoren nach einem der Ansprüche 1 bis 14 in Kombination untereinander zur Herstellung eines Mittels
a) zur Behandlung und Bekämpfung von Hepatitiden
b) mit bereits in der anti-viralen Therapie von Hepadnaviren verwendeten Therapeutika.

24. Verwendung nach einem der Ansprüche 1 bis 14, wobei Infektionen mit Hepatitisviren zur Verhinderung
a) einer Re-Infektion mit HBV bei Leber- und anderen Organtransplantationen; oder
b) einer Re-Infektion mit HBV bei Zelltherapien durch Gabe der Mittel vor, während und nach der Transplantation; oder
c) einer Re-Infektion mit HBV bei der Transplantation von virusfreien Organen auf chronische Virusträger, die immer Restvirus haben und sich neue Organe infizieren können wie auch bei der Übertragung von Virus-haltigen Organen von Spendern auf virusfreie Patienten; oder
d) der Etablierung einer systemischen Hepatitisvirus-Infektion unmittelbar nach Kontakt mit infektiösem Virus
behandelt werden.

25. Verwendung nach einem der Ansprüche 1 bis 14, wobei Infektionen mit Hepatitisviren
a) zur Vorbeugung einer Hepatitisvirus-Infekrion bei Personen mit hohem Risiko einer Neuinfektion, wie bei Ärzten, Risiko-Personal in Häusern mit hohem Besucherverkehr, Drogenabhängigen, Reisenden in hochendemische Gebiete für Hepatitisviren, in der Krankenbehandlung oder für Familienangehörige von chronischen Virusträgern; oder
b) zur Minderung oder Eliminierung einer Leberentzündung durch Immunsystemvermittelte Mechanismen.
behandelt werden.

## Claims

1. Use of proteasome inhibitors for the manufacture of an agent for the inhibition of release, maturation, and replication of a hepatitis virus.

2. Use according to claim 1, **characterised in that** the agent contains as an effective component at least one proteasome inhibitor, which is isolated from natural sources or is produced synthetically, or contains epoxyketone- or lactone structures, or contains derivatives from peptidealdhydes, glyoxal, boric acid, or pinacola ester.

3. Use according to claim 1 and 2, **characterized in that** the agent is used against viruses which are released from the cell surface during the replication cycle.

4. Use according to claim 1 and 2, **characterized in that** the agent contains one or more proteasome inhibitors as effective component in a pharmaceutical preparation, which inhibit, regulate or affect otherwise the activities of the ubiquitin/proteasome-pathway.

5. Use according to claim 4, **characterized in that** one or more proteasome inhibitors are used as agents which particular affect the enzymatic activities of the complete 26S proteasome complex and the free catalytic active 20S proteasome structure which is not assembled with regulatory subunits.

6. Use according to claim 4 and 5, **characterized in that** substances are used as agent which are taken up in form of proteasome inhibitors by higher eukaryotic organisms and that after cell uptake those reagents interact with the catalytic subunits of the proteasome and thus irreversibly or reversibly block all or several of the proteolytic activities of the proteasome - the trypsine-, chymotrypsin-and the postglutamyl peptide hydrolyzing activities - residing inside the 26S or the 20S proteasome complex.

7. Use according to one of the claims 4 to 6, **characterized in that** the pharmaceutical preparations contain in addition to proteasome inhibitors other agents which affect, regulate or inhibit the cellular ubiquitin-system.

8. Use according to claim 7, **characterized in that** the activities
a) the ubiquitin-conjugating enzyme and/or
b) the ubiquitin-hydrolyzing enzyme and/or
c) of cellular factors which interact with ubiquitin
are affected, regulated or inhibited.

9. Use according to claim 8, **characterized in that** the activities of cellular factors which are interact with ubiquitin as mono-ubiquitin or as poly-ubiquitin are affected, regulated or inhibited.

10. Use according to claim 1 to 9, **characterized in that** as proteasome inhibitors agents are used which are administered in vivo in different forms like oral, intravenous, intramuscular, subcutaneous or in encapsulated form with or without additions that determine cell specificity, at a dose rate regime that exhibit low cytotxicity, trigger no or only marginal side effects, have a relative high metabolic half-life, and a relative low clearance-rate in the organism.

11. Use according to one of the claims 1 to 10, **characterized in that** as proteasome inhibitors substances are used which
a) are isolated in natural form from microorganisms or other natural sources or
b) arise of natural agents by chemical modification or
c) are synthezised totally
d) are synthezised by gentherapeutic methods in vivo
e) are made by genetically methods in vitro
f) are made in microorganisms.

12. Use according to claim 11, **characterized in that** as proteasome inhibitors the following substances are used:
a) natural occurring proteasome inhibitors such as:
- peptide derivates which contain C-terminal epoxyketon structures or
- β-lactone-derivates or
- aclacinomycin A (also called aclarubicine) or
- lactacystine and ist chemical modified versions, such as the cell membrane penetrating version "clastolactacystein b- lactone"
b) synthetic produced proteasome inhibitors:
N-carbobenzoxy-L-leucinyl-L-leucinyl-L-leucinal (also called MG 132 or zLLL) or its boric acid -derivate MG232 or - N-Acetyl-L-Leuzinyl-L-Leuzinyl-L-Norleuzinal (called LLnL) or N-Carbobenzoxy-IleGlu(OBut)-Ala-Leu-H (also called PSI);
c) peptids which contain a C-terminal α,β-epoxyketon-structure, further vinyl-sulfones such as Carbobenzoxy-L-Leucinyl-L-Leucinyl-L-Leucin-vinyl-sulfone or- 4-Hydroxy-5-iodo- 3-nitrophenylactetyl-L- Leucinyl-L-Leucinyl-L-Leucin vinyl-sulfone (NLVS);
d) glyoxal- or boric acid rests such as Pyrazyl-CONH(CHPhe)CONH(CHisobutyl)B(OH)₂) and Dipeptidyl-boric acid-derivates or
e) Pinacol-esters ― such as Benzyloxycarbonyl(Cbz)-Leu-Leu-boroLeu -Pinacol-ester.

13. Use according to claim 11 to 12, **characterized in that** as particularly suitable proteasome inhibitors the epoxyketones epoxomicin (epoxomycin, molecular formula: C₂₈H₈₆N₄O₇) and/or eponemycin (eponemicin, molecular formula: C₂₀H₃₆N₂O₅) are used.

14. Use according to claim 11 to 12, **characterized in that** as particularly suitable proteasome inhibitors the compounds of the PS- serial
a) PS-519 as β-Lacton- and as lactacystine-derivate the compound 1R-[1S, 4R, 5S]-1-(1-hydroxy-2-methylpropyl)-4-propyl-6-oxa-2-azabicyclo[3.2.0]heptane-3,7-dione - molecular formula C₁₂H₁₉NO₄- and/or
b) PS-314 as peptidyl-boric-acid-derivate the compound N-pyrazinecarbonyl-L-phenylalanine-L-Leuzin- boric-acid - molecular formula C₁₉H₂₅BN₄O₄ - and/or
c) PS-273 (morpholine-CONH-(CH-naphthyl)-CONH-(CH-isobutyl)-B(OH)₂) and its enantiomere PS-293 and/or
d) the compound PS-296 (8-quinolyl-sulfonyl-CONH-(CH-napthyl)-CONH(CH-isobutyl)B(OH)₂) and/or
e) PS-303 (NH₂(CH-naphthyl)-CONH-(CH-isobutyl)-B(OH)₂) and/or
f) PS-321 as (morpholin-CONH-(CH-napthyl)-CONH-(CH-phenylalanine)-B(OH)₂); - and/or
g) PS-334 (CH₃-NH-(CH-naphthyl-CONH-(CH-isobutyl)-B(OH)₂) and/or
h) the compound PS-325 (2-quinol-CONH-(CH-homo-phenylalanine)-CONH-(CH-isobutyl)-B(OH)₂) and/or
i) PS-352 (phenyalanine-CH₂-CH₂-CONH-(CH-phenylalanine)-CONH-(CH-isobutyl)1-B(OH)₂) and/or
j) PS-383 (pyridyl-CONH-(CH*p*F-phenylalanine)-CONH-(CH-isobutyl)-B(OH)2)
are used.

15. Use according to one of the claims 1 to 14, **characterized in that** proteasome inhibitors
a) prevent partly or completely the production of infectious virions of hepatitis-viruses infected cells and/or
b) block the release of virions as well as nearly reduce the infectivity of released virions and/or
c) inhibit the reproduction of progeny virions, and thus new infection of hepatocytes and consequently the spread of a hepatitis-infection in vivo in the liver-tissue of a infected person.

16. Use according to one of the claims 1 to 14, **characterized in that** the reproduction of hepadna-viruses by proteasome inhibitors according to the mechanisms
a) blocking / reduction of the release of new virions
b) blocking / reduction of the infectivity of released virions
c) blocking / reduction of the dissemination of the infection in cultures of primary hepatocytes
d) blocking / reduction of the spread of infection in vivo in the liver-tissue of an infected person
is inhibited.

17. Verwendung gemäß einem der Ansprüche 1 bis 14, wobei
a) das Absterbens von Hepato-Karzimonzellen induziert wird; und / oder
b) das Entstehens von Leberzellkarzinomen unterdruckt und / oder verhindert wird; und / oder
c) Patienten mit etablierten Leberzellkarzinomen therapiert werden.

18. Use according to claim 17, whereas
a) HBV-induced cirrhosis of the liver and/or
b) HBV-induced hepato cell carcinoma and/or
c) HCV- induced liver carcinoma and/or
d) drug-induced liver carcinoma and/or
e) genetically caused liver carcinoma and/or
f) liver carcinoma caused by the environment
are treated/cured/prevented.

19. Use according to claim 17 and 18, whereas cells of liver carcinoma are specifically eliminated which arise from
a) HBV-infection and/or
b) HCV-infection or
c) corresponding Coinfection mit both viruses or
d) HDV/HBV- Coinfection.

20. Use according to claim 17 to 19, whereas the development, the growth and /or the formation of metastases of liver tumours are blocked, and liver carcinoma cells are preferentially removed from HBV and HCV infected patients and animals.

21. Use according to one of the claims 1 to 14, whereas
a) the expression, modification, and activity of the tumour-suppressing protein p53 are modulated and/or
b) the number of infected hepatitis-viruses producing cells are reduced in liver-cell tissue
c) both, the maintenance and the persistence of an already established infection with hepatitis-viruses, as well as secondary infections, and thus the spread of an infection including the interference with the transmission of hepatitis-viruses infections
are inhibited in vivo.

22. Use according to one of the claims 1 to 14, **characterized in that** proteasome inhibitors induce changes in the phosphorylation of nucleocapsid-proteins from of hepatitis-viruses, and thus reduce or block the release and infectivity of hepatitis-B and C- viruses.

23. Use of proteasome inhibitors according to one of the claims 1 to 14 in combination of the proteasome inhibitors among each other for the manufacture of an agent
a) for treatment and cure of hepatitis diseases and/ or
b) for combination with drugs already used for anti-viral therapy of hepadna-viruses.

24. Use according to one of the claims 1 to 14, whereas infections with hepatitis-viruses are treated in order to prevent
a) re-infection with HBV in the course of transplantation of liver or other organs, or
b) re-infection with HBV during cell therapy by administering of the agents before, during, and after a transplantation, or
c) re-infection with HBV during transplantation of virus-free organs into a chronic virus carrier which always contain residual viruses that potentially can infect the transplanted organs, as well as during transfer of virus-containing organs of donators into a virus-free recipient, or
d) establishment of a systemic hepatitis-infection immediately upon exposure to infectious viruses.

25. Use according to one of the claims 1 to 14, whereas infections with hepatitis-viruses are treated for the
a) prophylaxis of hepatitis-infection of persons with are exposed to a high risk of new infection, such as physicians, risk personal in domiciles with a high number of visitors, drug addicts, travellers in areas with high hepatitis virus endemic, in the health care of sick persons, or for family members of chronic virus carriers, or
b) reduction or elimination of a hepatitis by mechanisms related to the immune system.

## Revendications

1. Utilisation d'inhibiteurs de protéasome pour la fabrication d'un agent d'inhibition de la libération, de la maturation et de la réplication d'un virus de l'hépatite.

2. Utilisation selon la revendication 1, **caractérisée en ce que** l'agent contient en tant que composant actif au moins un inhibiteur de protéasome, qui a été isolé à partir de sources naturelles ou de microorganismes ou produit de manière synthétique ou est constitué de composés possédant des structures d'époxycétone ou de lactone ou de dérivés d'aldéhyde peptidique, d'acide glyoxalique, d'acide borique ou d'un ester de pinacol.

3. Utilisation selon les revendications 1 et 2, **caractérisée en ce que** l'agent est utilisé contre des virus, qui sont libérés dans le cadre du cycle de réplication par la surface cellulaire.

4. Utilisation selon les revendications 1 et 2, **caractérisée en ce que** l'agent contient un ou plusieurs inhibiteurs de protéasome en tant que composant actif dans une préparation pharmaceutique, qui inhibent, régulent ou influencent d'une autre manière les activités de la voie ubiquitine/protéasome.

5. Utilisation selon la revendication 4, **caractérisée en ce qu'**un ou plusieurs inhibiteurs de protéasome qui influencent spécifiquement les activités enzymatiques du complexe de protéasome 26S complet et de la structure libre de protéasome actif du point de vue catalytique 20S non assemblée à des sous-unités régulatrices sont utilisés en tant que substances.

6. Utilisation selon les revendications 4 et 5, **caractérisée en ce que** des substances qui sont reçues en tant qu'inhibiteurs de protéasome par des eucaryotes supérieurs et interagissent après la réception cellulaire avec les sous-unités catalytiques du protéasome et bloquent en l'occurrence de manière réversible ou irréversible toutes les ou une partie des activités protéolytiques du protéasome (les activités d'hydrolyse du peptide de la trypsine, de la chromotrypsine et du postglutamyle) au sein du complexe de protéasome 26S ou 20S sont utilisées en tant qu'agents.

7. Utilisation selon l'une quelconque des revendications 4 à 6, **caractérisée en ce que** les préparations pharmaceutiques contiennent, en plus d'inhibiteurs de protéasome, également d'autres agents qui influencent, régulent ou inhibent le système cellulaire de l'ubiquitine.

8. Utilisation selon la revendication 7, **caractérisée en ce que** les activités
a) de l'enzyme de conjugaison de l'ubiquitine et/ou
b) de l'enzyme d'hydrolyse de l'ubiquitine ; et/ou
c) de facteurs cellulaires qui interagissent avec l'ubiquitine sont influencées, régulées ou inhibées.

9. Utilisation selon la revendication 8, **caractérisée en ce que** les activités de facteurs cellulaires qui interagissent avec l'ubiquitine en tant que mono-ubiquitine ou en tant que poly-ubiquitine sont influencées, régulées ou inhibées.

10. Utilisation selon l'une quelconque des revendications 1 à 9, **caractérisée en ce que** des substances qui sont administrées sous différentes formes in vivo par voie orale, intraveineuse, intramusculaire, sous-cutanée ou sous forme encapsulée avec ou sans modifications porteuses de spécificité cellulaire, présentent une faible cytotoxicité en raison de l'utilisation d'un régime d'application et/ou de dosage déterminé, ne déclenchent pas ou peu d'effets secondaires, présentent une demi-vie métabolique relativement élevée et un taux de clairance relativement faible dans l'organisme sont utilisées en tant qu'inhibiteurs de protéasome.

11. Utilisation selon l'une quelconque des revendications 1 à 10, **caractérisée en ce que** des substances qui
a) sont isolées sous forme naturelle à partir de microorganismes ou d'autres sources naturelles ; ou
b) sont obtenues par des modifications chimiques à partir de substances naturelles ; ou
c) sont fabriquées de manière complètement synthétique ; ou
d) sont synthétisées par des procédés de thérapie génique in vivo ; ou
e) par des procédés de thérapie génique in vitro ou
f) fabriquées dans des microorganismes sont utilisées en tant qu'inhibiteurs de protéasome.

12. Utilisation selon la revendication 11, **caractérisée en ce que** des substances qui appartiennent aux catégories de substances suivantes :
a) inhibiteurs de protéasome naturels :
- dérivés de peptide qui contiennent des structures d'époxycétone C-terminales ; ou
- des dérivés de β-lactone ; ou
- de l'aclacinomycine A (également appelée aclarubicine) ; ou
- de la lactacystine et ses variantes modifiées chimiquement, telles que la variante « Clasto lactacystéine β-lactone » pouvant pénétrer dans la membrane cellulaire
b) inhibiteurs de protéasome fabriqués synthétiquement :
aldéhydes peptidiques modifiés tels que N-carbobenzoxy-L-leucinyl-L-leucinyl-L-leucinal (également appelé MG132 ou zLLL), son dérivé d'acide borique MG232 ; N-carbobenzoxy-Leu-Leu-Nva-H (appelé MG115) ; N-acétyl-leucinyl-L-leucinyl-L-norleucinal (appelé LLnL), N-carbobenzoxy-Ile-Glu(OBut)-Ala-Leu-H (également appelé PSI) ;
c) peptides qui portent une structure α,β-époxycétone à l'extrémité C, en outre des sulfones de vinyle tels que carbobenzoxy-L-leucinyl-L-leucinyl-L-leucin-vinylsulfone ou 4-hydroxy-5-iodo-3-nitrophénylacétyl-L-leucinyl-L-leucinyl-L-leucin-vinylsulfone (NLVS) ;
d) radicaux d'acide glyoxalique ou borique tels que pyrazyl-CONH(CHPhe)CONH(CHisobutyl)B(OH)₂) ainsi que des dérivés d'acide borique dipeptidyliques ou
e) esters de pinacol tels que l'ester de benzyloxycarbonyl(Cbz)-Leu-Leu-boroLeu-pinacol sont utilisées en tant qu'inhibiteurs de protéasome.

13. Utilisation selon les revendications 11 et 12, **caractérisée en ce que** les époxycétones époxomicine (époxomycine, formule moléculaire : C₂₈H₈₆N₄O₇) et/ou éponémycine (éponémycine, formule moléculaire : C₂₀H₃₆N₂O₅) sont des inhibiteurs de protéasome particulièrement appropriés.

14. Utilisation selon les revendications 11 et 12, **caractérisée en ce que** les composés
a) PS-519 en tant que dérivé de β-lactone ainsi qu'en tant que dérivé de lactacystine (le composé 1R-[1S, 4R, 5S]]-1-(1-hydroxy-2-méthylpropyl)-4-propyl-6-oxa-2-azabicyclo[3.2.0]heptane-3,7-dione - formule moléculaire C₁₂H₁₉NO₄-) et/ou
b) PS-314 en tant que dérivé d'acide borique peptidylique (le composé acide borique de N-pyrazinecarbonyl-L-phénylalanin-L-leucine - formule moléculaire C₁₉H₂₅BN₄O₄―) et/ou
c) PS-273 (morpholin-CONH-(CH-naphtyl)-CONH-(CH-isobutyl)-B(OH)₂) et son énantiomère PS-293 et/ou
d) le composé PS-296 (8-quinoléyl-sulfonyl-CONH-(CH-naphtyl)-CONH(-CH-isobutyl)-B(OH)₂) et/ou
e) PS-303 (NH₂(CH-naphtyl)-CONH-(CH-isobutyl)-B(OH)₂) et/ou
f) PS-321 en tant que (morpholin-CONH-(CH-naphtyl)-CONH-(CH-phénylalanin)-B(OH)₂) ; et/ou
g) PS-334 (CH₃-NH-(CH-naphtyl-CONH-(CH-isobutyl)-B(OH)₂) et/ou
h) le composé PS-325 (2-quinol-CONH-(CH-*homo*-phénylalanin)-CONH-(CH-isobutyl)-B(OH)₂) et/ou
i) PS-352 (phényalanin-CH₂-CH₂-CONH-(CH-phénylalanin)-CONH-(CH-isobutyl)1-B(OH)₂) et/ou
j) PS-383 (pyridyl-CONH-(CH*p*F-phénylalanin)-CONH-(CH-isobutyl)-B(OH)₂) sont utilisés en tant qu'inhibiteurs de protéasome particulièrement appropriés de la série PS.

15. Utilisation selon l'une quelconque des revendications 1 à 14, **caractérisée en ce que** des inhibiteurs de protéasome
a) éliminent largement ou complètement la production de virions infectieux de cellules infectées par le virus de l'hépatite et/ou
b) entraînent l'inhibition de la libération des virions ainsi qu'une diminution presque totale de l'infectiosité des virions libérés et/ou
c) empêchent la multiplication virale et ainsi la nouvelle infection d'hépatocytes et ainsi la propagation d'une infection par l'hépatite in vivo dans les tissus du foie d'une personne infectée.

16. Utilisation selon l'une quelconque des revendications 1 à 14, **caractérisée en ce que** la multiplication des virus Hepadna par des inhibiteurs de protéasome selon les mécanismes
a) blocage/diminution de la libération de nouveaux virions
b) blocage/diminution de l'infectiosité de virions libérés
c) blocage/diminution de la propagation de l'infection dans des cultures d'hépatocytes primaires
d) blocage/diminution de la propagation de l'infection in vivo dans les tissus du foie d'une personne infectée sont inhibés.

17. Utilisation selon l'une quelconque des revendications 1 à 14, dans laquelle
a) la mort de cellules de carcinome hépatique est induite ; et/ou
b) la naissance de carcinomes hépato-cellulaires est atténuée et/ou empêchée ; et/ou
c) les patients présentant des carcinomes hépato-cellulaires établis subissent une thérapie.

18. Utilisation selon la revendication 17, dans laquelle
a) les cirrhoses du foie induites par le HBV et/ou
b) les carcinomes hépato-cellulaires induits par le HBV et/ou
c) les carcinomes hépatiques induits par le HCV et/ou
d) les carcinomes hépatiques induits par des médicaments et/ou
e) les carcinomes hépatiques d'origine génétique et/ou
f) les carcinomes hépatiques liés à l'environnement sont traités/combattus/empêchés.

19. Utilisation selon les revendications 17 et 18, dans laquelle les cellules de carcinome hépatique qui apparaissent suite à une
a) infection par le HBV et/ou
b) infection par le HCV ou
c) une co-infection correspondante avec les deux virus ou
d) une co-infection par le HDV/HBV sont éliminées de manière ciblée.

20. Utilisation selon les revendications 17 à 19, dans laquelle la naissance, la croissance et/ou la transformation de tumeurs hépato-cellulaires en métastases est empêchée et les cellules de carcinome du foie chez des patients et animaux infectés par les virus HBV et HCV sont de préférence détruites.

21. Utilisation selon l'une quelconque des revendications 11 à 14, dans laquelle
a) l'expression, la modification et l'activité de la protéine p53 supprimant la tumeur sont modulées ; et/ou
b) le nombre de cellules infectées et produisant des virus de l'hépatite est réduit dans le tissu hépato-cellulaire ; et/ou
c) aussi bien le maintien et la persistance d'une infection déjà établie par des virus de l'hépatite que d'une infection secondaire et ainsi de la propagation d'une infection, y compris du blocage de la propagation d'une infection par des virus de l'hépatite sont inhibés in vivo.

22. Utilisation selon l'une quelconque des revendications 1 à 14, **caractérisée en ce que** des inhibiteurs de protéasome modifient la phosphorylation de protéines de nucléocapside spécifiques au virus de l'hépatite et ainsi atténuent ou bloquent la libération et l'infectiosité des virus B et C de l'hépatite.

23. Utilisation d'inhibiteurs de protéasome selon l'une quelconque des revendications 1 à 14 en combinaison entre elles pour la fabrication d'un agent
a) pour traiter et combattre les hépatites
b) avec des agents thérapeutiques déjà utilisés dans la thérapie anti-virale contre les virus Hepadna.

24. Utilisation selon l'une quelconque des revendications 1 à 14, dans laquelle les infections par les virus de l'hépatite sont traitées de manière à empêcher
a) une réinfection par le virus HBV lors de transplantations de foie et d'autres organes ; ou
b) une réinfection par le virus HBV lors de thérapies cellulaires par l'administration de l'agent avant, pendant et après la transplantation ; ou
c) une réinfection par le virus HBV lors de la transplantation d'organes sains sur des porteurs chroniques du virus, qui contiennent toujours des restes de virus et qui peuvent infecter de nouveaux organes ainsi que lors du transfert d'organes porteurs du virus de donneurs sur des patients sains ; ou
d) la mise en place d'une infection systémique par le virus de l'hépatite après un contact avec le virus infectieux.

25. Utilisation selon l'une quelconque des revendications 1 à 14, dans laquelle les infections par les virus de l'hépatite sont traitées
a) dans un but de prévention d'une infection par le virus de l'hépatite chez des individus présentant un risque élevé de nouvelle infection, tels que des médecins, le personnel à risque dans des hôpitaux de grande capacité, des toxicomanes, des voyageurs se rendant dans des zones fortement endémiques pour les virus de l'hépatite, lors du traitement de malades ou pour les membres de la famille de porteurs chroniques du virus ; ou
b) dans un but de réduction ou d'élimination d'une hépatite par des mécanismes du système immunitaire.
